# EUROPEAN PATENT APPLICATION

(11) **EP 3 323 892 A1**
(43) Date of publication of application: **23.05.2018**
(21) Application number: 16824480.4
(22) Date of filing: 13.07.2016
(51) Int. Cl.: C12N 15/113, A61K 31/7088, A61K 48/00, A61P 7/02, A61P 37/06

(54) **ANTISENSE OLIGONUCLEOTIDE INHIBITING 2GPI EXPRESSION**

(30) Priority: 13.07.2015 JP 2015140081
(71) Applicant: Kyowa Hakko Kirin Co., Ltd., Tokyo 100-8185 (JP)
(72) Inventor: YAMADA, Yoji, Machida-shi Tokyo 194-8533 (JP); IWAI, Hiroto, Machida-shi Tokyo 194-8533 (JP); MASUDA, Kazuhiro, Machida-shi Tokyo 194-8533 (JP)
(74) Representative: Vossius & Partner Patentanwälte Rechtsanwälte mbB
(86) International application number: PCT/JP2016/070642
(87) International publication number: WO 2017/010500

(57) **Abstract**

The present invention aims to provide a novel nucleic acid capable of suppressing expression of β2GPI, as well as a pharmaceutical composition for the prophylaxis or treatment of diseases associated with β2GPI expression.

The present invention solves the above-mentioned problem by providing an antisense oligonucleotide having a β2GPI expression suppressive activity, a pharmaceutical composition containing the antisense oligonucleotide, and a prophylactic or therapeutic drug for autoimmune diseases including APS, SLE and the like, and thrombosis in hemodialysis that contains the antisense oligonucleotide and suppresses β2GPI expression.

## Description

### [Technical Field]

The present invention relates to an antisense oligonucleotide for use in suppressing expression of β2GPI, or a pharmaceutical composition comprising the antisense oligonucleotide.

### [Background Art]

β2-Glycoprotein 1 (β2GPI) (also referred to as apolipoprotein H, apoH) is a soluble glycoprotein consisting of 326 amino acid residues, and is mainly produced in the liver (International Journal of Clinical and Laboratory Research, 1992, vol. 21, p256-263). β2GPI is considered to have many different kinds of physiological actions, and has been reported to be involved in platelet aggregation reaction, coagulation and fibrinolysis reaction, and oxidized LDL uptake in macrophages (non-patent document 1).

As for the association with diseases, it is known that β2GPI is a major corresponding antigen to antiphospholipid antibody that emerges in autoimmune diseases such as antiphospholipid antibody syndrome (APS) and systemic lupus erythematosus (SLE). Anti-β2GPI antibody is also deeply involved in the pathology formation in diseases, and it has also been revealed by researches using animal models and clinical researches that a complex formed by β2GPI and anti-β2GPI antibodies generates activation signals in membrane receptors of various cells such as vascular endothelial cell, monocyte, platelet, and trophoblast and, as a result, can induce pathology characteristic of APS, such as thrombosis and abnormal pregnancy (non-patent document 2). It is expected that the above-mentioned diseases can be prevented or treated by specifically inhibiting the formation of immune complex consisting of β2GPI and anti-β2GPI antibodies. However, since β2GPI is present in blood at a comparatively high concentration of 50-500 µg/mL, it is not easy to continuously inhibit all such β2GPI with, for example, general antibody drugs (non-patent document 3).

On the other hand, as a method of suppressing expression itself of genes, for example, an antisense method is known (patent document 1). To be specific, an oligonucleotide complementary to mRNA or mRNA precursor and the like of the target gene (antisense oligonucleotide) forms a double strand with the mRNA or mRNA precursor of the target gene when introduced into the cell, and can specifically suppress expression of the target gene. As a method of suppressing expression of gene other than the antisense method, a method utilizing, for example, RNA interference (hereinafter to be referred to as RNAi) and the like are known. With this method, expression of a target gene can be specifically suppressed by introducing a double-stranded RNA (siRNA) having the same sequence as the target gene (patent document 2).

While a part of the siRNA sequences targeting human β2GPI has been disclosed (patent document 3, 4), suppression of the expression of the gene is not known, nor is it known that antisense oligonucleotide suppresses expression of human β2GPI gene.

### [Document List]

### [Patent documents]

patent document 1: WO 98/56905
patent document 2: WO 2001/75164
patent document 3: WO 2005/116204
patent document 4: WO 2008/043561

### [non-patent documents]

non-patent document 1: Ann. N. Y. Acad. Sci., 1285, 44-58 (2013)
non-patent document 2: N. Engl. J. Med., 368, 1033-1044 (2013)
non-patent document 3: J. Thromb. Haemost., 9, 1275-1284 (2011)

### [SUMMARY OF THE INVENTION]

### [Problems to be Solved by the Invention]

The present invention aims to provide an antisense oligonucleotide capable of suppressing expression of β2GPI. The present invention also aims to provide a pharmaceutical composition for the prophylaxis or treatment of diseases associated with β2GPI expression.

### [Means of Solving the Problems]

The present invention relates to the following (1) - (17).
(1) An antisense oligonucleotide consisting of 8 - 80 bases in length that suppresses an expression of β2GPI, comprising a sequence hybridizable to a nucleic acid consisting of a base sequence shown in any of SEQ ID NOs: 201 - 399 ("target base sequence" described in Table 1-1 - 1-4) under stringent conditions.
(2) An antisense oligonucleotide consisting of 8 - 80 bases in length that suppresses an expression of β2GPI, comprising at least 8 continuous bases in a base sequence shown in any of SEQ ID NOs: 2 - 200 ("antisense base sequence" described in Table 1-1 - 1-4).
(3) An antisense oligonucleotide consisting of 8 - 80 bases in length and complementary to a base sequence shown in any of SEQ ID NOs: 201 - 399 (base sequence selected from the group described in "target base sequence" of Table 1-1 - 1-4).
(4) The antisense oligonucleotide of (3), comprising a base sequence shown in any of SEQ ID NOs: 2 - 200 (base sequence selected from the group described in "antisense base sequence" of Table 1-1 - 1-4).
(5) The antisense oligonucleotide of (3), comprising a base sequence shown in any of SEQ ID NOs: 2 - 200 wherein 1 or several bases are deleted, substituted or added.
(6) An antisense oligonucleotide consisting of a base sequence shown in any of SEQ ID NOs: 2 - 200.
(7) An antisense oligonucleotide consisting of a base sequence shown in any of SEQ ID NOs: 400 - 680.
(8) The antisense oligonucleotide of any one of (1) - (7), wherein the 5'-terminal vicinity and/or the 3'-terminal vicinity are/is constituted of a sugar moiety-modified nucleotide.
(9) The antisense oligonucleotide of any one of (1) - (8), comprising a ligand.
(10) A pharmaceutical composition comprising the antisense oligonucleotide of any one of (1) - (9).
(11) The pharmaceutical composition of (10) for the treatment or prophylaxis of an autoimmune disease or thrombosis.
(12) A method of treating a disorder mediated by an anti-β2GPI antibody, comprising a step of administering a therapeutically effective amount of the antisense oligonucleotide of any one of (1) - (9) or the pharmaceutical composition of (10) or (11) to a human in need of such treatment.
(13) The method of (12), wherein the aforementioned disorder is an autoimmune disease or thrombosis.
(14) Use of the antisense oligonucleotide of any one of (1) - (9) for the manufacture of a pharmaceutical composition for preventing or treating a disorder mediated by an anti-β2GPI antibody.
(15) The use of (14), wherein the aforementioned disorder is an autoimmune disease or thrombosis.
(16) The antisense oligonucleotide of any one of (1) - (9) for use in the prophylaxis or treatment of a disorder mediated by an anti-β2GPI antibody.
(17) The antisense nucleotide of (16), wherein the aforementioned disorder is an autoimmune disease or thrombosis.

### [Effect of the Invention]

Expression of β2GPI can be suppressed by administering the antisense oligonucleotide of the present invention or a pharmaceutical composition comprising the antisense oligonucleotide. The antisense oligonucleotide or a pharmaceutical composition comprising the antisense oligonucleotide of the present invention is useful for the prophylaxis or treatment of a disease associated with the expression of β2GPI, particularly a disorder mediated by an anti-β2GPI antibody.

### [Description of Embodiments]

As a gene encoding β2GPI (hereinafter to be also referred to as β2GPI gene), which is targeted by the antisense oligonucleotide of the present invention, a cDNA base sequence (SEQ ID NO: 1) corresponding to a full length mRNA of β2GPI, which is registered as Genbank Accession No.NM_000042, can be mentioned.

### 1. Antisense oligonucleotide of the present invention

In the present invention, the antisense oligonucleotide refers to an oligonucleotide complementary to a DNA encoding the target gene and to mRNA precursor and mRNA transcribed from such DNA. It suppresses the actions (transcription, editing after transcription, translation and the like) of DNA, mRNA precursor or mRNA, which is targeted by the antisense oligonucleotide, by forming a double strand or triple strand with the DNA, mRNA precursor or mRNA. The antisense oligonucleotide includes not only those completely complementary to DNA, mRNA precursor or mRNA to be the target, but also those containing one or several mismatches as long as they can hybridize to the DNA, mRNA precursor or mRNA under stringent conditions. The antisense oligonucleotide of the present invention may be introduced into the form of a hairpin oligomer or cyclic oligomer as long as it is nucleic acid that hybridizes to the target gene. The antisense oligonucleotide may contain a structural element such as an internal or terminal bulge or loop, and the like.

The present invention provides an antisense oligonucleotide that suppresses expression of β2GPI (to be also referred to as the antisense oligonucleotide of the present invention in the present specification).

The antisense oligonucleotide of the present invention may be any molecule as long as it is a molecule in which a nucleotide or a molecule having functions equivalent to those of the nucleotide is polymerized. Examples thereof include DNA that is a polymer of deoxyribonucleotide, RNA that is a polymer of ribonucleotide, chimeric nucleic acid composed of RNA and DNA, and nucleotide polymer in which at least one nucleotide of these nucleic acids is substituted by a molecule having functions equivalent to those of the nucleotide. Uracil (U) in RNA can be unambiguously read as thymine (T) in DNA.

Examples of the molecule having functions equivalent to those of the nucleotide include nucleotide derivatives and the like. The nucleotide derivative may be any molecule as long as it is a molecule obtained by modifying a nucleotide. For example, a molecule obtained by modifying deoxyribonucleotide or ribonucleotide and the like to improve or stabilize nuclease resistance, enhance affinity for complementary strand nucleic acid, enhance cell permeability or visualize same, as compared with DNA or RNA, are preferably used.

Examples of the molecule obtained by modifying a nucleotide include sugar moiety-modified nucleotide, phosphodiester bond-modified nucleotide, base-modified nucleotide, nucleotide in which at least one of a sugar moiety, a phosphodiester bond and a base is modified, and the like.

While the sugar moiety-modified nucleotide may be any as long as the chemical structure of the sugar of nucleotide is partly or entirely modified or substituted by any substituent, or substituted by any atom, 2'-modified nucleotide is preferably used.

Examples of the 2'-modified nucleotide include a 2'- modified nucleotide in which the 2'-OH group of ribose is substituted by a substituent selected from R, R'OR, SH, SR, NH₂, NHR, NR₂, N₃, CN, F, Cl, Br and I (R is alkyl or aryl, preferably alkyl having 1 - 6 carbon atoms, R' is alkylene, preferably alkylene having 1 - 6 carbon atoms), and preferable examples of the substituent include F and methoxy group. In addition, preferable examples of the substituent include a 2'- modified nucleotide substituted by a substituent selected from the group consisting of 2-(methoxy)ethoxy group, 3-aminopropoxy group, 2-[(N,N-dimethylamino)oxy]ethoxy group, 3-(N,N-dimethylamino)propoxy group, 2-[2-(N,N-dimethylamino)ethoxy]ethoxy group, 2-(methylamino)-2-oxoethoxy group, 2-(N-methylcarbamoyl)ethoxy group and 2-cyanoetoxy group, and the like. More preferable examples include 2'-modified nucleotide substituted by a substituent selected from the group consisting of methoxy group and 2-(methoxy)ethoxy group and the like.

As the sugar moiety-modified nucleotide, a crosslinking structure type artificial nucleic acid having two cyclic structures by introducing a crosslinking structure into the sugar moiety (Bridged Nucleic Acid) (BNA) can be used preferably. Specific examples thereof include locked artificial nucleic acid wherein the 2'-position oxygen atom and the 4'-position carbon atom are crosslinked via methylene (Locked Nucleic Acid) (LNA) [Tetrahedron Letters, 38, 8735, (1997) and Tetrahedron, 54, 3607, (1998)], ethylene crosslinking structure type artificial nucleic acid (Ethylene bridged nucleic acid) (ENA) [Nucleic Acid Research, 32, e175(2004)], Constrained Ethyl (cEt) [The Journal of Organic Chemistry 75, 1569 (2010)], Amido-Bridged Nucleic Acid (AmNA) [Chem Bio Chem 13, 2513 (2012)], and 2'-O,4'-C-Spirocyclopropylene bridged nucleic acid (scpBNA) [Chem. Commun., 51, 9737 (2015)] and the like.

Furthermore, peptide nucleic acid (PNA) [Acc. Chem. Res., 32, 624 (1999)], oxy-peptide nucleic acid (OPNA) [J. Am. Chem. Soc., 123, 4653 (2001)], peptide ribonucleic acid (PRNA) [J. Am. Chem. Soc., 122, 6900 (2000)] and the like can also be mentioned as the sugar moiety-modified nucleotide.

The phosphodiester bond-modified nucleotide may be any as long as the chemical structure of the phosphodiester bond is partly or entirely modified or substituted by any substituent, or substituted by any atom. Examples thereof include a nucleotide in which the phosphodiester bond is substituted by a phosphorothioate bond, a nucleotide in which the phosphodiester bond is substituted by a phosphorodithioate bond, a nucleotide in which the phosphodiester bond is substituted by an alkylphosphonate bond, a nucleotide in which the phosphodiester bond is substituted by a phosphoramidate bond and the like. Preferably, a nucleotide in which the phosphodiester bond is substituted by a phosphorothioate bond can be mentioned.

The base-modified nucleotide may be any as long as the chemical structure of the base of the nucleotide is partly or entirely modified or substituted by any substituent, or substituted by any atom. Examples thereof include one in which an oxygen atom in the base is substituted by a sulfur atom, one in which a hydrogen atom is substituted by an alkyl group having 1-6 carbon atoms, a halogen group, one in which a methyl group is substituted by hydrogen, hydroxymethyl, alkyl group having 2 - 6 carbon atoms, and one in which an amino group is substituted by alkyl group having 1 - 6 carbon atoms, alkanoyl group having 1 - 6 carbon atoms, oxo group, a hydroxy group, and the like. Use of 5-methylcytosine (5-mC) as a base-modified nucleotide instead of cytosine (C) is also one of the preferable forms of the present invention.

As the nucleotide derivative, one obtained by adding other chemical substance such as lipids such as cholesterol, fatty acid, tocopherol, retinoid and the like, saccharides such as N-acetylgalactosamine (GalNAc), galactose (Gal), mannose (Man) and the like, antibodies such as full antibody, Fab (Fragment antigen-binding antibody), scFv (Single-chain variable fragment antibody) and VHH (variable domain of heavy chain antibody) and the like, proteins such as low-density lipoprotein (LDL), human serum albumin and the like, peptides such as RGD, NGR, R9, CPP (cell penetrating peptide) and the like, small molecules such as phenazine, phenanthridine, anthraquinone,folic acid and the like, synthetic polymers such as synthetic polyamino acid and the like, nucleic acid aptamers, dye such as acridine, fluorescein, rhodamine, coumarin and the like, fluorophore such as Cy3 series, Alexa series, black hole quencher and the like, and the like, directly or via a linker, to a nucleotide or a nucleotide derivative wherein at least one of sugar moiety, phosphodiester bond and base is modified can also be mentioned. Specific examples thereof include polyamine-added nucleotide derivative, cholesterol-added nucleotide derivative, steroid-added nucleotide derivative, GalNAc-added nucleotide derivative, bile acid-added nucleotide derivative, fatty acid-added nucleotide derivative, vitamin-added nucleotide derivative, Cy5-added nucleotide derivative, Cy3-added nucleotide derivative, 6-FAM-added nucleotide derivative, and biotin-added nucleotide derivative and the like, preferably GalNAc-added nucleotide derivative can be mentioned. These can be modified at the 5'-terminal, 3'-terminal and/or inside of the sequence by reacting a modifier reactive on the solid phase, during elongation reaction on the solid phase. Alternatively, a nucleic acid into which a functional group such as an amino group, a mercapto group, an azide group, a triple bond and the like has been introduced may be previously synthesized and purified, and reacted with a modifier.

The nucleotide derivative may form a crosslinking structure, such as alkylene structure, peptide structure, nucleotide structure, ether structure, ester structure, a structure of a combination of at least one of these and the like, with other nucleotide or nucleotide derivative in the nucleic acid.

The antisense oligonucleotide of the present invention also encompasses an antisense oligonucleotide wherein the atoms in a molecule are partly or entirely substituted by an atom (isotope) having a different mass number.

In the present specification, "complement" means a relationship forming a base pairing between two bases, and refers to a double helix structure as a whole double-stranded region via a loose hydrogen bond, for example, the relationship between adenine and thymine or uracil, and the relationship between guanine and cytosine.

The length of the antisense oligonucleotide of the present invention is 8 - 80 bases, preferably 8 - 30 bases. For example, it may be 8 - 20 bases, 10 - 20 bases, 13 - 20 bases, 13 - 16 bases, 13 bases, 14 bases, 15 bases, 16 bases, 17 bases, 18 bases, 19 bases or 20 bases.

While particular preferable specific base sequences are described in the present DESCRIPTION, an antisense oligonucleotide 8 - 80 bases in length and containing at least 8 continuous bases selected from the antisense base sequences described in Tables 1-1 to 1-4 is also a preferable antisense oligonucleotide. Antisense oligonucleotide 8 - 80 bases, preferably 8 - 30 bases, in length and containing more preferably 9 or more, further preferably 10 or more, still more preferably 11 or more, particularly preferably 12 or more, most preferably 13, continuous bases selected from the antisense base sequences described in Table 1 are also preferable antisense oligonucleotides.

**[Table 1-1]**

| SEQ ID NO: | antisense base sequence | SEQ ID NO: | target base sequence |
|---|---|---|---|
| 2 | GAGCACTGGAGAA | 201 | TTCTCCAGTGCTC |
| 3 | TGAGCACTGGAGA | 202 | TCTCCAGTGCTCA |
| 4 | AGATGAGCACTGG | 203 | CCAGTGCTCATCT |
| 5 | AGCAACATGGCAG | 204 | CTGCCATGTTGCT |
| 6 | TAGCAACATGGCA | 205 | TGCCATGTTGCTA |
| 7 | ATAGCAACATGGC | 206 | GCCATGTTGCTAT |
| 8 | CAATAGCAACATG | 207 | CATGTTGCTATTG |
| 9 | GCAATAGCAACAT | 208 | ATGTTGCTATTGC |
| 10 | CCTGCAATAGCAA | 209 | TTGCTATTGCAGG |
| 11 | TCCTGCAATAGCA | 210 | TGCTATTGCAGGA |
| 12 | GTCCTGCAATAGC | 211 | GCTATTGCAGGAC |
| 13 | TCCGTCCTGCAAT | 212 | ATTGCAGGACGGA |
| 14 | GTCCGTCCTGCAA | 213 | TTGCAGGACGGAC |
| 15 | GGTCCGTCCTGCA | 214 | TGCAGGACGGACC |
| 16 | AGGTCCGTCCTGC | 215 | GCAGGACGGACCT |
| 17 | CAGGTCCGTCCTG | 216 | CAGGACGGACCTG |
| 18 | TGGGACAGGTCCG | 217 | CGGACCTGTCCCA |
| 19 | TTGGGACAGGTCC | 218 | GGACCTGTCCCAA |
| 20 | GGCTTGGGACAGG | 219 | CCTGTCCCAAGCC |
| 21 | TGGCTTGGGACAG | 220 | CTGTCCCAAGCCA |
| 22 | CTGGCTTGGGACA | 221 | TGTCCCAAGCCAG |
| 23 | TCTGGCTTGGGAC | 222 | GTCCCAAGCCAGA |
| 24 | CATCTGGCTTGGG | 223 | CCCAAGCCAGATG |
| 25 | TCATCTGGCTTGG | 224 | CCAAGCCAGATGA |
| 26 | ATCATCTGGCTTG | 225 | CAAGCCAGATGAT |
| 27 | AATCATCTGGCTT | 226 | AAGCCAGATGATT |
| 28 | AAATCATCTGGCT | 227 | AGCCAGATGATTT |
| 29 | TCCTGGCTCATAG | 228 | CTATGAGCCAGGA |
| 30 | CTCCTGGCTCATA | 229 | TATGAGCCAGGAG |
| 31 | TCTCCTGGCTCAT | 230 | ATGAGCCAGGAGA |
| 32 | CCGGCTTGCAGGA | 231 | TCCTGCAAGCCGG |
| 33 | CCCGGCTTGCAGG | 232 | CCTGCAAGCCGGG |
| 34 | GCCCGGCTTGCAG | 233 | CTGCAAGCCGGGC |
| 35 | AGCCCGGCTTGCA | 234 | TGCAAGCCGGGCT |
| 36 | TAGCCCGGCTTGC | 235 | GCAAGCCGGGCTA |
| 37 | ATAGCCCGGCTTG | 236 | CAAGCCGGGCTAT |
| 38 | CATAGCCCGGCTT | 237 | AAGCCGGGCTATG |
| 39 | ACACATAGCCCGG | 238 | CCGGGCTATGTGT |
| 40 | GACACATAGCCCG | 239 | CGGGCTATGTGTC |
| 41 | TGTGAGAGGGCAG | 240 | CTGCCCTCTCACA |
| 42 | TTGATGGGCCACA | 241 | TGTGGCCCATCAA |
| 43 | GTTGATGGGCCAC | 242 | GTGGCCCATCAAC |
| 44 | TGTTGATGGGCCA | 243 | TGGCCCATCAACA |
| 45 | GTGTTGATGGGCC | 244 | GGCCCATCAACAC |
| 46 | GGACATACTCTGG | 245 | CCAGAGTATGTCC |
| 47 | AGGACATACTCTG | 246 | CAGAGTATGTCCT |
| 48 | AAGGACATACTCT | 247 | AGAGTATGTCCTT |
| 49 | AAAGGACATACTC | 248 | GAGTATGTCCTTT |
| 50 | GCTCCATTTTCTA | 249 | TAGAAAATGGAGC |
| 51 | GGGATATTCAAAA | 250 | TTTTGAATATCCC |
| 52 | TGGGATATTCAAA | 251 | TTTGAATATCCCA |
| 53 | TTGGGATATTCAA | 252 | TTGAATATCCCAA |
| 54 | GTTGGGATATTCA | 253 | TGAATATCCCAAC |
| 55 | GCACTTGGCAGAA | 254 | TTCTGCCAAGTGC |
| 56 | TGCACTTGGCAGA | 255 | TCTGCCAAGTGCA |
| 57 | GTGCACTTGGCAG | 256 | CTGCCAAGTGCAC |
| 58 | AGTGCACTTGGCA | 257 | TGCCAAGTGCACT |

**[Table 1-2]**

| SEQ ID NO: | antisense base sequence | SEQ ID NO: | target base sequence |
|---|---|---|---|
| 59 | CAGTGCACTTGGC | 258 | GCCAAGTGCACTG |
| 60 | CTCAGTGCACTTG | 259 | CAAGTGCACTGAG |
| 61 | CCTCAGTGCACTT | 260 | AAGTGCACTGAGG |
| 62 | TCCTCAGTGCACT | 261 | AGTGCACTGAGGA |
| 63 | TTCCTCAGTGCAC | 262 | GTGCACTGAGGAA |
| 64 | CTTCCTCAGTGCA | 263 | TGCACTGAGGAAG |
| 65 | CCTTCCTCAGTGC | 264 | GCACTGAGGAAGG |
| 66 | TCCTTCCTCAGTG | 265 | CACTGAGGAAGGA |
| 67 | GCTGATGGCTTAT | 266 | ATAAGCCATCAGC |
| 68 | AGCTGATGGCTTA | 267 | TAAGCCATCAGCT |
| 69 | CAGCTGATGGCTT | 268 | AAGCCATCAGCTG |
| 70 | CCAGCTGATGGCT | 269 | AGCCATCAGCTGG |
| 71 | TCCAGCTGATGGC | 270 | GCCATCAGCTGGA |
| 72 | TTCCAGCTGATGG | 271 | CCATCAGCTGGAA |
| 73 | GTTTCCAGCTGAT | 272 | ATCAGCTGGAAAC |
| 74 | TGTTTCCAGCTGA | 273 | TCAGCTGGAAACA |
| 75 | TTGTTTCCAGCTG | 274 | CAGCTGGAAACAA |
| 76 | AACTGCTGTGTCC | 275 | GGACACAGCAGTT |
| 77 | AAAAACTGCTGTG | 276 | CACAGCAGTTTTT |
| 78 | TGGCAAACATTCA | 277 | TGAATGTTTGCCA |
| 79 | GTGGCAAACATTC | 278 | GAATGTTTGCCAC |
| 80 | GTTGTGGCAAACA | 279 | TGTTTGCCACAAC |
| 81 | TGTTGTGGCAAAC | 280 | GTTTGCCACAACA |
| 82 | GCATGTTGTGGCA | 281 | TGCCACAACATGC |
| 83 | CGCATGTTGTGGC | 282 | GCCACAACATGCG |
| 84 | TCGCATGTTGTGG | 283 | CCACAACATGCGA |
| 85 | ATCGCATGTTGTG | 284 | CACAACATGCGAT |
| 86 | ACATCGCATGTTG | 285 | CAACATGCGATGT |
| 87 | TTCCAAACATCGC | 286 | GCGATGTTTGGAA |
| 88 | AGTCCAATTTCCA | 287 | TGGAAATTGGACT |
| 89 | CCCTGCATTCTGG | 288 | CCAGAATGCAGGG |
| 90 | TCCCTGCATTCTG | 289 | CAGAATGCAGGGA |
| 91 | TTCCCTGCATTCT | 290 | AGAATGCAGGGAA |
| 92 | ATTGTCTGGTCTT | 291 | AAGACCAGACAAT |
| 93 | CATTGTCTGGTCT | 292 | AGACCAGACAATG |
| 94 | CCATTGTCTGGTC | 293 | GACCAGACAATGG |
| 95 | TCCATTGTCTGGT | 294 | ACCAGACAATGGA |
| 96 | ATCCATTGTCTGG | 295 | CCAGACAATGGAT |
| 97 | AATCCATTGTCTG | 296 | CAGACAATGGATT |
| 98 | GCAGGATAGTTCA | 297 | TGAACTATCCTGC |
| 99 | GGTTTTGCAGGAT | 298 | ATCCTGCAAAACC |
| 100 | TGGTTTTGCAGGA | 299 | TCCTGCAAAACCA |
| 101 | GGCTTTATCCTTG | 300 | CAAGGATAAAGCC |
| 102 | GTGGCTTTATCCT | 301 | AGGATAAAGCCAC |
| 103 | ATGTGGCTTTATC | 302 | GATAAAGCCACAT |
| 104 | AATGTGGCTTTAT | 303 | ATAAAGCCACATT |
| 105 | CCAAATGTGGCTT | 304 | AAGCCACATTTGG |
| 106 | CATCATGGCAGCC | 305 | GGCTGCCATGATG |
| 107 | CCATCATGGCAGC | 306 | GCTGCCATGATGG |
| 108 | TCCATCATGGCAG | 307 | CTGCCATGATGGA |
| 109 | ATCCATCATGGCA | 308 | TGCCATGATGGAT |
| 110 | TATCCATCATGGC | 309 | GCCATGATGGATA |
| 111 | ATATCCATCATGG | 310 | CCATGATGGATAT |
| 112 | CCCAGTTTGGTAC | 311 | GTACCAAACTGGG |
| 113 | TCCCAGTTTGGTA | 312 | TACCAAACTGGGA |
| 114 | TTCCCAGTTTGGT | 313 | ACCAAACTGGGAA |
| 115 | TTTCCCAGTTTGG | 314 | CCAAACTGGGAAA |

**[Table 1-3]**

| SEQ ID NO: | antisense base sequence | SEQ ID NO: | target base sequence |
|---|---|---|---|
| 116 | GTTTCCCAGTTTG | 315 | CAAACTGGGAAAC |
| 117 | CAGTTTCCCAGTT | 316 | AACTGGGAAACTG |
| 118 | CCAGTTTCCCAGT | 317 | ACTGGGAAACTGG |
| 119 | ACCAGTTTCCCAG | 318 | CTGGGAAACTGGT |
| 120 | GACCAGTTTCCCA | 319 | TGGGAAACTGGTC |
| 121 | AGACCAGTTTCCC | 320 | GGGAAACTGGTCT |
| 122 | GCAGACCAGTTTC | 321 | GAAACTGGTCTGC |
| 123 | GGCAGACCAGTTT | 322 | AAACTGGTCTGCC |
| 124 | TGGCAGACCAGTT | 323 | AACTGGTCTGCCA |
| 125 | ATGGCAGACCAGT | 324 | ACTGGTCTGCCAT |
| 126 | CATGGCAGACCAG | 325 | CTGGTCTGCCATG |
| 127 | GCATGGCAGACCA | 326 | TGGTCTGCCATGC |
| 128 | GGCATGGCAGACC | 327 | GGTCTGCCATGCC |
| 129 | TGGCATGGCAGAC | 328 | GTCTGCCATGCCA |
| 130 | TTGGCATGGCAGA | 329 | TCTGCCATGCCAA |
| 131 | CTTGGCATGGCAG | 330 | CTGCCATGCCAAG |
| 132 | ACTTGGCATGGCA | 331 | TGCCATGCCAAGT |
| 133 | AACTTGGCATGGC | 332 | GCCATGCCAAGTT |
| 134 | CAACTTGGCATGG | 333 | CCATGCCAAGTTG |
| 135 | ACAACTTGGCATG | 334 | CATGCCAAGTTGT |
| 136 | GCTTTACAACTTG | 335 | CAAGTTGTAAAGC |
| 137 | TCACAGGTACTTT | 336 | AAAGTACCTGTGA |
| 138 | ACACCACAGTGGC | 337 | GCCACTGTGGTGT |
| 139 | GTACACCACAGTG | 338 | CACTGTGGTGTAC |
| 140 | GGTACACCACAGT | 339 | ACTGTGGTGTACC |
| 141 | TGGTACACCACAG | 340 | CTGTGGTGTACCA |
| 142 | TTGGTACACCACA | 341 | TGTGGTGTACCAA |
| 143 | CTTGGTACACCAC | 342 | GTGGTGTACCAAG |
| 144 | CCTTGGTACACCA | 343 | TGGTGTACCAAGG |
| 145 | TCCTTGGTACACC | 344 | GGTGTACCAAGGA |
| 146 | CTCCTTGGTACAC | 345 | GTGTACCAAGGAG |
| 147 | TCTCCTTGGTACA | 346 | TGTACCAAGGAGA |
| 148 | CTCTCCTTGGTAC | 347 | GTACCAAGGAGAG |
| 149 | TCTCTCCTTGGTA | 348 | TACCAAGGAGAGA |
| 150 | CTCTCTCCTTGGT | 349 | ACCAAGGAGAGAG |
| 151 | TCTCTCTCCTTGG | 350 | CCAAGGAGAGAGA |
| 152 | CTCTCTCTCCTTG | 351 | CAAGGAGAGAGAG |
| 153 | ACTCTCTCTCCTT | 352 | AAGGAGAGAGAGT |
| 154 | TACTCTCTCTCCT | 353 | AGGAGAGAGAGTA |
| 155 | TTACTCTCTCTCC | 354 | GGAGAGAGAGTAA |
| 156 | GCATTCCATTCTT | 355 | AAGAATGGAATGC |
| 157 | AGCATTCCATTCT | 356 | AGAATGGAATGCT |
| 158 | AGCTACACTTCTT | 357 | AAGAAGTGTAGCT |
| 159 | TAGCTACACTTCT | 358 | AGAAGTGTAGCTA |
| 160 | TTGAAGCATTTGG | 359 | CCAAATGCTTCAA |
| 161 | GTTCCTTGAAGCA | 360 | TGCTTCAAGGAAC |
| 162 | TGTTCCTTGAAGC | 361 | GCTTCAAGGAACA |
| 163 | GTGTTCCTTGAAG | 362 | CTTCAAGGAACAC |
| 164 | TGTGTTCCTTGAA | 363 | TTCAAGGAACACA |
| 165 | CTGTGTTCCTTGA | 364 | TCAAGGAACACAG |
| 166 | ACTGTGTTCCTTG | 365 | CAAGGAACACAGT |
| 167 | AACTGTGTTCCTT | 366 | AAGGAACACAGTT |
| 168 | GAACTGTGTTCCT | 367 | AGGAACACAGTTC |
| 169 | AGAACTGTGTTCC | 368 | GGAACACAGTTCT |
| 170 | GAGAACTGTGTTC | 369 | GAACACAGTTCTC |
| 171 | AGAGAACTGTGTT | 370 | AACACAGTTCTCT |
| 172 | CAGAGAACTGTGT | 371 | ACACAGTTCTCTG |

**[Table 1-4]**

| SEQ ID NO: | antisense base sequence | SEQ ID NO: | target base sequence |
|---|---|---|---|
| 173 | CCAGAGAACTGTG | 372 | CACAGTTCTCTGG |
| 174 | GCCAGAGAACTGT | 373 | ACAGTTCTCTGGC |
| 175 | AGCCAGAGAACTG | 374 | CAGTTCTCTGGCT |
| 176 | AAGCCAGAGAACT | 375 | AGTTCTCTGGCTT |
| 177 | GCATCAGTTTTCC | 376 | GGAAAACTGATGC |
| 178 | TGCATCAGTTTTC | 377 | GAAAACTGATGCA |
| 179 | GATGCATCAGTTT | 378 | AAACTGATGCATC |
| 180 | GGATGCATCAGTT | 379 | AACTGATGCATCC |
| 181 | CGGATGCATCAGT | 380 | ACTGATGCATCCG |
| 182 | TCGGATGCATCAG | 381 | CTGATGCATCCGA |
| 183 | ATCGGATGCATCA | 382 | TGATGCATCCGAT |
| 184 | CATCGGATGCATC | 383 | GATGCATCCGATG |
| 185 | ACATCGGATGCAT | 384 | ATGCATCCGATGT |
| 186 | TACATCGGATGCA | 385 | TGCATCCGATGTA |
| 187 | TTACATCGGATGC | 386 | GCATCCGATGTAA |
| 188 | GCTTTACATCGGA | 387 | TCCGATGTAAAGC |
| 189 | GGCTTTACATCGG | 388 | CCGATGTAAAGCC |
| 190 | TGGCTTTACATCG | 389 | CGATGTAAAGCCA |
| 191 | TGTGGAATCTGAA | 390 | TTCAGATTCCACA |
| 192 | AGTGTGACATTTT | 391 | AAAATGTCACACT |
| 193 | AAGTGTGACATTT | 392 | AAATGTCACACTT |
| 194 | CCTTGGATGAACA | 393 | TGTTCATCCAAGG |
| 195 | TCCTTGGATGAAC | 394 | GTTCATCCAAGGA |
| 196 | GTTCCTTGGATGA | 395 | TCATCCAAGGAAC |
| 197 | GGTTCCTTGGATG | 396 | CAT CCAAGGAAC C |
| 198 | AGGTTCCTTGGAT | 397 | ATCCAAGGAACCT |
| 199 | TAGGTTCCTTGGA | 398 | TCCAAGGAACCTA |
| 200 | TTAGGTTCCTTGG | 399 | CCAAGGAACCTAA |

Typical preferable antisense oligonucleotides include oligonucleotides containing at least 8, more preferably 9 or more, further preferably 10 or more, still more preferably 11 or more, particularly preferably 12 or more, most preferably 13, continuous nucleic acid bases from the 5'-terminal of the antisense base sequences (sequences shown in any of SEQ ID NOs: 2 - 200) described in Tables 1-1 to 1-4. Similarly, preferable antisense oligonucleotides include oligonucleotides containing at least 8 or more, more preferably 9 or more, further preferably 10 or more, still more preferably 11 or more, particularly preferably 12 or more, most preferably 13, continuous nucleic acid bases from the 3'-terminal of the antisense base sequences described in Table 1.

As the antisense oligonucleotide of the present invention, the antisense oligonucleotides (sequences shown in any of SEQ ID NOs: 400 - 680) described in Table 2-1 to 2-3, Table 3-1 to 3-2 or 4 are preferably used.

As the antisense oligonucleotide of the present invention, antisense oligonucleotides which are the antisense oligonucleotides described in Table 2-1 to 2-3, Table 3-1 to 3-2 and 4 and containing antisense oligonucleotide sequences having a β2GPI relative expression level of 0.5 or less(e.g., sequences shown in any of SEQ ID NOs: 407, 408, 415, 418, 424, 427 - 430, 437, 440, 443, 445 - 454, 456 - 460, 462, 463, 465, 470, 471, 481 - 484, 492, 505, 506, 508, 509, 511 - 519, 523, 524 - 527, 529, 531 - 533, 535 - 537, 539, 542, 546 - 548, 550 - 554, 558 - 560, 565 - 569, 574, 575, 577, 578, 582 - 597, 599 - 641, 643 - 680) are more preferably used. Further preferably, antisense oligonucleotide sequences having a β2GPI relative expression level of 0.3 or less(e.g., sequences shown in any of SEQ ID NOs: 445, 450, 456, 457, 459, 462, 471, 482, 509, 513, 514, 518, 523, 525, 526, 529, 553, 565, 566, 567, 584, 585, 586 - 597, 599, 600, 602 - 611, 613, 615 - 621, 624 - 630, 633 - 641, 643, 644, 645, 647, 648, 649, 651 - 657, 659 - 680), particularly preferably, antisense oligonucleotide sequences having a β2GPI relative expression level of 0.1 or less(e.g., sequences shown in any of SEQ ID NOs: 586, 587, 588, 590, 591, 592, 593, 602, 603, 604, 605, 608, 617, 618, 620, 624, 625, 627, 636, 640, 641, 652, 656, 659, 660, 661, 662, 663, 664, 665, 666, 667, 668, 669, 671, 672, 673, 675), can be used.

As the antisense oligonucleotide of the present invention, a nucleic acid containing a nucleic acid composed of a base sequence complementary to a part of the target base sequence of a β2GPI gene and suppressing the expression of β2GPI is used. In the nucleic acid, 1 - 3 bases, preferably 1 - 2 bases, more preferably 1 base, may be deleted, substituted or added.

When the antisense oligonucleotide of the present invention is intracellularly introduced, it binds to complementary mRNA or complementary mRNA precursor and sterically inhibits translation of the mRNA or mRNA precursor into proteins, whereby expression of the β2GPI gene can be suppressed.

In addition, the intracellularly-introduced antisense oligonucleotide of the present invention sometimes binds to complementary mRNA or complementary mRNA precursor in the cells and cleaves the mRNA or mRNA precursor. As an example thereof, an action through RNaseH, which is an endonuclease that degrades RNA strand of RNA and DNA double strand, is known. When the antisense oligonucleotide of the present invention forms a double strand with mRNA and mRNA precursor in the cell, the double strand is recognized by the endogenous RNaseH and complementary mRNA strand can be enzymatically degraded.

To induce cleavage of mRNA and mRNA precursor by RNaseH, an antisense oligonucleotide having a DNA region of continuous 4 - 80 nucleotides is preferable. In this case, the antisense oligonucleotide preferably has a 0 - 80%, more preferably 10 - 60%, further preferably 20 - 50%, sugar moiety-modified nucleotide. When a sugar moiety-modified nucleotide is present, the DNA region more preferably consists of continuous 4 - 20 nucleotides, further preferably continuous 4 - 15 nucleotides, most preferably continuous 5 - 10 nucleotides. Furthermore, the position of the sugar moiety-modified nucleotide in the antisense oligonucleotide of the present invention is preferably in the vicinity of 5'-terminal and/or 3'-terminal, more preferably at a position within 30% of the total length from the 5' end and/or a position within 30% of the total length from the 3'-terminal, further preferably at a position within 25% of the total length from the 5' end and/or a position within 25% of the total length from the 3'-terminal.

The 5'-terminal vicinity and/or 3'-terminal vicinity of the antisense oligonucleotide of the present invention are/is particularly preferably constituted of a sugar moiety-modified nucleotide. In the present specification, the 5'-terminal vicinity being constituted of a sugar moiety-modified nucleotide means that 1 - 4, preferably 2 - 4, continuous nucleotides from the 5'-terminal are sugar moiety-modified nucleotides, and the 3'-terminal vicinity being constituted of a sugar moiety-modified nucleotide means that 1 - 4, preferably 2 - 4, continuous nucleotides from the 3'-terminal are sugar moiety-modified nucleotides. That is, as the antisense oligonucleotide of the present invention, an antisense oligonucleotide in which 1 - 4 nucleotides from the 5'-terminal thereof are sugar moiety-modified nucleotides is preferably used, and an antisense oligonucleotide in which 2 - 4 nucleotides from the 5'-terminal thereof are sugar moiety-modified nucleotides is more preferably used. As the antisense oligonucleotide of the present invention, an antisense oligonucleotide in which 1 - 4 nucleotides from the 3'-terminal thereof are sugar moiety-modified nucleotides is preferably used, and an antisense oligonucleotide in which 2 - 4 nucleotides from the 3'-terminal thereof are sugar moiety-modified nucleotides is more preferably used.

In the present invention, the stringent conditions mean conditions under which the antisense oligonucleotide of the present invention hybridizes to the target base sequence of the β2GPI gene but does not hybridize to other sequences, and even if it hybridizes, the amount thereof is drastically smaller than the amount of hybridization to the target base sequence, and only a relatively negligible trace amount. Such conditions can be easily selected by changing the temperature during hybridization reaction and washing, salt concentration of hybridization reaction mixture and washings and the like. Specifically, one embodiment of the stringent conditions includes, but is not limited to, hybridizing in 6xSSC (0.9 M NaCl, 0.09 M trisodium citrate) or 6xSSPE (3 M NaCl, 0.2 M NaH₂PO₄, 20 mM EDTA.2Na, pH 7.4) at 42°C, and further washing with 0.5xSSC at 42°C. As the hybridization method, Southern blot hybridization method and the like can be used. Specifically, hybridization can be performed according to the methods described in Molecular Cloning: A Laboratory Manual, Second Edition (1989) (Cold Spring Harbor Laboratory Press), Current Protocols in Molecular Biology (1994) (Wiley-Interscience) and the like.

A method of producing the antisense oligonucleotide of the present invention is not particularly limited, and a method using a known chemical synthesis, or an enzymatic transcription method and the like can be mentioned. As a method using a known chemical synthesis, a phosphoramidite method, a phosphorothioate method, a phosphotriester method, a CEM method [Nucleic Acid Research, 35, 3287 (2007)] and the like can be mentioned and, for example, it can be synthesized by ABI3900 High Throughput nucleic acid synthesizer (manufactured by Applied Biosystems). After completion of the synthesis, desorption from a solid phase, removal of a protecting group, purification of the object product and the like are performed. It is desirable to obtain an antisense oligonucleotide having purity of 90% or more, preferably 95% or more, by purification. As an enzymatic transcription method for producing the antisense oligonucleotide of the present invention, a method using a plasmid or DNA having the object nucleotide sequence as a template, and including transcription using phage RNA polymerase, for example, T7, T3, or SP6RNA polymerase, can be mentioned.

The antisense oligonucleotide of the present invention can be introduced into a cell by using a carrier for transfection, preferably a cationic carrier such as cationic liposome and the like. Also, it can be directly introduced into a cell by a calcium phosphate method, an electroporation method, a microinjection method and the like.

It is also possible to induce suppression of the expression of the target gene by forming a double strand of the antisense oligonucleotide of the present invention and the complementary oligonucleic acid, and introducing the double strand nucleic acid into the cell (patent document WO 2005/113571). In this case, the position of modification of the double strand nucleic acid with a ligand is preferably the 5'-terminal or 3'-terminal of the complementary oligonucleic acid.

### 2. antisense oligonucleotide having expression suppressive activity on β2GPI

The antisense oligonucleotide of the present invention composed of a base sequence complementary to a part of the base sequence of β2GPI gene can be designed based on, for example, a cDNA base sequence (SEQ ID NO: 1) of the full length mRNA of human β2GPI registered as Genbank Accession No.NM_000042 or genomic sequence. The cDNA of the full length mRNA of human β2GPI is registered as, for example, Genbank Accession No.NM_000042, and a genomic sequence containing mRNA precursor of human β2GPI is registered as, for example, Genbank Accession No. NC_000017.11.

Of the nucleic acids composed of a base sequence complementary to a part of the target base sequence of the β2GPI gene, the antisense oligonucleotide having an expression suppress activity against β2GPI includes, for example, antisense oligonucleotides constituted of the base sequences selected from the groups described in Table 1 - Table 4. The length of the antisense oligonucleotides is 8 - 80 bases, and 8 - 30 bases are preferable. For example, 8 - 20 bases, 10 - 20 bases, 13 - 20 bases, 13 - 16 bases, 13 bases, 14 bases, 15 bases, 16 bases, 17 bases, 18bases, 19 bases, 20 bases.

The expression of β2GPI can be suppressed by introducing these antisense oligonucleotides into a cell. For example, the antisense oligonucleotide of the present invention introduced into a cell at a concentration of several nM - several µM can suppress expression of β2GPI when cultured for 24 hr or more, for example, 48 hr.

Furthermore, the evaluation of the expression suppressive activity of the antisense oligonucleotide of the present invention on β2GPI can be performed by introducing the antisense oligonucleotide by using a cationic liposome and the like, or the antisense oligonucleotide as it is, or the antisense oligonucleotide bound to a certain ligand, into human cell line and the like, culturing same for a given period, and quantifying the expression level of β2GPI mRNA in the human cell line.

The antisense oligonucleotide of the present invention may contain a ligand. The ligand may directly modify the 5'terminal, 3'-terminal and/or inside of sequence of the antisense oligonucleotide of the present invention.

While the ligand contained in the antisense oligonucleotide of the present invention may be a molecule having affinity for a biological molecule, for example, lipids such as cholesterol, fatty acid, tocopherol, retinoid and the like, saccharides such as N-acetylgalactosamine (GalNAc), galactose (Gal), mannose (Man) and the like, antibodies such as full antibody, Fab, scFv, VHH and the like, proteins such as low-density lipoprotein (LDL), human serum albumin and the like, peptides such as RGD, NGR, R9, CPP and the like, small molecules such as folic acid and the like, synthesis polymers such as synthetic polyamino acid and the like, nucleic acid aptamers and the like can be mentioned, but it is not limited to these and these can also be used in combination.

Examples of the method for adding a ligand to the antisense oligonucleotide of the present invention include, but are not limited to, reacting a modifier, capable of reaction on the solid phase, during an elongation reaction on the solid phase, whereby the 5-'terminal, 3'-terminal and/or inside of sequence can be modified. In addition, a conjugate nucleic acid can also be obtained by synthesizing and purifying in advance a nucleic acid introduced with a functional group such as amino group, mercapto group, azido group or triple bond and the like, and reacting same with a modifier.

### 3. pharmaceutical composition of the present invention

The present invention relates to a pharmaceutical composition containing the antisense oligonucleotide of the present invention as an active ingredient (to be also referred to as the pharmaceutical composition of the present invention in the present specification).

The pharmaceutical composition can further contain a carrier effective for intracellular transfer of the antisense oligonucleotide of the present invention. The pharmaceutical composition of the present invention can be used as a therapeutic or prophylactic agent for autoimmune diseases such as APS and SLE, and thrombosis in hemodialysis and the like.

Examples of the carrier effective for intracellular transfer of the antisense oligonucleotide of the present invention include cationic carriers. Examples of the cationic carrier include a cationic liposome, a cationic polymer and the like. As a carrier effective for intracellular transfer of the antisense oligonucleotide, a carrier utilizing a virus envelope may also be used. As a cationic liposome, a liposome containing 2-o-(2-diethylaminoethyl)carbamoyl-1,3-o-dioleoylglycerol (hereinafter to be also referred to as liposome A), oligofectamine (Invitrogen), Lipofectin (Invitrogen), lipofectamine (Invitrogen), lipofectamine2000 (Invitrogen), DMRIE-C (Invitrogen), GeneSilencer (Gene Therapy Systems), TransMessenger (QIAGEN), TransIT TKO (Mirus) and the like are preferably used. As a cationic polymer, JetSI (Qbiogene Inc.), Jet-PEI (polyethyleneimine; Qbiogene Inc.) and the like are preferably used. As a carrier utilizing a virus envelope, GenomeOne (HVJ-E liposome; ISHIHARA SANGYO KAISHA, LTD.) and the like are preferably used.

The pharmaceutical composition of the present invention comprising the antisense oligonucleotide of the present invention and the above-mentioned carrier can be prepared by a method known to those of ordinary skill in the art. For example, it can be prepared by mixing a carrier dispersion liquid and an antisense oligonucleotide solution at suitable concentrations. When a cationic carrier is used, it can be prepared easily by mixing in an aqueous solution by a conventional method, since antisense oligonucleotide has a negative electric charge in aqueous solutions. Examples of the aqueous solvent used for the preparation of the composition include electrolytic solutions such as water for injection, distilled water for injection, saline and the like, sugar solutions such as glucose solution, maltose solution and the like, and the like. The conditions such as pH and temperature and the like for preparation of the composition can be appropriately selected by those of ordinary skill in the art. In the case of liposome A, for example, the pharmaceutical composition can be prepared by gradually adding antisense oligonucleotide in 10% aqueous maltose solution to 16 mg/ml liposome dispersion in 10% aqueous maltose solution at pH 7.4, 25°C with stirring.

Where necessary, the composition can also be formed as a uniform composition by a dispersion treatment using an ultrasonic dispersion apparatus, a high-pressure emulsion apparatus and the like. Since the method and conditions optimal for the preparation of a composition comprising a carrier and an antisense oligonucleotide depend on the carrier to be used, those of ordinary skill in the art can select an optimal method for the carrier to be used irrespective of the above-mentioned methods.

As the pharmaceutical composition of the present invention, a liposome constituted of a composite particle comprising an antisense oligonucleotide and a lead particle as constituent components, and a lipid membrane covering the composite particle, wherein a liquid containing a polar organic solvent at a concentration at which the constituent components of the lipid membrane can be dispersed and the composite particles can be dispersed is present in a liquid containing the polar organic solvent in which the constituent components of the lipid membrane can be dissolved, can also be used preferably, though the pharmaceutical composition is not limited thereto. Examples of the lead particle include a lipid assembly, a liposome, an emulsion particle, a polymer, a metal colloid, a fine particle preparation and the like, and a liposome is preferably used. The lead particle in the present invention may contain a complex of a combination of two or more from a lipid assembly, a liposome, an emulsion particle, a polymer, a metal colloid, a fine particle preparation and the like as a constituent component, or a complex of a combination of a lipid assembly, a liposome, an emulsion particle, a polymer, a metal colloid, a fine particle preparation and the like and other compound (e.g., sugar, lipid, inorganic compound etc.) as a constituent component.

Examples of the lipid membrane covering the composite particle include those comprising, for example, neutral lipid and polyethylene glycol-phosphatidyl ethanolamine and the like as the constituent components.

The liposome can be prepared according to, for example, the method described in WO 2006/080118 and the like.

A suitable mixing ratio of the antisense oligonucleotide and the carrier comprised in the pharmaceutical composition of the present invention is 1 - 200 parts by weight of a carrier per 1 part by weight of antisense oligonucleotide. It is preferably 2.5 - 100 parts by weight, further preferably 10 - 20 parts by weight, of a carrier per 1 part by weight of antisense oligonucleotide.

The pharmaceutical composition of the present invention may also comprise a pharmaceutically acceptable carrier, a diluent and the like besides the above-mentioned carrier. A pharmaceutically acceptable carrier, a diluent and the like are essentially chemically-inactive and harmless compositions, and do not at all influence the biological activity of the pharmaceutical composition of the present invention. Examples of the carrier and diluent include, but are not limited to, a salt solution, a sugar solution, a glycerol solution, ethanol and the like.

The pharmaceutical composition of the present invention can be preferably used for the treatment or prophylaxis of diseases relating to the expression of β2GPI, particularly, disorders mediated by an anti-β2GPI antibody. In the present specification, the disorders mediated by an anti-β2GPI antibody refer to autoimmune diseases such as antiphospholipid antibody. syndrome (APS) and SLE, and thrombosis in hemodialysis. Therefore, the pharmaceutical composition of the present invention can be used as a therapeutic agent or a prophylaxis agent for autoimmune diseases such as APS and SLE, and thrombosis in hemodialysis.

The pharmaceutical composition of the present invention comprises the complex in an amount effective for the treatment or prevention of diseases and is provided in a form permitting appropriate administration to patients. The formulation of the pharmaceutical composition of the present invention may be, for example, a liquid such as injection, eye drop, inhalation and the like, for example, an external preparation such as ointment, lotion and the like, and the like.

In the case of a liquid, the concentration range of the pharmaceutical composition of the present invention is generally 0.001 - 25%(w/v), preferably 0.01 - 5%(w/v), more preferably 0.1 - 2%(w/v). The pharmaceutical composition of the present invention may comprise an adequate amount of any pharmaceutically acceptable additive, for example, an emulsion adjuvant, a stabilizer, an isotonicifier, a pH adjuster and the like. Any pharmaceutically acceptable additive can be added in a suitable step before or after dispersion of the complex.

The pharmaceutical composition of the present invention can also be provided as a freeze-dried preparation. A freeze-dried preparation can be prepared by a dispersion treatment of an antisense oligonucleotide and a carrier, followed by a freeze-drying treatment. A freeze-drying treatment can be performed by a conventional method. For example, a given amount of a complex solution after the above-mentioned dispersion treatment is dispensed in a vial container under sterile conditions, predried for about 2 hr under the condition of about -40°C to -20°C, primarily predried at about 0 - 10°C under reduced pressure, then secondarily dried at about 15 - 25°C under reduced pressure to perform freeze-drying. Then, for example, the inside of the vial is substituted with a nitrogen gas and a cap is provided, whereby a freeze-dried preparation of the pharmaceutical composition of the present invention can be obtained.

When the pharmaceutical composition of the present invention is provided as a freeze-dried preparation, the pharmaceutical composition of the present invention can be used by redissolving by the addition of any suitable solution. Examples of the solution include electrolytic solutions such as water for injection, saline and the like, glucose solution, other general infusions and the like. While the liquid volume of this solution varies depending on the use and the like and is not particularly limited, it is preferably a 0.5- to 2-fold amount of the liquid volume before freeze-drying, or 500 ml or less.

The pharmaceutical composition of the present invention can be administered to animals including human by, for example, intravenous administration, intraarterial administration, oral administration, tissue administration, transdermal administration, transmucosal administration or rectal administration, and is preferably administered by an appropriate method according to the symptom of the patient. Particularly, intravenous administration, transdermal administration, and transmucosal administration are preferably used. In addition, topical administration such as topical administration to a cancer site and the like can also be employed. Examples of the dosage form suitable for these administration methods include various injections, oral preparations, drip infusions, absorbents, eye drops, ointments, lotions, suppositories and the like.

While the dose of the pharmaceutical composition of the present invention is desirably determined in consideration of drug, dosage form, condition of patient such as age, body weight and the like, administration route, nature and severity of the disease and the like, it is generally 0.1 mg - 10 g/day, preferably 1 mg - 500 mg/day, for an adult in the mass of the antisense oligonucleotide. In some cases, a dose below these levels may be sufficient, or a dose above these levels may be conversely required. The pharmaceutical composition can be administered one to several times per day, or can be administered at one to several day intervals.

### 4. Treatment method

The present invention further provides a method of treating diseases related to the expression of β2GPI, particularly, disorders mediated by an anti-β2GPI antibody, comprising a step of administering a therapeutically effective amount of the antisense oligonucleotide of the present invention or the pharmaceutical composition of the present invention to a human in need of such treatment (treatment method of the present invention).

The treatment method of the present invention is preferably a method of treating autoimmune diseases or thrombosis, which is characterized by administering a therapeutically effective amount of the antisense oligonucleotide of the present invention or the pharmaceutical composition of the present invention to a human in need of such treatment. Other steps and conditions are not limited in any manner.

In the treatment method of the present invention, for example, the administration method, dose, preparation method and the like of the aforementioned pharmaceutical composition of the present invention can be used.

The present invention is explained in the following by referring to Examples, which are not to be construed as limitative.

### [Example 1]

### measurement of knockdown activity of β2GPI mRNA

Huh7 cell (obtained from National Institutes of Biomedical Innovation, Health and Nutrition JCRB Cell Bank), which is a cell line derived from human liver cancer, were seeded to a 96-well culture plate at 5,000 cells/80 µL/well. As a medium, MEM medium (manufactured by Life technologies, catalog No. 11095-098) containing 10% fetal bovine serum (FBS) was used. As the antisense oligonucleotides, those described in Table 2 - Table 4 were synthesized by GeneDesign, Inc. or Hokkaido System Science CO., Ltd and used. In the antisense oligonucleotides of Table 2, lower case letters indicate DNA and capital letters indicate 2'-O-methylated RNA. In the antisense oligonucleotides of Table 3 and Table 4, lower case letters indicate DNA, capital letters indicate LNA, and mC shows 5-methylcytosine LNA. In all antisense oligonucleotides of Table 2 - Table 4, the phosphoric acid diester bond in each nucleotide is substituted by a phosphorothioate bond. The antisense oligonucleotide and Lipofectamine LTX & Plus reagent (manufactured by Life technologies, catalog No.: 15338) were diluted with Opti-MEM medium (manufactured by Life technologies, catalog No. 11058-021), and 20 µL of each antisense oligonucleotide/Lipofectamine mixture was added to 96-well culture plate to the final concentration of antisense oligonucleotide of 100 nM, and the mixture was cultured under the conditions 37°C, 5% CO₂ for 24 hr. The cells were washed with PBS (phosphate buffered saline), and cDNA was synthesized from each plate by using Cells-to-Ct kit (manufactured by Applied Biosystems, catalog No.: AM1728) and according to the method described in the manual attached to the product. The cDNA (5 µL) was added to MicroAmp Optical 96-well plate (manufactured by Applied Biosystems, catalog No. 4326659), and 10 µL of TaqMan Gene Expression Master Mix (manufactured by Applied Biosystems, catalog No. 4369016), 3 µL of UltraPure Distilled Water (manufactured by Life technologies, catalog No.: 10977-015), 1 µL of human β2GPI probe, and 1 µL of human GAPDH probe were further added. The real-time PCR of human β2GPI gene and human GAPDH (D-glyceraldehyde-3-phosphate dehydrogenase) was performed by using the ABI7900 HT real-time PCR system. GAPDH is a constitutively expressed gene and was measured as the internal control, and the β2GPI expression level was normalized. The β2GPI mRNA relative expression level when each antisense oligonucleotide was introduced, was calculated relative to the β2GPI mRNA amount when Huh7 cells were treated with a transfection reagent alone without addition of antisense oligonucleotide as 1.0. This experiment was performed 2 times and the mean of the β2GPI mRNA relative expression level is shown in Tables 2 to 4.

**[Table 2-1]**

| SEQ ID NO: | antisense base sequence | SEQ ID NO: | antisense oligonucleotide (5'→3') | β2GPI relative expression level |
|---|---|---|---|---|
| 2 | gagcactggagaa | 400 | GAgcactggaGAA | 0.570 |
| 3 | tgagcactggaga | 401 | UGagcactggAGA | 0.652 |
| 4 | agatgagcactgg | 402 | AGatgagcacUGG | 0.652 |
| 5 | agcaacatggcag | 403 | AGcaacatggCAG | 0.660 |
| 6 | tagcaacatggca | 404 | UAgcaacatgGCA | 0.697 |
| 7 | atagcaacatggc | 405 | AUagcaacatGGC | 0.648 |
| 12 | gtcctgcaatagc | 406 | GUcctgcaatAGC | 0.528 |
| 14 | gtccgtcctgcaa | 407 | GUccgtcctgCAA | 0.388 |
| 15 | ggtccgtcctgca | 408 | GGtccgtcctGCA | 0.447 |
| 16 | aggtccgtcctgc | 409 | AGgtccgtccUGC | 0.517 |
| 17 | caggtccgtcctg | 410 | CAggtccgtcCUG | 0.627 |
| 18 | tgggacaggtccg | 411 | UGggacaggtCCG | 0.583 |
| 19 | ttgggacaggtcc | 412 | UUgggacaggUCC | 0.538 |
| 20 | ggcttgggacagg | 413 | GGcttgggacAGG | 0.691 |
| 21 | tggcttgggacag | 414 | UGgcttgggaCAG | 0.555 |
| 22 | ctggcttgggaca | 415 | CUggcttgggACA | 0.412 |
| 23 | tctggcttgggac | 416 | UCtggcttggGAC | 0.583 |
| 24 | catctggcttggg | 417 | CAtctggcttGGG | 0.663 |
| 25 | tcatctggcttgg | 418 | UCatctggctUGG | 0.373 |
| 26 | atcatctggcttg | 419 | AUcatctggcUUG | 0.537 |
| 27 | aatcatctggctt | 420 | AAtcatctggCUU | 0.621 |
| 28 | aaatcatctggct | 421 | AAatcatctgGCU | 0.681 |
| 29 | tcctggctcatag | 422 | UCctggctcaUAG | 0.601 |
| 30 | ctcctggctcata | 423 | CUcctggctcAUA | 0.537 |
| 31 | tctcctggctcat | 424 | UCtcctggctCAU | 0.402 |
| 32 | ccggcttgcagga | 425 | CCggcttgcaGGA | 0.567 |
| 33 | cccggcttgcagg | 426 | CCcggcttgcAGG | 0.581 |
| 34 | gcccggcttgcag | 427 | GCccggcttgCAG | 0.477 |
| 35 | agcccggcttgca | 428 | AGcccggcttGCA | 0.325 |
| 36 | tagcccggcttgc | 429 | UAgcccggctUGC | 0.310 |
| 37 | atagcccggcttg | 430 | AUagcccggcUUG | 0.402 |
| 38 | catagcccggctt | 431 | CAtagcccggCUU | 0.583 |
| 39 | acacatagcccgg | 432 | ACacatagccCGG | 0.629 |
| 40 | gacacatagcccg | 433 | GAcacatagcCCG | 0.687 |
| 41 | tgtgagagggcag | 434 | UGtgagagggCAG | 0.652 |
| 42 | ttgatgggccaca | 435 | UUgatgggccACA | 0.572 |
| 43 | gttgatgggccac | 436 | GUtgatgggcCAC | 0.606 |
| 44 | tgttgatgggcca | 437 | UGttgatgggCCA | 0.479 |
| 45 | gtgttgatgggcc | 438 | GUgttgatggGCC | 0.535 |
| 46 | ggacatactctgg | 439 | GGacatactcUGG | 0.505 |
| 47 | aggacatactctg | 440 | AGgacatactCUG | 0.487 |
| 50 | gctccattttcta | 441 | GCtccattttCUA | 0.606 |
| 54 | gttgggatattca | 442 | GUtgggatatUCA | 0.663 |
| 55 | gcacttggcagaa | 443 | GCacttggcaGAA | 0.499 |
| 56 | tgcacttggcaga | 444 | UGcacttggcAGA | 0.517 |
| 57 | gtgcacttggcag | 445 | GUgcacttggCAG | 0.281 |
| 58 | agtgcacttggca | 446 | AGtgcacttgGCA | 0.482 |
| 59 | cagtgcacttggc | 447 | CAgtgcacttGGC | 0.480 |
| 60 | ctcagtgcacttg | 448 | CUcagtgcacUUG | 0.456 |
| 61 | cctcagtgcactt | 449 | CCtcagtgcaCUU | 0.387 |
| 62 | tcctcagtgcact | 450 | UCctcagtgcACU | 0.226 |
| 63 | ttcctcagtgcac | 451 | UUcctcagtgCAC | 0.334 |
| 64 | cttcctcagtgca | 452 | CUtcctcagtGCA | 0.364 |
| 65 | ccttcctcagtgc | 453 | CCttcctcagUGC | 0.415 |
| 66 | tccttcctcagtg | 454 | UCcttcctcaGUG | 0.425 |
| 67 | gctgatggcttat | 455 | GCtgatggctUAU | 0.685 |
| 68 | agctgatggctta | 456 | AGctgatggcUUA | 0.275 |
| 69 | cagctgatggctt | 457 | CAgctgatggCUU | 0.225 |
| 70 | ccagctgatggct | 458 | CCagctgatgGCU | 0.421 |
| 71 | tccagctgatggc | 459 | UCcagctgatGGC | 0.217 |
| 72 | ttccagctgatgg | 460 | UUccagctgaUGG | 0.454 |
| 73 | gtttccagctgat | 461 | GUttccagctGAU | 0.535 |
| 74 | tgtttccagctga | 462 | UGtttccagcUGA | 0.249 |

**[Table 2-2]**

| SEQ ID NO: | antisense base sequence | SEQ ID NO: | antisense oligonucleotide (5' →3') | β2GPI relative expression level |
|---|---|---|---|---|
| 75 | ttgtttccagctg | 463 | UUgtttccagCUG | 0.302 |
| 76 | aactgctgtgtcc | 464 | AActgctgtgUCC | 0.546 |
| 77 | aaaaactgctgtg | 465 | AAaaactgctGUG | 0.475 |
| 78 | tggcaaacattca | 466 | UGgcaaacatUCA | 0.581 |
| 79 | gtggcaaacattc | 467 | GUggcaaacaUUC | 0.660 |
| 80 | gttgtggcaaaca | 468 | GUtgtggcaaACA | 0.654 |
| 81 | tgttgtggcaaac | 469 | UGttgtggcaAAC | 0.676 |
| 82 | gcatgttgtggca | 470 | GCatgttgtgGCA | 0.408 |
| 83 | cgcatgttgtggc | 471 | CGcatgttgtGGC | 0.243 |
| 84 | tcgcatgttgtgg | 472 | UCgcatgttgUGG | 0.613 |
| 85 | atcgcatgttgtg | 473 | AUcgcatgttGUG | 0.685 |
| 86 | acatcgcatgttg | 474 | ACatcgcatgUUG | 0.683 |
| 87 | ttccaaacatcgc | 475 | UUccaaacatCGC | 0.576 |
| 88 | agtccaatttcca | 476 | AGtccaatttCCA | 0.626 |
| 89 | ccctgcattctgg | 477 | CCctgcattcUGG | 0.583 |
| 90 | tccctgcattctg | 478 | UCcctgcattCUG | 0.548 |
| 91 | ttccctgcattct | 479 | UUccctgcatUCU | 0.683 |
| 92 | attgtctggtctt | 480 | AUtgtctggtCUU | 0.562 |
| 93 | cattgtctggtct | 481 | CAttgtctggUCU | 0.356 |
| 94 | ccattgtctggtc | 482 | CCattgtctgGUC | 0.253 |
| 95 | tccattgtctggt | 483 | UCcattgtctGGU | 0.345 |
| 96 | atccattgtctgg | 484 | AUccattgtcUGG | 0.334 |
| 97 | aatccattgtctg | 485 | AAtccattgtCUG | 0.669 |
| 98 | gcaggatagttca | 486 | GCaggatagtUCA | 0.550 |
| 99 | ggttttgcaggat | 487 | GGttttgcagGAU | 0.562 |
| 100 | tggttttgcagga | 488 | UGgttttgcaGGA | 0.537 |
| 101 | ggctttatccttg | 489 | GGctttatccUUG | 0.610 |
| 102 | gtggctttatcct | 490 | GUggctttatCCU | 0.672 |
| 106 | catcatggcagcc | 491 | CAtcatggcaGCC | 0.688 |
| 107 | ccatcatggcagc | 492 | CCatcatggcAGC | 0.427 |
| 108 | tccatcatggcag | 493 | UCcatcatggCAG | 0.691 |
| 109 | atccatcatggca | 494 | AUccatcatgGCA | 0.614 |
| 110 | tatccatcatggc | 495 | UAtccatcatGGC | 0.407 |
| 111 | atatccatcatgg | 496 | AUatccatcaUGG | 0.607 |
| 112 | cccagtttggtac | 497 | CCcagtttggUAC | 0.378 |
| 113 | tcccagtttggta | 498 | UCccagtttgGUA | 0.637 |
| 114 | ttcccagtttggt | 499 | UUcccagtttGGU | 0.512 |
| 115 | tttcccagtttgg | 500 | UUtcccagttUGG | 0.632 |
| 116 | gtttcccagtttg | 501 | GUttcccagtUUG | 0.518 |
| 117 | cagtttcccagtt | 502 | CAgtttcccaGUU | 0.581 |
| 118 | ccagtttcccagt | 503 | CCagtttcccAGU | 0.548 |
| 119 | accagtttcccag | 504 | ACcagtttccCAG | 0.631 |
| 120 | gaccagtttccca | 505 | GAccagtttcCCA | 0.313 |
| 121 | agaccagtttccc | 506 | AGaccagtttCCC | 0.405 |
| 122 | gcagaccagtttc | 507 | GCagaccagtUUC | 0.694 |
| 123 | ggcagaccagttt | 508 | GGcagaccagUUU | 0.399 |
| 124 | tggcagaccagtt | 509 | UGgcagaccaGUU | 0.219 |
| 125 | atggcagaccagt | 510 | AUggcagaccAGU | 0.518 |
| 126 | catggcagaccag | 511 | CAtggcagacCAG | 0.426 |
| 127 | gcatggcagacca | 512 | GCatggcagaCCA | 0.374 |
| 128 | ggcatggcagacc | 513 | GGcatggcagACC | 0.246 |
| 129 | tggcatggcagac | 514 | UGgcatggcaGAC | 0.203 |
| 130 | ttggcatggcaga | 515 | UUggcatggcAGA | 0.479 |
| 131 | cttggcatggcag | 516 | CUtggcatggCAG | 0.305 |
| 132 | acttggcatggca | 517 | ACttggcatgGCA | 0.356 |
| 133 | aacttggcatggc | 518 | AActtggcatGGC | 0.221 |
| 134 | caacttggcatgg | 519 | CAacttggcaUGG | 0.465 |
| 135 | acaacttggcatg | 520 | ACaacttggcAUG | 0.679 |
| 136 | gctttacaacttg | 521 | GCtttacaacUUG | 0.598 |
| 137 | tcacaggtacttt | 522 | UCacaggtacUUU | 0.666 |
| 138 | acaccacagtggc | 523 | ACaccacagtGGC | 0.158 |
| 139 | gtacaccacagtg | 524 | GUacaccacaGUG | 0.478 |

**[Table 2-3]**

| SEQ ID NO: | antisense base sequence | SEQ ID NO: | antisense oligonucleotide (5'→3') | β2GPI relative expression level |
|---|---|---|---|---|
| 140 | ggtacaccacagt | 525 | GGtacaccacAGU | 0.267 |
| 141 | tggtacaccacag | 526 | UGgtacaccaCAG | 0.235 |
| 142 | ttggtacaccaca | 527 | UUggtacaccACA | 0.370 |
| 143 | cttggtacaccac | 528 | CUtggtacacCAC | 0.607 |
| 144 | ccttggtacacca | 529 | CCttggtacaCCA | 0.271 |
| 145 | tccttggtacacc | 530 | UCcttggtacACC | 0.544 |
| 146 | ctccttggtacac | 531 | CUccttggtaCAC | 0.325 |
| 147 | tctccttggtaca | 532 | UCtccttggtACA | 0.496 |
| 148 | ctctccttggtac | 533 | CUctccttggUAC | 0.340 |
| 149 | tctctccttggta | 534 | UCtctccttgGUA | 0.624 |
| 150 | ctctctccttggt | 535 | CUctctccttGGU | 0.397 |
| 151 | tctctctccttgg | 536 | UCtctctcctUGG | 0.309 |
| 152 | ctctctctccttg | 537 | CUctctctccUUG | 0.372 |
| 153 | actctctctcctt | 538 | ACtctctctcCUU | 0.526 |
| 154 | tactctctctcct | 539 | UActctctctCCU | 0.489 |
| 155 | ttactctctctcc | 540 | UUactctctcUCC | 0.604 |
| 156 | gcattccattctt | 541 | GCattccattCUU | 0.564 |
| 157 | agcattccattct | 542 | AGcattccatUCU | 0.342 |
| 158 | agctacacttctt | 543 | AGctacacttCUU | 0.507 |
| 159 | tagctacacttct | 544 | UAgctacactUCU | 0.647 |
| 160 | ttgaagcatttgg | 545 | UUgaagcattUGG | 0.697 |
| 161 | gttccttgaagca | 546 | GUtccttgaaGCA | 0.332 |
| 162 | tgttccttgaagc | 547 | UGttccttgaAGC | 0.324 |
| 163 | gtgttccttgaag | 548 | GUgttccttgAAG | 0.496 |
| 164 | tgtgttccttgaa | 549 | UGtgttccttGAA | 0.511 |
| 165 | ctgtgttccttga | 550 | CUgtgttcctUGA | 0.304 |
| 166 | actgtgttccttg | 551 | ACtgtgttccUUG | 0.353 |
| 167 | aactgtgttcctt | 552 | AActgtgttcCUU | 0.449 |
| 168 | gaactgtgttcct | 553 | GAactgtgttCCU | 0.262 |
| 169 | agaactgtgttcc | 554 | AGaactgtgtUCC | 0.416 |
| 170 | gagaactgtgttc | 555 | GAgaactgtgUUC | 0.588 |
| 171 | agagaactgtgtt | 556 | AGagaactgtGUU | 0.623 |
| 172 | cagagaactgtgt | 557 | CAgagaactgUGU | 0.569 |
| 173 | ccagagaactgtg | 558 | CCagagaactGUG | 0.428 |
| 174 | gccagagaactgt | 559 | GCcagagaacUGU | 0.457 |
| 175 | agccagagaactg | 560 | AGccagagaaCUG | 0.314 |
| 176 | aagccagagaact | 561 | AAgccagagaACU | 0.611 |
| 177 | gcatcagttttcc | 562 | GCatcagtttUCC | 0.686 |
| 178 | tgcatcagttttc | 563 | UGcatcagttUUC | 0.693 |
| 179 | gatgcatcagttt | 564 | GAtgcatcagUUU | 0.534 |
| 180 | ggatgcatcagtt | 565 | GGatgcatcaGUU | 0.134 |
| 181 | cggatgcatcagt | 566 | CGgatgcatcAGU | 0.188 |
| 182 | tcggatgcatcag | 567 | UCggatgcatCAG | 0.227 |
| 183 | atcggatgcatca | 568 | AUcggatgcaUCA | 0.386 |
| 184 | catcggatgcatc | 569 | CAtcggatgcAUC | 0.497 |
| 185 | acatcggatgcat | 570 | ACatcggatgCAU | 0.528 |
| 186 | tacatcggatgca | 571 | UAcatcggatGCA | 0.594 |
| 187 | ttacatcggatgc | 572 | UUacatcggaUGC | 0.594 |
| 188 | gctttacatcgga | 573 | GCtttacatcGGA | 0.626 |
| 189 | ggctttacatcgg | 574 | GGctttacatCGG | 0.359 |
| 190 | tggctttacatcg | 575 | UGgctttacaUCG | 0.411 |
| 191 | tgtggaatctgaa | 576 | UGtggaatctGAA | 0.671 |
| 192 | agtgtgacatttt | 577 | AGtgtgacatUUU | 0.428 |
| 193 | aagtgtgacattt | 578 | AAgtgtgacaUUU | 0.440 |
| 194 | ccttggatgaaca | 579 | CCttggatgaACA | 0.690 |
| 195 | tccttggatgaac | 580 | UCcttggatgAAC | 0.669 |
| 196 | gttccttggatga | 581 | GUtccttggaUGA | 0.558 |
| 197 | ggttccttggatg | 582 | GGttccttggAUG | 0.432 |
| 198 | aggttccttggat | 583 | AGgttccttgGAU | 0.354 |
| 199 | taggttccttgga | 584 | UAggttccttGGA | 0.278 |
| 200 | ttaggttccttgg | 585 | UUaggttcctUGG | 0.278 |

**[Table 3-1]**

| SEQ ID NO: | antisense base sequence | SEQ ID NO: | antisense oligonucleotide (5'→3') | β2GPI relative expression level |
|---|---|---|---|---|
| 6 | tagcaacatggca | 586 | TAgcaacatgGmCA | 0.033 |
| 7 | atagcaacatggc | 587 | ATagcaacatGGmC | 0.067 |
| 8 | caatagcaacatg | 588 | mCAatagcaacATG | 0.065 |
| 9 | gcaatagcaacat | 589 | GmCaatagcaamCAT | 0.220 |
| 10 | cctgcaatagcaa | 590 | mCmCtgcaatagmCAA | 0.093 |
| 11 | tcctgcaatagca | 591 | TmCctgcaataGmCA | 0.043 |
| 12 | gtcctgcaatagc | 592 | GTcctgcaatAGmC | 0.025 |
| 13 | tccgtcctgcaat | 593 | TmCcgtcctgcAAT | 0.082 |
| 14 | gtccgtcctgcaa | 594 | GTccgtcctgmCAA | 0.161 |
| 15 | ggtccgtcctgca | 595 | GGtccgtcctGmCA | 0.141 |
| 22 | ctggcttgggaca | 596 | mCTggcttgggAmCA | 0.169 |
| 25 | tcatctggcttgg | 597 | TmCatctggctTGG | 0.134 |
| 31 | tctcctggctcat | 598 | TmCtcctggctmCAT | 0.594 |
| 35 | agcccggcttgca | 599 | AGcccggcttGmCA | 0.295 |
| 36 | tagcccggcttgc | 600 | TAgcccggctTGmC | 0.231 |
| 37 | atagcccggcttg | 601 | ATagcccggcTTG | 0.357 |
| 45 | gtgttgatgggcc | 602 | GTgttgatggGmCmC | 0.058 |
| 46 | ggacatactctgg | 603 | GGacatactcTGG | 0.009 |
| 48 | aaggacatactct | 604 | AAggacatacTmCT | 0.020 |
| 49 | aaaggacatactc | 605 | AAaggacatamCTmC | 0.012 |
| 51 | gggatattcaaaa | 606 | GGgatattcaAAA | 0.294 |
| 52 | tgggatattcaaa | 607 | TGggatattcAAA | 0.104 |
| 53 | ttgggatattcaa | 608 | TTgggatattmCAA | 0.025 |
| 57 | gtgcacttggcag | 609 | GTgcacttggmCAG | 0.212 |
| 61 | cctcagtgcactt | 610 | mCmCtcagtgcamCTT | 0.201 |
| 62 | tcctcagtgcact | 611 | TmCctcagtgcAmCT | 0.122 |
| 65 | ccttcctcagtgc | 612 | mCmCttcctcagTGmC | 0.369 |
| 68 | agctgatggctta | 613 | AGctgatggcTTA | 0.193 |
| 69 | cagctgatggctt | 614 | mCAgctgatggmCTT | 0.448 |
| 70 | ccagctgatggct | 615 | mCmCagctgatgGmCT | 0.148 |
| 71 | tccagctgatggc | 616 | TmCcagctgatGGmC | 0.289 |
| 74 | tgtttccagctga | 617 | TGtttccagcTGA | 0.084 |
| 75 | ttgtttccagctg | 618 | TTgtttccagmCTG | 0.042 |
| 82 | gcatgttgtggca | 619 | GmCatgttgtgGmCA | 0.218 |
| 83 | cgcatgttgtggc | 620 | mCGcatgttgtGGmC | 0.053 |
| 93 | cattgtctggtct | 621 | mCAttgtctggTmCT | 0.217 |
| 94 | ccattgtctggtc | 622 | mCmCattgtctgGTmC | 0.324 |
| 95 | tccattgtctggt | 623 | TmCcattgtctGGT | 0.422 |
| 96 | atccattgtctgg | 624 | ATccattgtcTGG | 0.079 |
| 103 | atgtggctttatc | 625 | ATgtggctttATmC | 0.074 |
| 104 | aatgtggctttat | 626 | AAtgtggcttTAT | 0.111 |
| 105 | ccaaatgtggctt | 627 | mCmCaaatgtggmCTT | 0.019 |
| 107 | ccatcatggcagc | 628 | mCmCatcatggcAGmC | 0.129 |
| 110 | tatccatcatggc | 629 | TAtccatcatGGmC | 0.204 |
| 112 | cccagtttggtac | 630 | mCmCcagtttggTAmC | 0.210 |
| 120 | gaccagtttccca | 631 | GAccagtttcmCmCA | 0.414 |
| 121 | agaccagtttccc | 632 | AGaccagtttmCmCmC | 0.436 |
| 123 | ggcagaccagttt | 633 | GGcagaccagTTT | 0.138 |
| 124 | tggcagaccagtt | 634 | TGgcagaccaGTT | 0.187 |
| 126 | catggcagaccag | 635 | mCAtggcagacmCAG | 0.134 |
| 127 | gcatggcagacca | 636 | GmCatggcagamCmCA | 0.091 |
| 128 | ggcatggcagacc | 637 | GGcatggcagAmCmC | 0.163 |
| 129 | tggcatggcagac | 638 | TGgcatggcaGAmC | 0.265 |
| 131 | cttggcatggcag | 639 | mCTtggcatggmCAG | 0.134 |
| 132 | acttggcatggca | 640 | AmCttggcatgGmCA | 0.086 |
| 133 | aacttggcatggc | 641 | AActtggcatGGmC | 0.061 |
| 138 | acaccacagtggc | 642 | AmCaccacagtGGmC | 0.501 |
| 140 | ggtacaccacagt | 643 | GGtacaccacAGT | 0.137 |
| 144 | ccttggtacacca | 644 | mCmCttggtacamCmCA | 0.265 |
| 145 | tccttggtacacc | 645 | TmCcttggtacAmCmC | 0.232 |
| 146 | ctccttggtacac | 646 | mCTccttggtamCAmC | 0.493 |
| 148 | ctctccttggtac | 647 | mCTctccttggTAmC | 0.119 |
| 150 | ctctctccttggt | 648 | mCTctctccttGGT | 0.250 |

**[Table 3-2]**

| SEQ ID NO: | antisense base sequence | SEQ ID NO: | antisense oligonucleotide (5'→3') | β2GPI relative expression level |
|---|---|---|---|---|
| 151 | tctctctccttgg | 649 | TmCtctctcctTGG | 0.196 |
| 152 | ctctctctccttg | 650 | mCTctctctccTTG | 0.456 |
| 157 | agcattccattct | 651 | AGcattccatTmCT | 0.283 |
| 161 | gttccttgaagca | 652 | GTtccttgaaGmCA | 0.067 |
| 162 | tgttccttgaagc | 653 | TGttccttgaAGmC | 0.112 |
| 164 | tgtgttccttgaa | 654 | TGtgttccttGAA | 0.271 |
| 165 | ctgtgttccttga | 655 | mCTgtgttcctTGA | 0.148 |
| 166 | actgtgttccttg | 656 | AmCtgtgttccTTG | 0.081 |
| 168 | gaactgtgttcct | 657 | GAactgtgttmCmCT | 0.218 |
| 169 | agaactgtgttcc | 658 | AGaactgtgtTmCmC | 0.361 |
| 174 | gccagagaactgt | 659 | GmCcagagaacTGT | 0.007 |
| 175 | agccagagaactg | 660 | AGccagagaamCTG | 0.013 |
| 179 | gatgcatcagttt | 661 | GAtgcatcagTTT | 0.084 |
| 180 | ggatgcatcagtt | 662 | GGatgcatcaGTT | 0.052 |
| 181 | cggatgcatcagt | 663 | mCGgatgcatcAGT | 0.061 |
| 182 | tcggatgcatcag | 664 | TmCggatgcatmCAG | 0.072 |
| 183 | atcggatgcatca | 665 | ATcggatgcaTmCA | 0.054 |
| 189 | ggctttacatcgg | 666 | GGctttacatmCGG | 0.021 |
| 192 | agtgtgacatttt | 667 | AGtgtgacatTTT | 0.061 |
| 193 | aagtgtgacattt | 668 | AAgtgtgacaTTT | 0.035 |
| 197 | ggttccttggatg | 669 | GGttccttggATG | 0.036 |
| 198 | aggttccttggat | 670 | AGgttccttgGAT | 0.109 |
| 199 | taggttccttgga | 671 | TAggttccttGGA | 0.026 |
| 200 | ttaggttccttgg | 672 | TTaggttcctTGG | 0.010 |

**[Table 4]**

| SEQ ID NO: | antisense oligonucleotide (5'→3') | β2GPI relative expression level |
|---|---|---|
| 673 | GGacatactcTGG | 0.070 |
| 674 | GGAcatactcTGG | 0.249 |
| 675 | AGGacatactcTGG | 0.069 |
| 676 | AAGgacatactcTGG | 0.141 |
| 677 | AAGgacatactmCTGG | 0.107 |
| 678 | AAGGacatactcTGG | 0.205 |
| 679 | AAGGacatactmCTGG | 0.219 |
| 680 | AAAGgacatactmCTGG | 0.121 |

### [Industrial Applicability]

The present invention provides an antisense oligonucleotide having activity to suppress expression of β2GPI, a pharmaceutical composition comprising the antisense oligonucleotide as an active ingredient, and the like. The antisense oligonucleotide and pharmaceutical composition of the present invention suppress expression of β2GPI, and are useful for the prophylaxis or treatment of autoimmune diseases such as APS, SLE and the like and thrombosis in hemodialysis.

The contents disclosed in any publication stated in the present specification, including patents, patent applications and scientific literatures, are hereby incorporated in their entireties by reference, to the extent that they have been disclosed herein.

This application is based on a patent application No. 2015-140081 filed in Japan (filing date: July 13, 2015), the contents of which are incorporated in full herein.

## Claims

1. An antisense oligonucleotide consisting of 8 - 80 bases in length that suppresses an expression of β2GPI, comprising a sequence hybridizable to a nucleic acid consisting of a base sequence shown in any of SEQ ID NOs: 201 - 399 under stringent conditions.

2. An antisense oligonucleotide consisting of 8 - 80 bases in length that suppresses an expression of β2GPI, comprising at least 8 continuous bases in a base sequence shown in any of SEQ ID NOs: 2 - 200.

3. An antisense oligonucleotide consisting of 8 - 80 bases in length and complementary to a base sequence shown in any of SEQ ID NOs: 201 - 399.

4. The antisense oligonucleotide according to claim 3, comprising a base sequence shown in any of SEQ ID NOs: 2 - 200.

5. The antisense oligonucleotide according to claim 3, comprising a base sequence shown in any of SEQ ID NOs: 2 - 200 wherein 1 or several bases are deleted, substituted or added.

6. An antisense oligonucleotide consisting of a base sequence shown in any of SEQ ID NOs: 2 - 200.

7. An antisense oligonucleotide consisting of a base sequence shown in any of SEQ ID NOs: 400 - 680.

8. The antisense oligonucleotide according to any one of claims 1 to 7, wherein the 5'-terminal vicinity and/or the 3'-terminal vicinity are/is constituted of a sugar moiety-modified nucleotide.

9. The antisense oligonucleotide according to any one of claims 1 to 8, comprising a ligand.

10. A pharmaceutical composition comprising the antisense oligonucleotide according to any one of claims 1 to 9.

11. The pharmaceutical composition according to claim 10 for the treatment or prophylaxis of an autoimmune disease or thrombosis.

12. A method of treating a disorder mediated by an anti-β2GPI antibody, comprising a step of administering a therapeutically effective amount of the antisense oligonucleotide according to any one of claims 1 to 9 or the pharmaceutical composition according to claim 10 or 11 to a human in need of such treatment.

13. The method according to claim 12, wherein the aforementioned disorder is an autoimmune disease or thrombosis.

14. Use of the antisense oligonucleotide according to any one of claims 1 to 9 for the manufacture of a pharmaceutical composition for preventing or treating a disorder mediated by an anti-β2GPI antibody.

15. The use according to claim 14, wherein the aforementioned disorder is an autoimmune disease or thrombosis.

16. The antisense oligonucleotide according to any one of claims 1 to 9 for use in the prophylaxis or treatment of a disorder mediated by an anti-β2GPI antibody.

17. The antisense nucleotide according to claim 16, wherein the aforementioned disorder is an autoimmune disease or thrombosis.


SEQUENCE LISTING

<110>  KYOWA HAKKO KIRIN CO., LTD

<120>  Antisense Oligonucleotide for Inhibiting Expression of beta2GPI

<130>  092502

<150>  JP 2015-140081
<151>  2015-07-13

<160>  680

<170>  PatentIn version 3.5

<210>  1
<211>  1216
<212>  DNA
<213>  Homo sapiens

<400>  1
ggctctgtct ttttagcaga cgaaaaccac tttggtagtg ccagtgtgac tcatccacaa      60

tgatttctcc agtgctcatc ttgttctcga gttttctctg ccatgttgct attgcaggac     120

ggacctgtcc caagccagat gatttaccat tttccacagt ggtcccgtta aaaacattct     180

atgagccagg agaagagatt acgtattcct gcaagccggg ctatgtgtcc cgaggaggga     240

tgagaaagtt tatctgccct ctcacaggac tgtggcccat caacactctg aaatgtacac     300

ccagagtatg tccttttgct ggaatcttag aaaatggagc cgtacgctat acgacttttg     360

aatatcccaa cacgatcagt ttttcttgta acactgggtt ttatctgaat ggcgctgatt     420

ctgccaagtg cactgaggaa ggaaaatgga gcccggagct tcctgtctgt gctcccatca     480

tctgccctcc accatccata cctacgtttg caacacttcg tgtttataag ccatcagctg     540

gaaacaattc cctctatcgg gacacagcag tttttgaatg tttgccacaa catgcgatgt     600

ttggaaatga tacaattacc tgcacgacac atggaaattg gactaaatta ccagaatgca     660

gggaagtaaa atgcccattc ccatcaagac cagacaatgg atttgtgaac tatcctgcaa     720

aaccaacact ttattacaag gataaagcca catttggctg ccatgatgga tattctctgg     780

atggcccgga agaaatagaa tgtaccaaac tgggaaactg gtctgccatg ccaagttgta     840

aagcatcttg taaagtacct gtgaaaaaag ccactgtggt gtaccaagga gagagagtaa     900

agattcagga aaaatttaag aatggaatgc tacatggtga taaagtttct ttcttctgca     960

aaaataagga aaagaagtgt agctatacag aggatgctca gtgtatagat ggcactatcg    1020

aagtccccaa atgcttcaag gaacacagtt ctctggcttt ttggaaaact gatgcatccg    1080

atgtaaagcc atgctaaggt ggttttcaga ttccacacaa aatgtcacac ttgtttcttg    1140

ttcatccaag gaacctaatt gaaatttaaa aataaagcta ctgaatttat tgccgcaccc    1200

aaaaaaaaaa aaaaaa                                                    1216

<210>  2
<211>  13
<212>  DNA
<213>  Artificial Sequence

<220>
<223>  Synthetic oligonucleotide

<400>  2
gagcactgga gaa                                                          13


<210>  3
<211>  13
<212>  DNA
<213>  Artificial Sequence

<220>
<223>  Synthetic oligonucleotide

<400>  3
tgagcactgg aga                                                          13


<210>  4
<211>  13
<212>  DNA
<213>  Artificial Sequence

<220>
<223>  Synthetic oligonucleotide

<400>  4
agatgagcac tgg                                                          13


<210>  5
<211>  13
<212>  DNA
<213>  Artificial Sequence

<220>
<223>  Synthetic oligonucleotide

<400>  5
agcaacatgg cag                                                          13


<210>  6
<211>  13
<212>  DNA
<213>  Artificial Sequence

<220>
<223>  Synthetic oligonucleotide

<400>  6
tagcaacatg gca                                                          13


<210>  7
<211>  13

<212>  DNA
<213>  Artificial Sequence

<220>
<223>  Synthetic oligonucleotide

<400>  7
atagcaacat ggc                                                          13


<210>  8
<211>  13
<212>  DNA
<213>  Artificial Sequence

<220>
<223>  Synthetic oligonucleotide

<400>  8
caatagcaac atg                                                          13


<210>  9
<211>  13
<212>  DNA
<213>  Artificial Sequence

<220>
<223>  Synthetic oligonucleotide

<400>  9
gcaatagcaa cat                                                          13


<210>  10
<211>  13
<212>  DNA
<213>  Artificial Sequence

<220>
<223>  Synthetic oligonucleotide

<400>  10
cctgcaatag caa                                                          13


<210>  11
<211>  13
<212>  DNA
<213>  Artificial Sequence

<220>
<223>  Synthetic oligonucleotide

<400>  11
tcctgcaata gca                                                          13


<210>  12
<211>  13
<212>  DNA
<213>  Artificial Sequence

<220>
<223>  Synthetic oligonucleotide

<400>  12
gtcctgcaat agc                                                          13


<210>  13
<211>  13
<212>  DNA
<213>  Artificial Sequence

<220>
<223>  Synthetic oligonucleotide

<400>  13
tccgtcctgc aat                                                          13


<210>  14
<211>  13
<212>  DNA
<213>  Artificial Sequence

<220>
<223>  Synthetic oligonucleotide

<400>  14
gtccgtcctg caa                                                          13


<210>  15
<211>  13
<212>  DNA
<213>  Artificial Sequence

<220>
<223>  Synthetic oligonucleotide

<400>  15
ggtccgtcct gca                                                          13


<210>  16
<211>  13
<212>  DNA
<213>  Artificial Sequence

<220>
<223>  Synthetic oligonucleotide

<400>  16
aggtccgtcc tgc                                                          13


<210>  17
<211>  13
<212>  DNA
<213>  Artificial Sequence

<220>
<223>  Synthetic oligonucleotide

<400>  17
caggtccgtc ctg                                                          13


<210>  18
<211>  13
<212>  DNA
<213>  Artificial Sequence

<220>
<223>  Synthetic oligonucleotide

<400>  18
tgggacaggt ccg                                                          13


<210>  19
<211>  13
<212>  DNA
<213>  Artificial Sequence

<220>
<223>  Synthetic oligonucleotide

<400>  19
ttgggacagg tcc                                                          13


<210>  20
<211>  13
<212>  DNA
<213>  Artificial Sequence

<220>
<223>  Synthetic oligonucleotide

<400>  20
ggcttgggac agg                                                          13


<210>  21
<211>  13
<212>  DNA
<213>  Artificial Sequence

<220>
<223>  Synthetic oligonucleotide

<400>  21
tggcttggga cag                                                          13


<210>  22
<211>  13
<212>  DNA
<213>  Artificial Sequence

<220>
<223>  Synthetic oligonucleotide

<400>  22
ctggcttggg aca                                                          13

<210>  23
<211>  13
<212>  DNA
<213>  Artificial Sequence

<220>
<223>  Synthetic oligonucleotide

<400>  23
tctggcttgg gac                                                          13


<210>  24
<211>  13
<212>  DNA
<213>  Artificial Sequence

<220>
<223>  Synthetic oligonucleotide

<400>  24
catctggctt ggg                                                          13


<210>  25
<211>  13
<212>  DNA
<213>  Artificial Sequence

<220>
<223>  Synthetic oligonucleotide

<400>  25
tcatctggct tgg                                                          13


<210>  26
<211>  13
<212>  DNA
<213>  Artificial Sequence

<220>
<223>  Synthetic oligonucleotide

<400>  26
atcatctggc ttg                                                          13


<210>  27
<211>  13
<212>  DNA
<213>  Artificial Sequence

<220>
<223>  Synthetic oligonucleotide

<400>  27
aatcatctgg ctt                                                          13


<210>  28
<211>  13

<212>  DNA
<213>  Artificial Sequence

<220>
<223>  Synthetic oligonucleotide

<400>  28
aaatcatctg gct                                                          13


<210>  29
<211>  13
<212>  DNA
<213>  Artificial Sequence

<220>
<223>  Synthetic oligonucleotide

<400>  29
tcctggctca tag                                                          13


<210>  30
<211>  13
<212>  DNA
<213>  Artificial Sequence

<220>
<223>  Synthetic oligonucleotide

<400>  30
ctcctggctc ata                                                          13


<210>  31
<211>  13
<212>  DNA
<213>  Artificial Sequence

<220>
<223>  Synthetic oligonucleotide

<400>  31
tctcctggct cat                                                          13


<210>  32
<211>  13
<212>  DNA
<213>  Artificial Sequence

<220>
<223>  Synthetic oligonucleotide

<400>  32
ccggcttgca gga                                                          13


<210>  33
<211>  13
<212>  DNA
<213>  Artificial Sequence

<220>
<223>  Synthetic oligonucleotide

<400>  33
cccggcttgc agg                                                          13


<210>  34
<211>  13
<212>  DNA
<213>  Artificial Sequence

<220>
<223>  Synthetic oligonucleotide

<400>  34
gcccggcttg cag                                                          13


<210>  35
<211>  13
<212>  DNA
<213>  Artificial Sequence

<220>
<223>  Synthetic oligonucleotide

<400>  35
agcccggctt gca                                                          13


<210>  36
<211>  13
<212>  DNA
<213>  Artificial Sequence

<220>
<223>  Synthetic oligonucleotide

<400>  36
tagcccggct tgc                                                          13


<210>  37
<211>  13
<212>  DNA
<213>  Artificial Sequence

<220>
<223>  Synthetic oligonucleotide

<400>  37
atagcccggc ttg                                                          13


<210>  38
<211>  13
<212>  DNA
<213>  Artificial Sequence

<220>
<223>  Synthetic oligonucleotide

<400>  38
catagcccgg ctt                                                          13


<210>  39
<211>  13
<212>  DNA
<213>  Artificial Sequence

<220>
<223>  Synthetic oligonucleotide

<400>  39
acacatagcc cgg                                                          13


<210>  40
<211>  13
<212>  DNA
<213>  Artificial Sequence

<220>
<223>  Synthetic oligonucleotide

<400>  40
gacacatagc ccg                                                          13


<210>  41
<211>  13
<212>  DNA
<213>  Artificial Sequence

<220>
<223>  Synthetic oligonucleotide

<400>  41
tgtgagaggg cag                                                          13


<210>  42
<211>  13
<212>  DNA
<213>  Artificial Sequence

<220>
<223>  Synthetic oligonucleotide

<400>  42
ttgatgggcc aca                                                          13


<210>  43
<211>  13
<212>  DNA
<213>  Artificial Sequence

<220>
<223>  Synthetic oligonucleotide

<400>  43
gttgatgggc cac                                                          13

<210>  44
<211>  13
<212>  DNA
<213>  Artificial Sequence

<220>
<223>  Synthetic oligonucleotide

<400>  44
tgttgatggg cca                                                          13


<210>  45
<211>  13
<212>  DNA
<213>  Artificial Sequence

<220>
<223>  Synthetic oligonucleotide

<400>  45
gtgttgatgg gcc                                                          13


<210>  46
<211>  13
<212>  DNA
<213>  Artificial Sequence

<220>
<223>  Synthetic oligonucleotide

<400>  46
ggacatactc tgg                                                          13


<210>  47
<211>  13
<212>  DNA
<213>  Artificial Sequence

<220>
<223>  Synthetic oligonucleotide

<400>  47
aggacatact ctg                                                          13


<210>  48
<211>  13
<212>  DNA
<213>  Artificial Sequence

<220>
<223>  Synthetic oligonucleotide

<400>  48
aaggacatac tct                                                          13


<210>  49
<211>  13

<212>  DNA
<213>  Artificial Sequence

<220>
<223>  Synthetic oligonucleotide

<400>  49
aaaggacata ctc                                                          13


<210>  50
<211>  13
<212>  DNA
<213>  Artificial Sequence

<220>
<223>  Synthetic oligonucleotide

<400>  50
gctccatttt cta                                                          13


<210>  51
<211>  13
<212>  DNA
<213>  Artificial Sequence

<220>
<223>  Synthetic oligonucleotide

<400>  51
gggatattca aaa                                                          13


<210>  52
<211>  13
<212>  DNA
<213>  Artificial Sequence

<220>
<223>  Synthetic oligonucleotide

<400>  52
tgggatattc aaa                                                          13


<210>  53
<211>  13
<212>  DNA
<213>  Artificial Sequence

<220>
<223>  Synthetic oligonucleotide

<400>  53
ttgggatatt caa                                                          13


<210>  54
<211>  13
<212>  DNA
<213>  Artificial Sequence

<220>
<223>  Synthetic oligonucleotide

<400>  54
gttgggatat tca                                                          13


<210>  55
<211>  13
<212>  DNA
<213>  Artificial Sequence

<220>
<223>  Synthetic oligonucleotide

<400>  55
gcacttggca gaa                                                          13


<210>  56
<211>  13
<212>  DNA
<213>  Artificial Sequence

<220>
<223>  Synthetic oligonucleotide

<400>  56
tgcacttggc aga                                                          13


<210>  57
<211>  13
<212>  DNA
<213>  Artificial Sequence

<220>
<223>  Synthetic oligonucleotide

<400>  57
gtgcacttgg cag                                                          13


<210>  58
<211>  13
<212>  DNA
<213>  Artificial Sequence

<220>
<223>  Synthetic oligonucleotide

<400>  58
agtgcacttg gca                                                          13


<210>  59
<211>  13
<212>  DNA
<213>  Artificial Sequence

<220>
<223>  Synthetic oligonucleotide

<400>  59
cagtgcactt ggc                                                          13


<210>  60
<211>  13
<212>  DNA
<213>  Artificial Sequence

<220>
<223>  Synthetic oligonucleotide

<400>  60
ctcagtgcac ttg                                                          13


<210>  61
<211>  13
<212>  DNA
<213>  Artificial Sequence

<220>
<223>  Synthetic oligonucleotide

<400>  61
cctcagtgca ctt                                                          13


<210>  62
<211>  13
<212>  DNA
<213>  Artificial Sequence

<220>
<223>  Synthetic oligonucleotide

<400>  62
tcctcagtgc act                                                          13


<210>  63
<211>  13
<212>  DNA
<213>  Artificial Sequence

<220>
<223>  Synthetic oligonucleotide

<400>  63
ttcctcagtg cac                                                          13


<210>  64
<211>  13
<212>  DNA
<213>  Artificial Sequence

<220>
<223>  Synthetic oligonucleotide

<400>  64
cttcctcagt gca                                                          13

<210>  65
<211>  13
<212>  DNA
<213>  Artificial Sequence

<220>
<223>  Synthetic oligonucleotide

<400>  65
ccttcctcag tgc                                                          13


<210>  66
<211>  13
<212>  DNA
<213>  Artificial Sequence

<220>
<223>  Synthetic oligonucleotide

<400>  66
tccttcctca gtg                                                          13


<210>  67
<211>  13
<212>  DNA
<213>  Artificial Sequence

<220>
<223>  Synthetic oligonucleotide

<400>  67
gctgatggct tat                                                          13


<210>  68
<211>  13
<212>  DNA
<213>  Artificial Sequence

<220>
<223>  Synthetic oligonucleotide

<400>  68
agctgatggc tta                                                          13


<210>  69
<211>  13
<212>  DNA
<213>  Artificial Sequence

<220>
<223>  Synthetic oligonucleotide

<400>  69
cagctgatgg ctt                                                          13


<210>  70
<211>  13

<212>  DNA
<213>  Artificial Sequence

<220>
<223>  Synthetic oligonucleotide

<400>  70
ccagctgatg gct                                                          13


<210>  71
<211>  13
<212>  DNA
<213>  Artificial Sequence

<220>
<223>  Synthetic oligonucleotide

<400>  71
tccagctgat ggc                                                          13


<210>  72
<211>  13
<212>  DNA
<213>  Artificial Sequence

<220>
<223>  Synthetic oligonucleotide

<400>  72
ttccagctga tgg                                                          13


<210>  73
<211>  13
<212>  DNA
<213>  Artificial Sequence

<220>
<223>  Synthetic oligonucleotide

<400>  73
gtttccagct gat                                                          13


<210>  74
<211>  13
<212>  DNA
<213>  Artificial Sequence

<220>
<223>  Synthetic oligonucleotide

<400>  74
tgtttccagc tga                                                          13


<210>  75
<211>  13
<212>  DNA
<213>  Artificial Sequence

<220>
<223>  Synthetic oligonucleotide

<400>  75
ttgtttccag ctg                                                          13


<210>  76
<211>  13
<212>  DNA
<213>  Artificial Sequence

<220>
<223>  Synthetic oligonucleotide

<400>  76
aactgctgtg tcc                                                          13


<210>  77
<211>  13
<212>  DNA
<213>  Artificial Sequence

<220>
<223>  Synthetic oligonucleotide

<400>  77
aaaaactgct gtg                                                          13


<210>  78
<211>  13
<212>  DNA
<213>  Artificial Sequence

<220>
<223>  Synthetic oligonucleotide

<400>  78
tggcaaacat tca                                                          13


<210>  79
<211>  13
<212>  DNA
<213>  Artificial Sequence

<220>
<223>  Synthetic oligonucleotide

<400>  79
gtggcaaaca ttc                                                          13


<210>  80
<211>  13
<212>  DNA
<213>  Artificial Sequence

<220>
<223>  Synthetic oligonucleotide

<400>  80
gttgtggcaa aca                                                          13


<210>  81
<211>  13
<212>  DNA
<213>  Artificial Sequence

<220>
<223>  Synthetic oligonucleotide

<400>  81
tgttgtggca aac                                                          13


<210>  82
<211>  13
<212>  DNA
<213>  Artificial Sequence

<220>
<223>  Synthetic oligonucleotide

<400>  82
gcatgttgtg gca                                                          13


<210>  83
<211>  13
<212>  DNA
<213>  Artificial Sequence

<220>
<223>  Synthetic oligonucleotide

<400>  83
cgcatgttgt ggc                                                          13


<210>  84
<211>  13
<212>  DNA
<213>  Artificial Sequence

<220>
<223>  Synthetic oligonucleotide

<400>  84
tcgcatgttg tgg                                                          13


<210>  85
<211>  13
<212>  DNA
<213>  Artificial Sequence

<220>
<223>  Synthetic oligonucleotide

<400>  85
atcgcatgtt gtg                                                          13

<210>  86
<211>  13
<212>  DNA
<213>  Artificial Sequence

<220>
<223>  Synthetic oligonucleotide

<400>  86
acatcgcatg ttg                                                          13


<210>  87
<211>  13
<212>  DNA
<213>  Artificial Sequence

<220>
<223>  Synthetic oligonucleotide

<400>  87
ttccaaacat cgc                                                          13


<210>  88
<211>  13
<212>  DNA
<213>  Artificial Sequence

<220>
<223>  Synthetic oligonucleotide

<400>  88
agtccaattt cca                                                          13


<210>  89
<211>  13
<212>  DNA
<213>  Artificial Sequence

<220>
<223>  Synthetic oligonucleotide

<400>  89
ccctgcattc tgg                                                          13


<210>  90
<211>  13
<212>  DNA
<213>  Artificial Sequence

<220>
<223>  Synthetic oligonucleotide

<400>  90
tccctgcatt ctg                                                          13


<210>  91
<211>  13

<212>  DNA
<213>  Artificial Sequence

<220>
<223>  Synthetic oligonucleotide

<400>  91
ttccctgcat tct                                                          13


<210>  92
<211>  13
<212>  DNA
<213>  Artificial Sequence

<220>
<223>  Synthetic oligonucleotide

<400>  92
attgtctggt ctt                                                          13


<210>  93
<211>  13
<212>  DNA
<213>  Artificial Sequence

<220>
<223>  Synthetic oligonucleotide

<400>  93
cattgtctgg tct                                                          13


<210>  94
<211>  13
<212>  DNA
<213>  Artificial Sequence

<220>
<223>  Synthetic oligonucleotide

<400>  94
ccattgtctg gtc                                                          13


<210>  95
<211>  13
<212>  DNA
<213>  Artificial Sequence

<220>
<223>  Synthetic oligonucleotide

<400>  95
tccattgtct ggt                                                          13


<210>  96
<211>  13
<212>  DNA
<213>  Artificial Sequence

<220>
<223>  Synthetic oligonucleotide

<400>  96
atccattgtc tgg                                                          13


<210>  97
<211>  13
<212>  DNA
<213>  Artificial Sequence

<220>
<223>  Synthetic oligonucleotide

<400>  97
aatccattgt ctg                                                          13


<210>  98
<211>  13
<212>  DNA
<213>  Artificial Sequence

<220>
<223>  Synthetic oligonucleotide

<400>  98
gcaggatagt tca                                                          13


<210>  99
<211>  13
<212>  DNA
<213>  Artificial Sequence

<220>
<223>  Synthetic oligonucleotide

<400>  99
ggttttgcag gat                                                          13


<210>  100
<211>  13
<212>  DNA
<213>  Artificial Sequence

<220>
<223>  Synthetic oligonucleotide

<400>  100
tggttttgca gga                                                          13


<210>  101
<211>  13
<212>  DNA
<213>  Artificial Sequence

<220>
<223>  Synthetic oligonucleotide

<400>  101
ggctttatcc ttg                                                          13


<210>  102
<211>  13
<212>  DNA
<213>  Artificial Sequence

<220>
<223>  Synthetic oligonucleotide

<400>  102
gtggctttat cct                                                          13


<210>  103
<211>  13
<212>  DNA
<213>  Artificial Sequence

<220>
<223>  Synthetic oligonucleotide

<400>  103
atgtggcttt atc                                                          13


<210>  104
<211>  13
<212>  DNA
<213>  Artificial Sequence

<220>
<223>  Synthetic oligonucleotide

<400>  104
aatgtggctt tat                                                          13


<210>  105
<211>  13
<212>  DNA
<213>  Artificial Sequence

<220>
<223>  Synthetic oligonucleotide

<400>  105
ccaaatgtgg ctt                                                          13


<210>  106
<211>  13
<212>  DNA
<213>  Artificial Sequence

<220>
<223>  Synthetic oligonucleotide

<400>  106
catcatggca gcc                                                          13

<210>  107
<211>  13
<212>  DNA
<213>  Artificial Sequence

<220>
<223>  Synthetic oligonucleotide

<400>  107
ccatcatggc agc                                                          13


<210>  108
<211>  13
<212>  DNA
<213>  Artificial Sequence

<220>
<223>  Synthetic oligonucleotide

<400>  108
tccatcatgg cag                                                          13


<210>  109
<211>  13
<212>  DNA
<213>  Artificial Sequence

<220>
<223>  Synthetic oligonucleotide

<400>  109
atccatcatg gca                                                          13


<210>  110
<211>  13
<212>  DNA
<213>  Artificial Sequence

<220>
<223>  Synthetic oligonucleotide

<400>  110
tatccatcat ggc                                                          13


<210>  111
<211>  13
<212>  DNA
<213>  Artificial Sequence

<220>
<223>  Synthetic oligonucleotide

<400>  111
atatccatca tgg                                                          13


<210>  112
<211>  13

<212>  DNA
<213>  Artificial Sequence

<220>
<223>  Synthetic oligonucleotide

<400>  112
cccagtttgg tac                                                          13


<210>  113
<211>  13
<212>  DNA
<213>  Artificial Sequence

<220>
<223>  Synthetic oligonucleotide

<400>  113
tcccagtttg gta                                                          13


<210>  114
<211>  13
<212>  DNA
<213>  Artificial Sequence

<220>
<223>  Synthetic oligonucleotide

<400>  114
ttcccagttt ggt                                                          13


<210>  115
<211>  13
<212>  DNA
<213>  Artificial Sequence

<220>
<223>  Synthetic oligonucleotide

<400>  115
tttcccagtt tgg                                                          13


<210>  116
<211>  13
<212>  DNA
<213>  Artificial Sequence

<220>
<223>  Synthetic oligonucleotide

<400>  116
gtttcccagt ttg                                                          13


<210>  117
<211>  13
<212>  DNA
<213>  Artificial Sequence

<220>
<223>  Synthetic oligonucleotide

<400>  117
cagtttccca gtt                                                          13


<210>  118
<211>  13
<212>  DNA
<213>  Artificial Sequence

<220>
<223>  Synthetic oligonucleotide

<400>  118
ccagtttccc agt                                                          13


<210>  119
<211>  13
<212>  DNA
<213>  Artificial Sequence

<220>
<223>  Synthetic oligonucleotide

<400>  119
accagtttcc cag                                                          13


<210>  120
<211>  13
<212>  DNA
<213>  Artificial Sequence

<220>
<223>  Synthetic oligonucleotide

<400>  120
gaccagtttc cca                                                          13


<210>  121
<211>  13
<212>  DNA
<213>  Artificial Sequence

<220>
<223>  Synthetic oligonucleotide

<400>  121
agaccagttt ccc                                                          13


<210>  122
<211>  13
<212>  DNA
<213>  Artificial Sequence

<220>
<223>  Synthetic oligonucleotide

<400>  122
gcagaccagt ttc                                                          13


<210>  123
<211>  13
<212>  DNA
<213>  Artificial Sequence

<220>
<223>  Synthetic oligonucleotide

<400>  123
ggcagaccag ttt                                                          13


<210>  124
<211>  13
<212>  DNA
<213>  Artificial Sequence

<220>
<223>  Synthetic oligonucleotide

<400>  124
tggcagacca gtt                                                          13


<210>  125
<211>  13
<212>  DNA
<213>  Artificial Sequence

<220>
<223>  Synthetic oligonucleotide

<400>  125
atggcagacc agt                                                          13


<210>  126
<211>  13
<212>  DNA
<213>  Artificial Sequence

<220>
<223>  Synthetic oligonucleotide

<400>  126
catggcagac cag                                                          13


<210>  127
<211>  13
<212>  DNA
<213>  Artificial Sequence

<220>
<223>  Synthetic oligonucleotide

<400>  127
gcatggcaga cca                                                          13

<210>  128
<211>  13
<212>  DNA
<213>  Artificial Sequence

<220>
<223>  Synthetic oligonucleotide

<400>  128
ggcatggcag acc                                                          13


<210>  129
<211>  13
<212>  DNA
<213>  Artificial Sequence

<220>
<223>  Synthetic oligonucleotide

<400>  129
tggcatggca gac                                                          13


<210>  130
<211>  13
<212>  DNA
<213>  Artificial Sequence

<220>
<223>  Synthetic oligonucleotide

<400>  130
ttggcatggc aga                                                          13


<210>  131
<211>  13
<212>  DNA
<213>  Artificial Sequence

<220>
<223>  Synthetic oligonucleotide

<400>  131
cttggcatgg cag                                                          13


<210>  132
<211>  13
<212>  DNA
<213>  Artificial Sequence

<220>
<223>  Synthetic oligonucleotide

<400>  132
acttggcatg gca                                                          13


<210>  133
<211>  13

<212>  DNA
<213>  Artificial Sequence

<220>
<223>  Synthetic oligonucleotide

<400>  133
aacttggcat ggc                                                          13


<210>  134
<211>  13
<212>  DNA
<213>  Artificial Sequence

<220>
<223>  Synthetic oligonucleotide

<400>  134
caacttggca tgg                                                          13


<210>  135
<211>  13
<212>  DNA
<213>  Artificial Sequence

<220>
<223>  Synthetic oligonucleotide

<400>  135
acaacttggc atg                                                          13


<210>  136
<211>  13
<212>  DNA
<213>  Artificial Sequence

<220>
<223>  Synthetic oligonucleotide

<400>  136
gctttacaac ttg                                                          13


<210>  137
<211>  13
<212>  DNA
<213>  Artificial Sequence

<220>
<223>  Synthetic oligonucleotide

<400>  137
tcacaggtac ttt                                                          13


<210>  138
<211>  13
<212>  DNA
<213>  Artificial Sequence

<220>
<223>  Synthetic oligonucleotide

<400>  138
acaccacagt ggc                                                          13


<210>  139
<211>  13
<212>  DNA
<213>  Artificial Sequence

<220>
<223>  Synthetic oligonucleotide

<400>  139
gtacaccaca gtg                                                          13


<210>  140
<211>  13
<212>  DNA
<213>  Artificial Sequence

<220>
<223>  Synthetic oligonucleotide

<400>  140
ggtacaccac agt                                                          13


<210>  141
<211>  13
<212>  DNA
<213>  Artificial Sequence

<220>
<223>  Synthetic oligonucleotide

<400>  141
tggtacacca cag                                                          13


<210>  142
<211>  13
<212>  DNA
<213>  Artificial Sequence

<220>
<223>  Synthetic oligonucleotide

<400>  142
ttggtacacc aca                                                          13


<210>  143
<211>  13
<212>  DNA
<213>  Artificial Sequence

<220>
<223>  Synthetic oligonucleotide

<400>  143
cttggtacac cac                                                          13


<210>  144
<211>  13
<212>  DNA
<213>  Artificial Sequence

<220>
<223>  Synthetic oligonucleotide

<400>  144
ccttggtaca cca                                                          13


<210>  145
<211>  13
<212>  DNA
<213>  Artificial Sequence

<220>
<223>  Synthetic oligonucleotide

<400>  145
tccttggtac acc                                                          13


<210>  146
<211>  13
<212>  DNA
<213>  Artificial Sequence

<220>
<223>  Synthetic oligonucleotide

<400>  146
ctccttggta cac                                                          13


<210>  147
<211>  13
<212>  DNA
<213>  Artificial Sequence

<220>
<223>  Synthetic oligonucleotide

<400>  147
tctccttggt aca                                                          13


<210>  148
<211>  13
<212>  DNA
<213>  Artificial Sequence

<220>
<223>  Synthetic oligonucleotide

<400>  148
ctctccttgg tac                                                          13

<210>  149
<211>  13
<212>  DNA
<213>  Artificial Sequence

<220>
<223>  Synthetic oligonucleotide

<400>  149
tctctccttg gta                                                          13


<210>  150
<211>  13
<212>  DNA
<213>  Artificial Sequence

<220>
<223>  Synthetic oligonucleotide

<400>  150
ctctctcctt ggt                                                          13


<210>  151
<211>  13
<212>  DNA
<213>  Artificial Sequence

<220>
<223>  Synthetic oligonucleotide

<400>  151
tctctctcct tgg                                                          13


<210>  152
<211>  13
<212>  DNA
<213>  Artificial Sequence

<220>
<223>  Synthetic oligonucleotide

<400>  152
ctctctctcc ttg                                                          13


<210>  153
<211>  13
<212>  DNA
<213>  Artificial Sequence

<220>
<223>  Synthetic oligonucleotide

<400>  153
actctctctc ctt                                                          13


<210>  154
<211>  13

<212>  DNA
<213>  Artificial Sequence

<220>
<223>  Synthetic oligonucleotide

<400>  154
tactctctct cct                                                          13


<210>  155
<211>  13
<212>  DNA
<213>  Artificial Sequence

<220>
<223>  Synthetic oligonucleotide

<400>  155
ttactctctc tcc                                                          13


<210>  156
<211>  13
<212>  DNA
<213>  Artificial Sequence

<220>
<223>  Synthetic oligonucleotide

<400>  156
gcattccatt ctt                                                          13


<210>  157
<211>  13
<212>  DNA
<213>  Artificial Sequence

<220>
<223>  Synthetic oligonucleotide

<400>  157
agcattccat tct                                                          13


<210>  158
<211>  13
<212>  DNA
<213>  Artificial Sequence

<220>
<223>  Synthetic oligonucleotide

<400>  158
agctacactt ctt                                                          13


<210>  159
<211>  13
<212>  DNA
<213>  Artificial Sequence

<220>
<223>  Synthetic oligonucleotide

<400>  159
tagctacact tct                                                          13


<210>  160
<211>  13
<212>  DNA
<213>  Artificial Sequence

<220>
<223>  Synthetic oligonucleotide

<400>  160
ttgaagcatt tgg                                                          13


<210>  161
<211>  13
<212>  DNA
<213>  Artificial Sequence

<220>
<223>  Synthetic oligonucleotide

<400>  161
gttccttgaa gca                                                          13


<210>  162
<211>  13
<212>  DNA
<213>  Artificial Sequence

<220>
<223>  Synthetic oligonucleotide

<400>  162
tgttccttga agc                                                          13


<210>  163
<211>  13
<212>  DNA
<213>  Artificial Sequence

<220>
<223>  Synthetic oligonucleotide

<400>  163
gtgttccttg aag                                                          13


<210>  164
<211>  13
<212>  DNA
<213>  Artificial Sequence

<220>
<223>  Synthetic oligonucleotide

<400>  164
tgtgttcctt gaa                                                          13


<210>  165
<211>  13
<212>  DNA
<213>  Artificial Sequence

<220>
<223>  Synthetic oligonucleotide

<400>  165
ctgtgttcct tga                                                          13


<210>  166
<211>  13
<212>  DNA
<213>  Artificial Sequence

<220>
<223>  Synthetic oligonucleotide

<400>  166
actgtgttcc ttg                                                          13


<210>  167
<211>  13
<212>  DNA
<213>  Artificial Sequence

<220>
<223>  Synthetic oligonucleotide

<400>  167
aactgtgttc ctt                                                          13


<210>  168
<211>  13
<212>  DNA
<213>  Artificial Sequence

<220>
<223>  Synthetic oligonucleotide

<400>  168
gaactgtgtt cct                                                          13


<210>  169
<211>  13
<212>  DNA
<213>  Artificial Sequence

<220>
<223>  Synthetic oligonucleotide

<400>  169
agaactgtgt tcc                                                          13

<210>  170
<211>  13
<212>  DNA
<213>  Artificial Sequence

<220>
<223>  Synthetic oligonucleotide

<400>  170
gagaactgtg ttc                                                          13


<210>  171
<211>  13
<212>  DNA
<213>  Artificial Sequence

<220>
<223>  Synthetic oligonucleotide

<400>  171
agagaactgt gtt                                                          13


<210>  172
<211>  13
<212>  DNA
<213>  Artificial Sequence

<220>
<223>  Synthetic oligonucleotide

<400>  172
cagagaactg tgt                                                          13


<210>  173
<211>  13
<212>  DNA
<213>  Artificial Sequence

<220>
<223>  Synthetic oligonucleotide

<400>  173
ccagagaact gtg                                                          13


<210>  174
<211>  13
<212>  DNA
<213>  Artificial Sequence

<220>
<223>  Synthetic oligonucleotide

<400>  174
gccagagaac tgt                                                          13


<210>  175
<211>  13

<212>  DNA
<213>  Artificial Sequence

<220>
<223>  Synthetic oligonucleotide

<400>  175
agccagagaa ctg                                                          13


<210>  176
<211>  13
<212>  DNA
<213>  Artificial Sequence

<220>
<223>  Synthetic oligonucleotide

<400>  176
aagccagaga act                                                          13


<210>  177
<211>  13
<212>  DNA
<213>  Artificial Sequence

<220>
<223>  Synthetic oligonucleotide

<400>  177
gcatcagttt tcc                                                          13


<210>  178
<211>  13
<212>  DNA
<213>  Artificial Sequence

<220>
<223>  Synthetic oligonucleotide

<400>  178
tgcatcagtt ttc                                                          13


<210>  179
<211>  13
<212>  DNA
<213>  Artificial Sequence

<220>
<223>  Synthetic oligonucleotide

<400>  179
gatgcatcag ttt                                                          13


<210>  180
<211>  13
<212>  DNA
<213>  Artificial Sequence

<220>
<223>  Synthetic oligonucleotide

<400>  180
ggatgcatca gtt                                                          13


<210>  181
<211>  13
<212>  DNA
<213>  Artificial Sequence

<220>
<223>  Synthetic oligonucleotide

<400>  181
cggatgcatc agt                                                          13


<210>  182
<211>  13
<212>  DNA
<213>  Artificial Sequence

<220>
<223>  Synthetic oligonucleotide

<400>  182
tcggatgcat cag                                                          13


<210>  183
<211>  13
<212>  DNA
<213>  Artificial Sequence

<220>
<223>  Synthetic oligonucleotide

<400>  183
atcggatgca tca                                                          13


<210>  184
<211>  13
<212>  DNA
<213>  Artificial Sequence

<220>
<223>  Synthetic oligonucleotide

<400>  184
catcggatgc atc                                                          13


<210>  185
<211>  13
<212>  DNA
<213>  Artificial Sequence

<220>
<223>  Synthetic oligonucleotide

<400>  185
acatcggatg cat                                                          13


<210>  186
<211>  13
<212>  DNA
<213>  Artificial Sequence

<220>
<223>  Synthetic oligonucleotide

<400>  186
tacatcggat gca                                                          13


<210>  187
<211>  13
<212>  DNA
<213>  Artificial Sequence

<220>
<223>  Synthetic oligonucleotide

<400>  187
ttacatcgga tgc                                                          13


<210>  188
<211>  13
<212>  DNA
<213>  Artificial Sequence

<220>
<223>  Synthetic oligonucleotide

<400>  188
gctttacatc gga                                                          13


<210>  189
<211>  13
<212>  DNA
<213>  Artificial Sequence

<220>
<223>  Synthetic oligonucleotide

<400>  189
ggctttacat cgg                                                          13


<210>  190
<211>  13
<212>  DNA
<213>  Artificial Sequence

<220>
<223>  Synthetic oligonucleotide

<400>  190
tggctttaca tcg                                                          13

<210>  191
<211>  13
<212>  DNA
<213>  Artificial Sequence

<220>
<223>  Synthetic oligonucleotide

<400>  191
tgtggaatct gaa                                                          13


<210>  192
<211>  13
<212>  DNA
<213>  Artificial Sequence

<220>
<223>  Synthetic oligonucleotide

<400>  192
agtgtgacat ttt                                                          13


<210>  193
<211>  13
<212>  DNA
<213>  Artificial Sequence

<220>
<223>  Synthetic oligonucleotide

<400>  193
aagtgtgaca ttt                                                          13


<210>  194
<211>  13
<212>  DNA
<213>  Artificial Sequence

<220>
<223>  Synthetic oligonucleotide

<400>  194
ccttggatga aca                                                          13


<210>  195
<211>  13
<212>  DNA
<213>  Artificial Sequence

<220>
<223>  TCCTTGGATGAAC

<400>  195
tccttggatg aac                                                          13


<210>  196
<211>  13

<212>  DNA
<213>  Artificial Sequence

<220>
<223>  Synthetic oligonucleotide

<400>  196
gttccttgga tga                                                          13


<210>  197
<211>  13
<212>  DNA
<213>  Artificial Sequence

<220>
<223>  Synthetic oligonucleotide

<400>  197
ggttccttgg atg                                                          13


<210>  198
<211>  13
<212>  DNA
<213>  Artificial Sequence

<220>
<223>  Synthetic oligonucleotide

<400>  198
aggttccttg gat                                                          13


<210>  199
<211>  13
<212>  DNA
<213>  Artificial Sequence

<220>
<223>  Synthetic oligonucleotide

<400>  199
taggttcctt gga                                                          13


<210>  200
<211>  13
<212>  DNA
<213>  Artificial Sequence

<220>
<223>  Synthetic oligonucleotide

<400>  200
ttaggttcct tgg                                                          13


<210>  201
<211>  13
<212>  DNA
<213>  Homo sapiens

<400>  201
ttctccagtg ctc                                                          13


<210>  202
<211>  13
<212>  DNA
<213>  Homo sapiens

<400>  202
tctccagtgc tca                                                          13


<210>  203
<211>  13
<212>  DNA
<213>  Homo sapiens

<400>  203
ccagtgctca tct                                                          13


<210>  204
<211>  13
<212>  DNA
<213>  Homo sapiens

<400>  204
ctgccatgtt gct                                                          13


<210>  205
<211>  13
<212>  DNA
<213>  Homo sapiens

<400>  205
tgccatgttg cta                                                          13


<210>  206
<211>  13
<212>  DNA
<213>  Homo sapiens

<400>  206
gccatgttgc tat                                                          13


<210>  207
<211>  13
<212>  DNA
<213>  Homo sapiens

<400>  207
catgttgcta ttg                                                          13


<210>  208
<211>  13
<212>  DNA
<213>  Homo sapiens

<400>  208
atgttgctat tgc                                                          13


<210>  209
<211>  13
<212>  DNA
<213>  Homo sapiens

<400>  209
ttgctattgc agg                                                          13


<210>  210
<211>  13
<212>  DNA
<213>  Homo sapiens

<400>  210
tgctattgca gga                                                          13


<210>  211
<211>  13
<212>  DNA
<213>  Homo sapiens

<400>  211
gctattgcag gac                                                          13


<210>  212
<211>  13
<212>  DNA
<213>  Homo sapiens

<400>  212
attgcaggac gga                                                          13


<210>  213
<211>  13
<212>  DNA
<213>  Homo sapiens

<400>  213
ttgcaggacg gac                                                          13


<210>  214
<211>  13
<212>  DNA
<213>  Homo sapiens

<400>  214
tgcaggacgg acc                                                          13


<210>  215
<211>  13
<212>  DNA
<213>  Homo sapiens

<400>  215
gcaggacgga cct                                                          13


<210>  216
<211>  13
<212>  DNA
<213>  Homo sapiens

<400>  216
caggacggac ctg                                                          13


<210>  217
<211>  13
<212>  DNA
<213>  Homo sapiens

<400>  217
cggacctgtc cca                                                          13


<210>  218
<211>  13
<212>  DNA
<213>  Homo sapiens

<400>  218
ggacctgtcc caa                                                          13


<210>  219
<211>  13
<212>  DNA
<213>  Homo sapiens

<400>  219
cctgtcccaa gcc                                                          13


<210>  220
<211>  13
<212>  DNA
<213>  Homo sapiens

<400>  220
ctgtcccaag cca                                                          13


<210>  221
<211>  13
<212>  DNA
<213>  Homo sapiens

<400>  221
tgtcccaagc cag                                                          13


<210>  222
<211>  13
<212>  DNA
<213>  Homo sapiens

<400>  222
gtcccaagcc aga                                                          13


<210>  223
<211>  13
<212>  DNA
<213>  Homo sapiens

<400>  223
cccaagccag atg                                                          13


<210>  224
<211>  13
<212>  DNA
<213>  Homo sapiens

<400>  224
ccaagccaga tga                                                          13


<210>  225
<211>  13
<212>  DNA
<213>  Homo sapiens

<400>  225
caagccagat gat                                                          13


<210>  226
<211>  13
<212>  DNA
<213>  Homo sapiens

<400>  226
aagccagatg att                                                          13


<210>  227
<211>  13
<212>  DNA
<213>  Homo sapiens

<400>  227
agccagatga ttt                                                          13


<210>  228
<211>  13
<212>  DNA
<213>  Homo sapiens

<400>  228
ctatgagcca gga                                                          13


<210>  229
<211>  13
<212>  DNA
<213>  Homo sapiens

<400>  229
tatgagccag gag                                                          13


<210>  230
<211>  13
<212>  DNA
<213>  Homo sapiens

<400>  230
atgagccagg aga                                                          13


<210>  231
<211>  13
<212>  DNA
<213>  Homo sapiens

<400>  231
tcctgcaagc cgg                                                          13


<210>  232
<211>  13
<212>  DNA
<213>  Homo sapiens

<400>  232
cctgcaagcc ggg                                                          13


<210>  233
<211>  13
<212>  DNA
<213>  Homo sapiens

<400>  233
ctgcaagccg ggc                                                          13


<210>  234
<211>  13
<212>  DNA
<213>  Homo sapiens

<400>  234
tgcaagccgg gct                                                          13


<210>  235
<211>  13
<212>  DNA
<213>  Homo sapiens

<400>  235
gcaagccggg cta                                                          13


<210>  236
<211>  13
<212>  DNA
<213>  Homo sapiens

<400>  236
caagccgggc tat                                                          13


<210>  237
<211>  13
<212>  DNA
<213>  Homo sapiens

<400>  237
aagccgggct atg                                                          13


<210>  238
<211>  13
<212>  DNA
<213>  Homo sapiens

<400>  238
ccgggctatg tgt                                                          13


<210>  239
<211>  13
<212>  DNA
<213>  Homo sapiens

<400>  239
cgggctatgt gtc                                                          13


<210>  240
<211>  13
<212>  DNA
<213>  Homo sapiens

<400>  240
ctgccctctc aca                                                          13


<210>  241
<211>  13
<212>  DNA
<213>  Homo sapiens

<400>  241
tgtggcccat caa                                                          13


<210>  242
<211>  13
<212>  DNA
<213>  Homo sapiens

<400>  242
gtggcccatc aac                                                          13


<210>  243
<211>  13
<212>  DNA
<213>  Homo sapiens

<400>  243
tggcccatca aca                                                          13


<210>  244
<211>  13
<212>  DNA
<213>  Homo sapiens

<400>  244
ggcccatcaa cac                                                          13


<210>  245
<211>  13
<212>  DNA
<213>  Homo sapiens

<400>  245
ccagagtatg tcc                                                          13


<210>  246
<211>  13
<212>  DNA
<213>  Homo sapiens

<400>  246
cagagtatgt cct                                                          13


<210>  247
<211>  13
<212>  DNA
<213>  Homo sapiens

<400>  247
agagtatgtc ctt                                                          13


<210>  248
<211>  13
<212>  DNA
<213>  Homo sapiens

<400>  248
gagtatgtcc ttt                                                          13


<210>  249
<211>  13
<212>  DNA
<213>  Homo sapiens

<400>  249
tagaaaatgg agc                                                          13


<210>  250
<211>  13
<212>  DNA
<213>  Homo sapiens

<400>  250
ttttgaatat ccc                                                          13


<210>  251
<211>  13
<212>  DNA
<213>  Homo sapiens

<400>  251
tttgaatatc cca                                                          13


<210>  252
<211>  13
<212>  DNA
<213>  Homo sapiens

<400>  252
ttgaatatcc caa                                                          13


<210>  253
<211>  13
<212>  DNA
<213>  Homo sapiens

<400>  253
tgaatatccc aac                                                          13


<210>  254
<211>  13
<212>  DNA
<213>  Homo sapiens

<400>  254
ttctgccaag tgc                                                          13


<210>  255
<211>  13
<212>  DNA
<213>  Homo sapiens

<400>  255
tctgccaagt gca                                                          13


<210>  256
<211>  13
<212>  DNA
<213>  Homo sapiens

<400>  256
ctgccaagtg cac                                                          13


<210>  257
<211>  13
<212>  DNA
<213>  Homo sapiens

<400>  257
tgccaagtgc act                                                          13


<210>  258
<211>  13
<212>  DNA
<213>  Homo sapiens

<400>  258
gccaagtgca ctg                                                          13


<210>  259
<211>  13
<212>  DNA
<213>  Homo sapiens

<400>  259
caagtgcact gag                                                          13


<210>  260
<211>  13
<212>  DNA
<213>  Homo sapiens

<400>  260
aagtgcactg agg                                                          13


<210>  261
<211>  13
<212>  DNA
<213>  Homo sapiens

<400>  261
agtgcactga gga                                                          13


<210>  262
<211>  13
<212>  DNA
<213>  Homo sapiens

<400>  262
gtgcactgag gaa                                                          13


<210>  263
<211>  13
<212>  DNA
<213>  Homo sapiens

<400>  263
tgcactgagg aag                                                          13


<210>  264
<211>  13
<212>  DNA
<213>  Homo sapiens

<400>  264
gcactgagga agg                                                          13


<210>  265
<211>  13
<212>  DNA
<213>  Homo sapiens

<400>  265
cactgaggaa gga                                                          13


<210>  266
<211>  13
<212>  DNA
<213>  Homo sapiens

<400>  266
ataagccatc agc                                                          13


<210>  267
<211>  13
<212>  DNA
<213>  Homo sapiens

<400>  267
taagccatca gct                                                          13


<210>  268
<211>  13
<212>  DNA
<213>  Homo sapiens

<400>  268
aagccatcag ctg                                                          13


<210>  269
<211>  13
<212>  DNA
<213>  Homo sapiens

<400>  269
agccatcagc tgg                                                          13


<210>  270
<211>  13
<212>  DNA
<213>  Homo sapiens

<400>  270
gccatcagct gga                                                          13


<210>  271
<211>  13
<212>  DNA
<213>  Homo sapiens

<400>  271
ccatcagctg gaa                                                          13


<210>  272
<211>  13
<212>  DNA
<213>  Homo sapiens

<400>  272
atcagctgga aac                                                          13


<210>  273
<211>  13
<212>  DNA
<213>  Homo sapiens

<400>  273
tcagctggaa aca                                                          13


<210>  274
<211>  13
<212>  DNA
<213>  Homo sapiens

<400>  274
cagctggaaa caa                                                          13


<210>  275
<211>  13
<212>  DNA
<213>  Homo sapiens

<400>  275
ggacacagca gtt                                                          13


<210>  276
<211>  13
<212>  DNA
<213>  Homo sapiens

<400>  276
cacagcagtt ttt                                                          13


<210>  277
<211>  13
<212>  DNA
<213>  Homo sapiens

<400>  277
tgaatgtttg cca                                                          13


<210>  278
<211>  13
<212>  DNA
<213>  Homo sapiens

<400>  278
gaatgtttgc cac                                                          13


<210>  279
<211>  13
<212>  DNA
<213>  Homo sapiens

<400>  279
tgtttgccac aac                                                          13


<210>  280
<211>  13
<212>  DNA
<213>  Homo sapiens

<400>  280
gtttgccaca aca                                                          13


<210>  281
<211>  13
<212>  DNA
<213>  Homo sapiens

<400>  281
tgccacaaca tgc                                                          13


<210>  282
<211>  13
<212>  DNA
<213>  Homo sapiens

<400>  282
gccacaacat gcg                                                          13


<210>  283
<211>  13
<212>  DNA
<213>  Homo sapiens

<400>  283
ccacaacatg cga                                                          13


<210>  284
<211>  13
<212>  DNA
<213>  Homo sapiens

<400>  284
cacaacatgc gat                                                          13


<210>  285
<211>  13
<212>  DNA
<213>  Homo sapiens

<400>  285
caacatgcga tgt                                                          13


<210>  286
<211>  13
<212>  DNA
<213>  Homo sapiens

<400>  286
gcgatgtttg gaa                                                          13


<210>  287
<211>  13
<212>  DNA
<213>  Homo sapiens

<400>  287
tggaaattgg act                                                          13


<210>  288
<211>  13
<212>  DNA
<213>  Homo sapiens

<400>  288
ccagaatgca ggg                                                          13


<210>  289
<211>  13
<212>  DNA
<213>  Homo sapiens

<400>  289
cagaatgcag gga                                                          13


<210>  290
<211>  13
<212>  DNA
<213>  Homo sapiens

<400>  290
agaatgcagg gaa                                                          13


<210>  291
<211>  13
<212>  DNA
<213>  Homo sapiens

<400>  291
aagaccagac aat                                                          13


<210>  292
<211>  13
<212>  DNA
<213>  Homo sapiens

<400>  292
agaccagaca atg                                                          13


<210>  293
<211>  13
<212>  DNA
<213>  Homo sapiens

<400>  293
gaccagacaa tgg                                                          13


<210>  294
<211>  13
<212>  DNA
<213>  Homo sapiens

<400>  294
accagacaat gga                                                          13


<210>  295
<211>  13
<212>  DNA
<213>  Homo sapiens

<400>  295
ccagacaatg gat                                                          13


<210>  296
<211>  13
<212>  DNA
<213>  Homo sapiens

<400>  296
cagacaatgg att                                                          13


<210>  297
<211>  13
<212>  DNA
<213>  Homo sapiens

<400>  297
tgaactatcc tgc                                                          13


<210>  298
<211>  13
<212>  DNA
<213>  Homo sapiens

<400>  298
atcctgcaaa acc                                                          13


<210>  299
<211>  13
<212>  DNA
<213>  Homo sapiens

<400>  299
tcctgcaaaa cca                                                          13


<210>  300
<211>  13
<212>  DNA
<213>  Homo sapiens

<400>  300
caaggataaa gcc                                                          13


<210>  301
<211>  13
<212>  DNA
<213>  Homo sapiens

<400>  301
aggataaagc cac                                                          13


<210>  302
<211>  13
<212>  DNA
<213>  Homo sapiens

<400>  302
gataaagcca cat                                                          13


<210>  303
<211>  13
<212>  DNA
<213>  Homo sapiens

<400>  303
ataaagccac att                                                          13


<210>  304
<211>  13
<212>  DNA
<213>  Homo sapiens

<400>  304
aagccacatt tgg                                                          13


<210>  305
<211>  13
<212>  DNA
<213>  Homo sapiens

<400>  305
ggctgccatg atg                                                          13


<210>  306
<211>  13
<212>  DNA
<213>  Homo sapiens

<400>  306
gctgccatga tgg                                                          13


<210>  307
<211>  13
<212>  DNA
<213>  Homo sapiens

<400>  307
ctgccatgat gga                                                          13


<210>  308
<211>  13
<212>  DNA
<213>  Homo sapiens

<400>  308
tgccatgatg gat                                                          13


<210>  309
<211>  13
<212>  DNA
<213>  Homo sapiens

<400>  309
gccatgatgg ata                                                          13


<210>  310
<211>  13
<212>  DNA
<213>  Homo sapiens

<400>  310
ccatgatgga tat                                                          13


<210>  311
<211>  13
<212>  DNA
<213>  Homo sapiens

<400>  311
gtaccaaact ggg                                                          13


<210>  312
<211>  13
<212>  DNA
<213>  Homo sapiens

<400>  312
taccaaactg gga                                                          13


<210>  313
<211>  13
<212>  DNA
<213>  Homo sapiens

<400>  313
accaaactgg gaa                                                          13


<210>  314
<211>  13
<212>  DNA
<213>  Homo sapiens

<400>  314
ccaaactggg aaa                                                          13


<210>  315
<211>  13
<212>  DNA
<213>  Homo sapiens

<400>  315
caaactggga aac                                                          13


<210>  316
<211>  13
<212>  DNA
<213>  Homo sapiens

<400>  316
aactgggaaa ctg                                                          13


<210>  317
<211>  13
<212>  DNA
<213>  Homo sapiens

<400>  317
actgggaaac tgg                                                          13


<210>  318
<211>  13
<212>  DNA
<213>  Homo sapiens

<400>  318
ctgggaaact ggt                                                          13


<210>  319
<211>  13
<212>  DNA
<213>  Homo sapiens

<400>  319
tgggaaactg gtc                                                          13


<210>  320
<211>  13
<212>  DNA
<213>  Homo sapiens

<400>  320
gggaaactgg tct                                                          13


<210>  321
<211>  13
<212>  DNA
<213>  Homo sapiens

<400>  321
gaaactggtc tgc                                                          13


<210>  322
<211>  13
<212>  DNA
<213>  Homo sapiens

<400>  322
aaactggtct gcc                                                          13


<210>  323
<211>  13
<212>  DNA
<213>  Homo sapiens

<400>  323
aactggtctg cca                                                          13


<210>  324
<211>  13
<212>  DNA
<213>  Homo sapiens

<400>  324
actggtctgc cat                                                          13


<210>  325
<211>  13
<212>  DNA
<213>  Homo sapiens

<400>  325
ctggtctgcc atg                                                          13


<210>  326
<211>  13
<212>  DNA
<213>  Homo sapiens

<400>  326
tggtctgcca tgc                                                          13


<210>  327
<211>  13
<212>  DNA
<213>  Homo sapiens

<400>  327
ggtctgccat gcc                                                          13


<210>  328
<211>  13
<212>  DNA
<213>  Homo sapiens

<400>  328
gtctgccatg cca                                                          13


<210>  329
<211>  13
<212>  DNA
<213>  Homo sapiens

<400>  329
tctgccatgc caa                                                          13


<210>  330
<211>  13
<212>  DNA
<213>  Homo sapiens

<400>  330
ctgccatgcc aag                                                          13


<210>  331
<211>  13
<212>  DNA
<213>  Homo sapiens

<400>  331
tgccatgcca agt                                                          13


<210>  332
<211>  13
<212>  DNA
<213>  Homo sapiens

<400>  332
gccatgccaa gtt                                                          13


<210>  333
<211>  13
<212>  DNA
<213>  Homo sapiens

<400>  333
ccatgccaag ttg                                                          13


<210>  334
<211>  13
<212>  DNA
<213>  Homo sapiens

<400>  334
catgccaagt tgt                                                          13


<210>  335
<211>  13
<212>  DNA
<213>  Homo sapiens

<400>  335
caagttgtaa agc                                                          13


<210>  336
<211>  13
<212>  DNA
<213>  Homo sapiens

<400>  336
aaagtacctg tga                                                          13


<210>  337
<211>  13
<212>  DNA
<213>  Homo sapiens

<400>  337
gccactgtgg tgt                                                          13


<210>  338
<211>  13
<212>  DNA
<213>  Homo sapiens

<400>  338
cactgtggtg tac                                                          13


<210>  339
<211>  13
<212>  DNA
<213>  Homo sapiens

<400>  339
actgtggtgt acc                                                          13


<210>  340
<211>  13
<212>  DNA
<213>  Homo sapiens

<400>  340
ctgtggtgta cca                                                          13


<210>  341
<211>  13
<212>  DNA
<213>  Homo sapiens

<400>  341
tgtggtgtac caa                                                          13


<210>  342
<211>  13
<212>  DNA
<213>  Homo sapiens

<400>  342
gtggtgtacc aag                                                          13


<210>  343
<211>  13
<212>  DNA
<213>  Homo sapiens

<400>  343
tggtgtacca agg                                                          13


<210>  344
<211>  13
<212>  DNA
<213>  Homo sapiens

<400>  344
ggtgtaccaa gga                                                          13


<210>  345
<211>  13
<212>  DNA
<213>  Homo sapiens

<400>  345
gtgtaccaag gag                                                          13


<210>  346
<211>  13
<212>  DNA
<213>  Homo sapiens

<400>  346
tgtaccaagg aga                                                          13


<210>  347
<211>  13
<212>  DNA
<213>  Homo sapiens

<400>  347
gtaccaagga gag                                                          13


<210>  348
<211>  13
<212>  DNA
<213>  Homo sapiens

<400>  348
taccaaggag aga                                                          13


<210>  349
<211>  13
<212>  DNA
<213>  Homo sapiens

<400>  349
accaaggaga gag                                                          13


<210>  350
<211>  13
<212>  DNA
<213>  Homo sapiens

<400>  350
ccaaggagag aga                                                          13


<210>  351
<211>  13
<212>  DNA
<213>  Homo sapiens

<400>  351
caaggagaga gag                                                          13


<210>  352
<211>  13
<212>  DNA
<213>  Homo sapiens

<400>  352
aaggagagag agt                                                          13


<210>  353
<211>  13
<212>  DNA
<213>  Homo sapiens

<400>  353
aggagagaga gta                                                          13


<210>  354
<211>  13
<212>  DNA
<213>  Homo sapiens

<400>  354
ggagagagag taa                                                          13


<210>  355
<211>  13
<212>  DNA
<213>  Homo sapiens

<400>  355
aagaatggaa tgc                                                          13


<210>  356
<211>  13
<212>  DNA
<213>  Homo sapiens

<400>  356
agaatggaat gct                                                          13


<210>  357
<211>  13
<212>  DNA
<213>  Homo sapiens

<400>  357
aagaagtgta gct                                                          13


<210>  358
<211>  13
<212>  DNA
<213>  Homo sapiens

<400>  358
agaagtgtag cta                                                          13


<210>  359
<211>  13
<212>  DNA
<213>  Homo sapiens

<400>  359
ccaaatgctt caa                                                          13


<210>  360
<211>  13
<212>  DNA
<213>  Homo sapiens

<400>  360
tgcttcaagg aac                                                          13


<210>  361
<211>  13
<212>  DNA
<213>  Homo sapiens

<400>  361
gcttcaagga aca                                                          13


<210>  362
<211>  13
<212>  DNA
<213>  Homo sapiens

<400>  362
cttcaaggaa cac                                                          13


<210>  363
<211>  13
<212>  DNA
<213>  Homo sapiens

<400>  363
ttcaaggaac aca                                                          13


<210>  364
<211>  13
<212>  DNA
<213>  Homo sapiens

<400>  364
tcaaggaaca cag                                                          13


<210>  365
<211>  13
<212>  DNA
<213>  Homo sapiens

<400>  365
caaggaacac agt                                                          13


<210>  366
<211>  13
<212>  DNA
<213>  Homo sapiens

<400>  366
aaggaacaca gtt                                                          13


<210>  367
<211>  13
<212>  DNA
<213>  Homo sapiens

<400>  367
aggaacacag ttc                                                          13


<210>  368
<211>  13
<212>  DNA
<213>  Homo sapiens

<400>  368
ggaacacagt tct                                                          13


<210>  369
<211>  13
<212>  DNA
<213>  Homo sapiens

<400>  369
gaacacagtt ctc                                                          13


<210>  370
<211>  13
<212>  DNA
<213>  Homo sapiens

<400>  370
aacacagttc tct                                                          13


<210>  371
<211>  13
<212>  DNA
<213>  Homo sapiens

<400>  371
acacagttct ctg                                                          13


<210>  372
<211>  13
<212>  DNA
<213>  Homo sapiens

<400>  372
cacagttctc tgg                                                          13


<210>  373
<211>  13
<212>  DNA
<213>  Homo sapiens

<400>  373
acagttctct ggc                                                          13


<210>  374
<211>  13
<212>  DNA
<213>  Homo sapiens

<400>  374
cagttctctg gct                                                          13


<210>  375
<211>  13
<212>  DNA
<213>  Homo sapiens

<400>  375
agttctctgg ctt                                                          13


<210>  376
<211>  13
<212>  DNA
<213>  Homo sapiens

<400>  376
ggaaaactga tgc                                                          13


<210>  377
<211>  13
<212>  DNA
<213>  Homo sapiens

<400>  377
gaaaactgat gca                                                          13


<210>  378
<211>  13
<212>  DNA
<213>  Homo sapiens

<400>  378
aaactgatgc atc                                                          13


<210>  379
<211>  13
<212>  DNA
<213>  Homo sapiens

<400>  379
aactgatgca tcc                                                          13


<210>  380
<211>  13
<212>  DNA
<213>  Homo sapiens

<400>  380
actgatgcat ccg                                                          13


<210>  381
<211>  13
<212>  DNA
<213>  Homo sapiens

<400>  381
ctgatgcatc cga                                                          13


<210>  382
<211>  13
<212>  DNA
<213>  Homo sapiens

<400>  382
tgatgcatcc gat                                                          13


<210>  383
<211>  13
<212>  DNA
<213>  Homo sapiens

<400>  383
gatgcatccg atg                                                          13


<210>  384
<211>  13
<212>  DNA
<213>  Homo sapiens

<400>  384
atgcatccga tgt                                                          13


<210>  385
<211>  13
<212>  DNA
<213>  Homo sapiens

<400>  385
tgcatccgat gta                                                          13


<210>  386
<211>  13
<212>  DNA
<213>  Homo sapiens

<400>  386
gcatccgatg taa                                                          13


<210>  387
<211>  13
<212>  DNA
<213>  Homo sapiens

<400>  387
tccgatgtaa agc                                                          13


<210>  388
<211>  13
<212>  DNA
<213>  Homo sapiens

<400>  388
ccgatgtaaa gcc                                                          13


<210>  389
<211>  13
<212>  DNA
<213>  Homo sapiens

<400>  389
cgatgtaaag cca                                                          13


<210>  390
<211>  13
<212>  DNA
<213>  Homo sapiens

<400>  390
ttcagattcc aca                                                          13


<210>  391
<211>  13
<212>  DNA
<213>  Homo sapiens

<400>  391
aaaatgtcac act                                                          13


<210>  392
<211>  13
<212>  DNA
<213>  Homo sapiens

<400>  392
aaatgtcaca ctt                                                          13


<210>  393
<211>  13
<212>  DNA
<213>  Homo sapiens

<400>  393
tgttcatcca agg                                                          13


<210>  394
<211>  13
<212>  DNA
<213>  Homo sapiens

<400>  394
gttcatccaa gga                                                          13


<210>  395
<211>  13
<212>  DNA
<213>  Homo sapiens

<400>  395
tcatccaagg aac                                                          13


<210>  396
<211>  13
<212>  DNA
<213>  Homo sapiens

<400>  396
catccaagga acc                                                          13


<210>  397
<211>  13
<212>  DNA
<213>  Homo sapiens

<400>  397
atccaaggaa cct                                                          13


<210>  398
<211>  13
<212>  DNA
<213>  Homo sapiens

<400>  398
tccaaggaac cta                                                          13


<210>  399
<211>  13
<212>  DNA
<213>  Homo sapiens

<400>  399
ccaaggaacc taa                                                          13


<210>  400
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  2'-O-methyladenosine

<400>  400
gagcactgga gaa                                                          13


<210>  401

<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  2'-O-methyladenosine

<400>  401
ugagcactgg aga                                                          13


<210>  402
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)

<223>  um

<220>
<221>  modified_base
<222>  (12)..(12)
<223>  gm

<220>
<221>  modified_base
<222>  (13)..(13)
<223>  gm

<400>  402
agatgagcac ugg                                                          13


<210>  403
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  403
agcaacatgg cag                                                          13


<210>  404
<211>  13
<212>  DNA
<213>  Artificial Sequence

<220>
<223>  Synthetic oligonucleotide:Combined DNA/RNA Molecule: nucleotides

3 to 10 are deoxyribonucleotides-other nucleotides are
ribonucleotides

<220>
<221> modified_base
<222> (1)..(1)
<223> um

<220>
<221> modified_base
<222> (2)..(2)
<223> 2'-O-methyladenosine

<220>
<221> modified_base
<222> (11)..(11)
<223> gm

<220>
<221> modified_base
<222> (12)..(12)
<223> cm

<220>
<221> modified_base
<222> (13)..(13)
<223> 2'-O-methyladenosine

<400> 404
uagcaacatg gca 13

<210> 405
<211> 13
<212> DNA
<213> Artificial Sequence

<220>
<223> Synthetic oligonucleotide:Combined DNA/RNA Molecule: nucleotides
3 to 10 are deoxyribonucleotides-other nucleotides are
ribonucleotides

<220>
<221> modified_base
<222> (1)..(1)
<223> 2'-O-methyladenosine

<220>
<221> modified_base
<222> (2)..(2)
<223> um

<220>
<221> modified_base
<222> (11)..(11)
<223> gm

<220>
<221> modified_base
<222> (12)..(12)
<223> gm

<220>
<221>  modified_base
<222>  (13)..(13)
<223>  cm

<400>  405
auagcaacat ggc                                                          13


<210>  406
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  cm

<400>  406
gucctgcaat agc                                                          13


<210>  407
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base

<222>  (1)..(1)
<223>  gm

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  2'-O-methyladenosine

<400>  407
guccgtcctg caa                                                          13


<210>  408
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  2'-O-methyladenosine

<400>  408
ggtccgtcct gca                                                          13


<210>  409
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  cm

<400>  409
aggtccgtcc ugc                                                          13


<210>  410
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)

<223>  2'-O-methyladenosine

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  gm

<400>  410
caggtccgtc cug                                                          13


<210>  411
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  cm

<220>
<221>  modified_base
<222>  (13)..(13)
<223>  gm

<400>  411
ugggacaggt ccg                                                          13


<210>  412
<211>  13

<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  cm

<400>  412
uugggacagg ucc                                                          13

<210>  413
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  gm

<400>  413
ggcttgggac agg                                                          13


<210>  414
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  414
uggcttggga cag                                                          13


<210>  415
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are

ribonucleotides

<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  2'-O-methyladenosine

<220>
<221>  modified_base
<222>  (12)..(12)
<223>  cm

<220>
<221>  modified_base
<222>  (13)..(13)
<223>  2'-O-methyladenosine

<400>  415
cuggcttggg aca                                                          13

<210>  416
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  cm

<400>  416
uctggcttgg gac                                                          13


<210>  417
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  gm

<400>  417
catctggctt ggg                                                          13


<210>  418
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)

<223>  um

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  418
ucatctggct ugg                                                          13


<210>  419
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  419

aucatctggc uug                                                          13


<210>  420
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  um

<400>  420
aatcatctgg cuu                                                          13


<210>  421
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  um

<400>  421
aaatcatctg gcu                                                          13


<210>  422
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  2'-O-methyladenosine

<220>
<221>  modified_base
<222>  (13)..(13)
<223>  gm

<400>  422
ucctggctca uag                                                          13


<210>  423
<211>  13
<212>  DNA

<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  2'-O-methyladenosine

<220>
<221>  modified_base
<222>  (12)..(12)
<223>  um

<220>
<221>  modified_base
<222>  (13)..(13)
<223>  2'-O-methyladenosine

<400>  423
cucctggctc aua                                                          13


<210>  424
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  um

<400>  424
uctcctggct cau                                                          13


<210>  425
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  2'-O-methyladenosine

<400>  425
ccggcttgca gga                                                          13


<210>  426
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  gm

<400>  426
cccggcttgc agg                                                          13


<210>  427
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  2'-O-methyladenosine

<220>

<221>  modified_base
<222>  (13)..(13)
<223>  gm

<400>  427
gcccggcttg cag                                                          13


<210>  428
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  2'-O-methyladenosine

<400>  428
agcccggctt gca                                                          13


<210>  429
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  cm

<400>  429
uagcccggct ugc                                                          13


<210>  430
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  430
auagcccggc uug                                                          13

<210>  431
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  um

<400>  431
catagcccgg cuu                                                          13


<210>  432
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  gm

<220>
<221>  modified_base
<222>  (13)..(13)
<223>  gm

<400>  432
acacatagcc cgg                                                          13


<210>  433
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  cm

<220>
<221>  modified_base
<222>  (13)..(13)
<223>  gm

<400>  433
gacacatagc ccg                                                          13


<210>  434
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  434
ugtgagaggg cag                                                          13

<210>  435
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  2'-O-methyladenosine

<220>

<221>  modified_base
<222>  (12)..(12)
<223>  cm

<220>
<221>  modified_base
<222>  (13)..(13)
<223>  2'-O-methyladenosine

<400>  435
uugatgggcc aca                                                          13


<210>  436
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  cm

<400>  436
gutgatgggc cac                                                          13


<210>  437
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  cm

<220>
<221>  modified_base
<222>  (13)..(13)
<223>  2'-O-methyladenosine

<400>  437
ugttgatggg cca                                                          13


<210>  438
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  cm

<220>
<221>  modified_base

<222>  (13)..(13)
<223>  cm

<400>  438
gugttgatgg gcc                                                          13


<210>  439
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  439
ggacatactc ugg                                                          13


<210>  440
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  gm

<400>  440
aggacatact cug                                                          13


<210>  441
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  2'-O-methyladenosine

<400>  441
gctccatttt cua                                                          13

<210>  442
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  2'-O-methyladenosine

<400>  442
gutgggatat uca                                                          13

<210>  443
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  gm

<220>
<221>  modified_base
<222>  (2)..(2)
<223>  cm

<220>

<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  2'-O-methyladenosine

<400>  443
gcacttggca gaa                                                          13


<210>  444
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  2'-O-methyladenosine

<400>  444
ugcacttggc aga                                                          13


<210>  445
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  445
gugcacttgg cag                                                          13

<210>  446
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  gm

<220>
<221>  modified_base

<222>  (12)..(12)
<223>  cm

<220>
<221>  modified_base
<222>  (13)..(13)
<223>  2'-O-methyladenosine

<400>  446
agtgcacttg gca                                                          13


<210>  447
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  cm

<400>  447
cagtgcactt ggc                                                          13


<210>  448
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  448
cucagtgcac uug                                                          13


<210>  449
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  um

<220>
<221>  modified_base
<222>  (13)..(13)

<223>  um

<400>  449
cctcagtgca cuu                                                          13


<210>  450
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  2'-O-methyladenosine

<220>
<221>  modified_base
<222>  (12)..(12)
<223>  cm

<220>
<221>  modified_base
<222>  (13)..(13)
<223>  um

<400>  450
ucctcagtgc acu                                                          13


<210>  451
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>

<221>  modified_base
<222>  (2)..(2)
<223>  um

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  cm

<400>  451
uucctcagtg cac                                                          13


<210>  452
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  2'-O-methyladenosine

<400>  452
cutcctcagt gca                                                          13

<210>  453
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  cm

<400>  453
ccttcctcag ugc                                                          13


<210>  454
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  cm

<220>
<221>  modified_base

<222>  (11)..(11)
<223>  gm

<220>
<221>  modified_base
<222>  (12)..(12)
<223>  um

<220>
<221>  modified_base
<222>  (13)..(13)
<223>  gm

<400>  454
uccttcctca gug                                                          13


<210>  455
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  2'-O-methyladenosine

<220>
<221>  modified_base
<222>  (13)..(13)
<223>  um

<400>  455
gctgatggct uau                                                          13


<210>  456
<211>  13
<212>  DNA
<213>  Artificial Sequence

<220>

<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  2'-O-methyladenosine

<400>  456
agctgatggc uua                                                          13

<210>  457
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)

<223>  um

<220>
<221>  modified_base
<222>  (13)..(13)
<223>  um

<400>  457
cagctgatgg cuu                                                          13


<210>  458
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  um

<400>  458
ccagctgatg gcu                                                          13


<210>  459
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>

<221>  modified_base
<222>  (1)..(1)
<223>  um

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  cm

<400>  459
uccagctgat ggc                                                          13


<210>  460
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  460
uuccagctga ugg                                                          13


<210>  461
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  um

<400>  461
guttccagct gau                                                          13


<210>  462
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base

<222>  (2)..(2)
<223>  gm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  2'-O-methyladenosine

<400>  462
ugtttccagc uga                                                          13


<210>  463
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  gm

<400>  463
uugtttccag cug                                                          13


<210>  464

<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  cm

<400>  464
aactgctgtg ucc                                                          13

<210>  465
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)

<223>  gm

<220>
<221>  modified_base
<222>  (12)..(12)
<223>  um

<220>
<221>  modified_base
<222>  (13)..(13)
<223>  gm

<400>  465
aaaaactgct gug                                                          13


<210>  466
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  2'-O-methyladenosine

<400>  466
uggcaaacat uca                                                          13


<210>  467
<211>  13
<212>  DNA
<213>  Artificial Sequence

<220>
<223>  Synthetic oligonucleotide:Combined DNA/RNA Molecule: nucleotides

3 to 10 are deoxyribonucleotides-other nucleotides are
ribonucleotides

<220>
<221> modified_base
<222> (1)..(1)
<223> gm

<220>
<221> modified_base
<222> (2)..(2)
<223> um

<220>
<221> modified_base
<222> (11)..(11)
<223> um

<220>
<221> modified_base
<222> (12)..(12)
<223> um

<220>
<221> modified_base
<222> (13)..(13)
<223> cm

<400> 467
guggcaaaca uuc 13

<210> 468
<211> 13
<212> DNA
<213> Artificial Sequence

<220>
<223> Synthetic oligonucleotide:Combined DNA/RNA Molecule: nucleotides
3 to 10 are deoxyribonucleotides-other nucleotides are
ribonucleotides

<220>
<221> modified_base
<222> (1)..(1)
<223> gm

<220>
<221> modified_base
<222> (2)..(2)
<223> um

<220>
<221> modified_base
<222> (11)..(11)
<223> 2'-O-methyladenosine

<220>
<221> modified_base
<222> (12)..(12)
<223> cm

<220>
<221>  modified_base
<222>  (13)..(13)
<223>  2'-O-methyladenosine

<400>  468
gutgtggcaa aca                                                          13


<210>  469
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  2'-O-methyladenosine

<220>
<221>  modified_base
<222>  (13)..(13)
<223>  cm

<400>  469
ugttgtggca aac                                                          13


<210>  470
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base

<222>  (1)..(1)
<223>  gm

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  2'-O-methyladenosine

<400>  470
gcatgttgtg gca                                                          13


<210>  471
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  cm

<400>  471
cgcatgttgt ggc                                                          13


<210>  472
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  472
ucgcatgttg ugg                                                          13


<210>  473
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)

<223>  um

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  gm

<400>  473
aucgcatgtt gug                                                          13


<210>  474
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  474
acatcgcatg uug                                                          13


<210>  475
<211>  13

<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  gm

<220>
<221>  modified_base
<222>  (13)..(13)
<223>  cm

<400>  475
uuccaaacat cgc                                                          13


<210>  476
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  cm

<220>
<221>  modified_base
<222>  (13)..(13)
<223>  2'-O-methyladenosine

<400>  476
agtccaattt cca                                                          13


<210>  477
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  477
ccctgcattc ugg                                                          13


<210>  478
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are

ribonucleotides

<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  gm

<400>  478
uccctgcatt cug                                                          13

<210>  479
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  um

<400>  479
uuccctgcat ucu                                                          13


<210>  480
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  um

<400>  480
autgtctggt cuu                                                          13


<210>  481
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)

<223>  cm

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  um

<400>  481
cattgtctgg ucu                                                          13


<210>  482
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  cm

<400>  482

ccattgtctg guc                                                          13


<210>  483
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  um

<400>  483
uccattgtct ggu                                                          13


<210>  484
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  484
auccattgtc ugg                                                          13


<210>  485
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  gm

<400>  485
aatccattgt cug                                                          13


<210>  486
<211>  13
<212>  DNA

<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  2'-O-methyladenosine

<400>  486
gcaggatagt uca                                                          13


<210>  487
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  um

<400>  487
ggttttgcag gau                                                          13


<210>  488
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  2'-O-methyladenosine

<400>  488
uggttttgca gga                                                          13


<210>  489
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  489
ggctttatcc uug                                                          13


<210>  490
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  cm

<220>

<221>  modified_base
<222>  (13)..(13)
<223>  um

<400>  490
guggctttat ccu                                                          13


<210>  491
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  cm

<400>  491
catcatggca gcc                                                          13


<210>  492
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  cm

<400>  492
ccatcatggc agc                                                          13


<210>  493
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  493
uccatcatgg cag                                                          13

<210>  494
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  2'-O-methyladenosine

<400>  494
auccatcatg gca                                                          13


<210>  495
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  cm

<400>  495
uatccatcat ggc                                                          13


<210>  496
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  496
auatccatca ugg                                                          13


<210>  497
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  2'-O-methyladenosine

<220>
<221>  modified_base
<222>  (13)..(13)
<223>  cm

<400>  497
cccagtttgg uac                                                          13

<210>  498
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  gm

<220>

<221>  modified_base
<222>  (12)..(12)
<223>  um

<220>
<221>  modified_base
<222>  (13)..(13)
<223>  2'-O-methyladenosine

<400>  498
ucccagtttg gua                                                          13


<210>  499
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  um

<400>  499
uucccagttt ggu                                                          13


<210>  500
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  500
uutcccagtt ugg                                                          13


<210>  501
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  um

<220>
<221>  modified_base

<222>  (13)..(13)
<223>  gm

<400>  501
guttcccagt uug                                                          13


<210>  502
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  um

<400>  502
cagtttccca guu                                                          13


<210>  503
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  um

<400>  503
ccagtttccc agu                                                          13


<210>  504
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  504
accagtttcc cag                                                          13

<210>  505
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  gm

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  cm

<220>
<221>  modified_base
<222>  (13)..(13)
<223>  2'-O-methyladenosine

<400>  505
gaccagtttc cca                                                          13

<210>  506
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2'-O-methyladenosine

<220>
<221>  modified_base
<222>  (2)..(2)
<223>  gm

<220>

<221>  modified_base
<222>  (11)..(11)
<223>  cm

<220>
<221>  modified_base
<222>  (12)..(12)
<223>  cm

<220>
<221>  modified_base
<222>  (13)..(13)
<223>  cm

<400>  506
agaccagttt ccc                                                          13


<210>  507
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  cm

<400>  507
gcagaccagt uuc                                                          13


<210>  508
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  um

<400>  508
ggcagaccag uuu                                                          13


<210>  509
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  gm

<220>
<221>  modified_base

<222>  (12)..(12)
<223>  um

<220>
<221>  modified_base
<222>  (13)..(13)
<223>  um

<400>  509
uggcagacca guu                                                          13


<210>  510
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  um

<400>  510
auggcagacc agu                                                          13


<210>  511
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  511
catggcagac cag                                                          13


<210>  512
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  cm

<220>
<221>  modified_base
<222>  (13)..(13)

<223>  2'-O-methyladenosine

<400>  512
gcatggcaga cca                                                          13


<210>  513
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  cm

<220>
<221>  modified_base
<222>  (13)..(13)
<223>  cm

<400>  513
ggcatggcag acc                                                          13


<210>  514
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>

<221>  modified_base
<222>  (2)..(2)
<223>  gm

<220>
<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  cm

<400>  514
uggcatggca gac                                                          13


<210>  515
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  2'-O-methyladenosine

<400>  515
uuggcatggc aga                                                          13

<210>  516
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  516
cutggcatgg cag                                                          13


<210>  517
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base

<222>   (11)..(11)
<223>   gm

<220>
<221>   modified_base
<222>   (12)..(12)
<223>   cm

<220>
<221>   modified_base
<222>   (13)..(13)
<223>   2'-O-methyladenosine

<400>   517
acttggcatg gca                                                          13


<210>   518
<211>   13
<212>   DNA
<213>   Artificial Sequence

<220>
<223>   Synthetic oligonucleotide:Combined DNA/RNA Molecule: nucleotides
        3 to 10 are deoxyribonucleotides-other nucleotides are
        ribonucleotides


<220>
<221>   modified_base
<222>   (1)..(1)
<223>   2'-O-methyladenosine

<220>
<221>   modified_base
<222>   (2)..(2)
<223>   2'-O-methyladenosine

<220>
<221>   modified_base
<222>   (11)..(11)
<223>   gm

<220>
<221>   modified_base
<222>   (12)..(12)
<223>   gm

<220>
<221>   modified_base
<222>   (13)..(13)
<223>   cm

<400>   518
aacttggcat ggc                                                          13


<210>   519
<211>   13
<212>   DNA
<213>   Artificial Sequence

<220>

<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  519
caacttggca ugg                                                          13


<210>  520
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  2'-O-methyladenosine

<220>
<221>  modified_base
<222>  (12)..(12)

<223>  um

<220>
<221>  modified_base
<222>  (13)..(13)
<223>  gm

<400>  520
acaacttggc aug                                                          13


<210>  521
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  521
gctttacaac uug                                                          13


<210>  522
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>

<221>  modified_base
<222>  (1)..(1)
<223>  um

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  um

<400>  522
ucacaggtac uuu                                                          13


<210>  523
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  cm

<400>  523
acaccacagt ggc                                                          13


<210>  524
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  gm

<400>  524
guacaccaca gug                                                          13


<210>  525
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base

<222>  (2)..(2)
<223>  gm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  um

<400>  525
ggtacaccac agu                                                          13


<210>  526
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  526
uggtacacca cag                                                          13


<210>  527

<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  2'-O-methyladenosine

<220>
<221>  modified_base
<222>  (12)..(12)
<223>  cm

<220>
<221>  modified_base
<222>  (13)..(13)
<223>  2'-O-methyladenosine

<400>  527
uuggtacacc aca                                                          13


<210>  528
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  um

<220>
<221>  modified_base
<222>  (11)..(11)

<223>  cm

<220>
<221>  modified_base
<222>  (12)..(12)
<223>  2'-O-methyladenosine

<220>
<221>  modified_base
<222>  (13)..(13)
<223>  cm

<400>  528
cutggtacac cac                                                          13


<210>  529
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  cm

<220>
<221>  modified_base
<222>  (13)..(13)
<223>  2'-O-methyladenosine

<400>  529
ccttggtaca cca                                                          13


<210>  530
<211>  13
<212>  DNA
<213>  Artificial Sequence

<220>
<223>  Synthetic oligonucleotide:Combined DNA/RNA Molecule: nucleotides

3 to 10 are deoxyribonucleotides-other nucleotides are
ribonucleotides

<220>
<221> modified_base
<222> (1)..(1)
<223> um

<220>
<221> modified_base
<222> (2)..(2)
<223> cm

<220>
<221> modified_base
<222> (11)..(11)
<223> 2'-O-methyladenosine

<220>
<221> modified_base
<222> (12)..(12)
<223> cm

<220>
<221> modified_base
<222> (13)..(13)
<223> cm

<400> 530
uccttggtac acc 13

<210> 531
<211> 13
<212> DNA
<213> Artificial Sequence

<220>
<223> Synthetic oligonucleotide:Combined DNA/RNA Molecule: nucleotides
3 to 10 are deoxyribonucleotides-other nucleotides are
ribonucleotides

<220>
<221> modified_base
<222> (1)..(1)
<223> cm

<220>
<221> modified_base
<222> (2)..(2)
<223> um

<220>
<221> modified_base
<222> (11)..(11)
<223> cm

<220>
<221> modified_base
<222> (12)..(12)
<223> 2'-O-methyladenosine

<220>
<221>  modified_base
<222>  (13)..(13)
<223>  cm

<400>  531
cuccttggta cac                                                          13


<210>  532
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  2'-O-methyladenosine

<220>
<221>  modified_base
<222>  (12)..(12)
<223>  cm

<220>
<221>  modified_base
<222>  (13)..(13)
<223>  2'-O-methyladenosine

<400>  532
uctccttggt aca                                                          13


<210>  533
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base

<222>  (1)..(1)
<223>  cm

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  2'-O-methyladenosine

<220>
<221>  modified_base
<222>  (13)..(13)
<223>  cm

<400>  533
cuctccttgg uac                                                          13


<210>  534
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  2'-O-methyladenosine

<400>  534
uctctccttg gua                                                          13


<210>  535
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  um

<400>  535
cuctctcctt ggu                                                          13


<210>  536
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)

<223>  cm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  536
uctctctcct ugg                                                          13


<210>  537
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  537
cuctctctcc uug                                                          13


<210>  538
<211>  13

<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides



<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  um

<400>  538
actctctctc cuu                                                          13


<210>  539
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides



<220>
<221>  modified_base
<222>  (1)..(1)
<223>  um

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  cm

<220>
<221>  modified_base
<222>  (13)..(13)
<223>  um

<400>  539
uactctctct ccu                                                          13


<210>  540
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  cm

<400>  540
uuactctctc ucc                                                          13


<210>  541
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are

ribonucleotides

<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  um

<400>  541
gcattccatt cuu                                                          13

<210>  542
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  um

<400>  542
agcattccat ucu                                                          13


<210>  543
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  um

<400>  543
agctacactt cuu                                                          13


<210>  544
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)

<223>  um

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  um

<400>  544
uagctacact ucu                                                          13


<210>  545
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  545

uugaagcatt ugg                                                          13

<210>  546
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  2'-O-methyladenosine

<400>  546
gutccttgaa gca                                                          13

<210>  547
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  cm

<400>  547
ugttccttga agc                                                          13


<210>  548
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  2'-O-methyladenosine

<220>
<221>  modified_base
<222>  (12)..(12)
<223>  2'-O-methyladenosine

<220>
<221>  modified_base
<222>  (13)..(13)
<223>  gm

<400>  548
gugttccttg aag                                                          13


<210>  549
<211>  13
<212>  DNA

<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  2'-O-methyladenosine

<400>  549
ugtgttcctt gaa                                                          13


<210>  550
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  2'-O-methyladenosine

<400>  550
cugtgttcct uga                                                          13


<210>  551
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  gm

<400>  551
actgtgttcc uug                                                          13


<210>  552
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  um

<400>  552
aactgtgttc cuu                                                          13


<210>  553
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  2'-O-methyladenosine

<220>
<221>  modified_base
<222>  (11)..(11)
<223>  cm

<220>
<221>  modified_base
<222>  (12)..(12)
<223>  cm

<220>

<221>  modified_base
<222>  (13)..(13)
<223>  um

<400>  553
gaactgtgtt ccu                                                          13


<210>  554
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  cm

<400>  554
agaactgtgt ucc                                                          13


<210>  555
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  cm

<400>  555
gagaactgtg uuc                                                          13


<210>  556
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  um

<400>  556
agagaactgt guu                                                          13

<210>  557
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  um

<400>  557
cagagaactg ugu                                                          13


<210>  558
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  gm

<400>  558
ccagagaact gug                                                          13


<210>  559
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  um

<400>  559
gccagagaac ugu                                                          13


<210>  560
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  um

<220>
<221>  modified_base
<222>  (13)..(13)
<223>  gm

<400>  560
agccagagaa cug                                                          13


<210>  561
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)
<223>  2'-O-methyladenosine

<220>

<221>  modified_base
<222>  (12)..(12)
<223>  cm

<220>
<221>  modified_base
<222>  (13)..(13)
<223>  um

<400>  561
aagccagaga acu                                                          13


<210>  562
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  cm

<400>  562
gcatcagttt ucc                                                          13


<210>  563
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  cm

<400>  563
ugcatcagtt uuc                                                          13


<210>  564
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  gm

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  um

<220>
<221>  modified_base

<222>  (13)..(13)
<223>  um

<400>  564
gatgcatcag uuu                                                          13


<210>  565
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  um

<220>
<221>  modified_base
<222>  (13)..(13)
<223>  um

<400>  565
ggatgcatca guu                                                          13


<210>  566
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  gm

<220>
<221>  modified_base
<222>  (13)..(13)
<223>  um

<400>  566
cggatgcatc agu                                                          13


<210>  567
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  gm

<400>  567
ucggatgcat cag                                                          13

<210>  568
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  cm

<220>
<221>  modified_base
<222>  (13)..(13)
<223>  2'-O-methyladenosine

<400>  568
aucggatgca uca                                                          13


<210>  569
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  2'-O-methyladenosine

<220>

<221>  modified_base
<222>  (11)..(11)
<223>  2'-O-methyladenosine

<220>
<221>  modified_base
<222>  (12)..(12)
<223>  um

<220>
<221>  modified_base
<222>  (13)..(13)
<223>  cm

<400>  569
catcggatgc auc                                                          13


<210>  570
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  cm

<220>
<221>  modified_base
<222>  (11)..(11)
<223>  cm

<220>
<221>  modified_base
<222>  (12)..(12)
<223>  2'-O-methyladenosine

<220>
<221>  modified_base
<222>  (13)..(13)
<223>  um

<400>  570
acatcggatg cau                                                          13


<210>  571
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  cm

<220>
<221>  modified_base
<222>  (13)..(13)
<223>  2'-O-methyladenosine

<400>  571
uacatcggat gca                                                          13


<210>  572
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base

<222>  (12)..(12)
<223>  gm

<220>
<221>  modified_base
<222>  (13)..(13)
<223>  cm

<400>  572
uuacatcgga ugc                                                          13


<210>  573
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  cm

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  2'-O-methyladenosine

<400>  573
gctttacatc gga                                                          13


<210>  574
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  cm

<220>
<221>  modified_base
<222>  (12)..(12)
<223>  gm

<220>
<221>  modified_base
<222>  (13)..(13)
<223>  gm

<400>  574
ggctttacat cgg                                                          13


<210>  575
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  um

<220>
<221>  modified_base
<222>  (12)..(12)
<223>  cm

<220>
<221>  modified_base
<222>  (13)..(13)

<223>  gm

<400>  575
uggctttaca ucg                                                          13


<210>  576
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  gm

<220>
<221>  modified_base
<222>  (11)..(11)
<223>  gm

<220>
<221>  modified_base
<222>  (12)..(12)
<223>  2'-O-methyladenosine

<220>
<221>  modified_base
<222>  (13)..(13)
<223>  2'-O-methyladenosine

<400>  576
ugtggaatct gaa                                                          13


<210>  577
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2'-O-methyladenosine

<220>

<221>  modified_base
<222>  (2)..(2)
<223>  gm

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  um

<400>  577
agtgtgacat uuu                                                          13


<210>  578
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2'-O-methyladenosine

<220>
<221>  modified_base
<222>  (2)..(2)
<223>  2'-O-methyladenosine

<220>
<221>  modified_base
<222>  (11)..(11)
<223>  um

<220>
<221>  modified_base
<222>  (12)..(12)
<223>  um

<220>
<221>  modified_base
<222>  (13)..(13)
<223>  um

<400>  578
aagtgtgaca uuu                                                          13

<210>  579
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  cm

<220>
<221>  modified_base
<222>  (2)..(2)
<223>  cm

<220>
<221>  modified_base
<222>  (11)..(11)
<223>  2'-O-methyladenosine

<220>
<221>  modified_base
<222>  (12)..(12)
<223>  cm

<220>
<221>  modified_base
<222>  (13)..(13)
<223>  2'-O-methyladenosine

<400>  579
ccttggatga aca                                                          13


<210>  580
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  cm

<220>
<221>  modified_base

<222>  (11)..(11)
<223>  2'-O-methyladenosine

<220>
<221>  modified_base
<222>  (12)..(12)
<223>  2'-O-methyladenosine

<220>
<221>  modified_base
<222>  (13)..(13)
<223>  cm

<400>  580
uccttggatg aac                                                          13


<210>  581
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  gm

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  2'-O-methyladenosine

<400>  581
gutccttgga uga                                                          13


<210>  582
<211>  13
<212>  DNA
<213>  Artificial Sequence

<220>

<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  gm

<220>
<221>  modified_base
<222>  (2)..(2)
<223>  gm

<220>
<221>  modified_base
<222>  (11)..(11)
<223>  2'-O-methyladenosine

<220>
<221>  modified_base
<222>  (12)..(12)
<223>  um

<220>
<221>  modified_base
<222>  (13)..(13)
<223>  gm

<400>  582
ggttccttgg aug                                                          13

<210>  583
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  2'-O-methyladenosine

<220>
<221>  modified_base
<222>  (2)..(2)
<223>  gm

<220>
<221>  modified_base
<222>  (11)..(11)
<223>  gm

<220>
<221>  modified_base
<222>  (12)..(12)

<223>  2'-O-methyladenosine

<220>
<221>  modified_base
<222>  (13)..(13)
<223>  um

<400>  583
aggttccttg gau                                                          13


<210>  584
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  um

<220>
<221>  modified_base
<222>  (2)..(2)
<223>  2'-O-methyladenosine

<220>
<221>  modified_base
<222>  (11)..(11)
<223>  gm

<220>
<221>  modified_base
<222>  (12)..(12)
<223>  gm

<220>
<221>  modified_base
<222>  (13)..(13)
<223>  2'-O-methyladenosine

<400>  584
uaggttcctt gga                                                          13


<210>  585
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>

<221>  modified_base
<222>  (1)..(1)
<223>  um

<220>
<221>  modified_base
<222>  (2)..(2)
<223>  um

<220>
<221>  modified_base
<222>  (11)..(11)
<223>  um

<220>
<221>  modified_base
<222>  (12)..(12)
<223>  gm

<220>
<221>  modified_base
<222>  (13)..(13)
<223>  gm

<400>  585
uuaggttcct ugg                                                          13


<210>  586
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)

<223>  2 ′-O,4 ′-C-methylene- linked bicyclic ribonucleotide

<400>  586
tagcaacatg gca                                                          13


<210>  587
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 ′-O,4 ′-C-methylene- linked bicyclic ribonucleotide

<220>
<221>  modified_base
<222>  (2)..(2)
<223>  2 ′-O,4 ′-C-methylene- linked bicyclic ribonucleotide

<220>
<221>  modified_base
<222>  (11)..(11)
<223>  2 ′-O,4 ′-C-methylene- linked bicyclic ribonucleotide

<220>
<221>  modified_base
<222>  (12)..(12)
<223>  2 ′-O,4 ′-C-methylene- linked bicyclic ribonucleotide

<220>
<221>  modified_base
<222>  (13)..(13)
<223>  5-methylcytosine; 2 ′-O,4 ′-C-methylene- linked bicyclic
       ribonucleotide

<400>  587
atagcaacat ggc                                                          13


<210>  588
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 ′-O,4 ′-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  588
caatagcaac atg                                                          13


<210>  589
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  589
gcaatagcaa cat                                                          13


<210>  590
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  590
cctgcaatag caa                                                          13


<210>  591
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  2 '-O,4 '-C-methylene- linked bicyclic ribonucleotide

<400>  591
tcctgcaata gca                                                          13


<210>  592
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  592
gtcctgcaat agc                                                          13


<210>  593
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  593
tccgtcctgc aat                                                          13


<210>  594
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base

<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  594
gtccgtcctg caa                                                          13


<210>  595
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  595
ggtccgtcct gca                                                          13

<210>  596
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  596
ctggcttggg aca                                                          13


<210>  597
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic

ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  597
tcatctggct tgg                                                          13


<210>  598
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  598
tctcctggct cat                                                          13

<210>  599
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  599
agcccggctt gca                                                          13


<210>  600
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>

<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  600
tagcccggct tgc                                                          13


<210>  601
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  601
atagcccggc ttg                                                          13


<210>  602
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  5-methylcytosine; 2 '-O,4 '-C-methylene- linked bicyclic
      ribonucleotide

<400>  602
gtgttgatgg gcc                                                          13

<210>  603
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221> modified_base
<222> (12)..(12)
<223> 2 '-O,4 '-C-methylene- linked bicyclic ribonucleotide

<220>
<221> modified_base
<222> (13)..(13)
<223> 2 '-O,4 '-C-methylene- linked bicyclic ribonucleotide

<400> 603
ggacatactc tgg 13


<210> 604
<211> 13
<212> DNA
<213> Artificial Sequence

<220>
<223> Synthetic oligonucleotide:Combined DNA/RNA Molecule: nucleotides
3 to 10 are deoxyribonucleotides-other nucleotides are
ribonucleotides


<220>
<221> modified_base
<222> (1)..(1)
<223> 2 '-O,4 '-C-methylene- linked bicyclic ribonucleotide

<220>
<221> modified_base
<222> (2)..(2)
<223> 2 '-O,4 '-C-methylene- linked bicyclic ribonucleotide

<220>
<221> modified_base
<222> (11)..(11)
<223> 2 '-O,4 '-C-methylene- linked bicyclic ribonucleotide

<220>
<221> modified_base
<222> (12)..(12)
<223> 5-methylcytosine; 2 '-O,4 '-C-methylene- linked bicyclic
ribonucleotide

<220>
<221> modified_base
<222> (13)..(13)
<223> 2 '-O,4 '-C-methylene- linked bicyclic ribonucleotide

<400> 604
aaggacatac tct 13


<210> 605
<211> 13
<212> DNA
<213> Artificial Sequence

<220>
<223> Synthetic oligonucleotide:Combined DNA/RNA Molecule: nucleotides

3 to 10 are deoxyribonucleotides-other nucleotides are
ribonucleotides

<220>
<221> modified_base
<222> (1)..(1)
<223> 2 '-O,4 '-C-methylene- linked bicyclic ribonucleotide

<220>
<221> modified_base
<222> (2)..(2)
<223> 2 '-O,4 '-C-methylene- linked bicyclic ribonucleotide

<220>
<221> modified_base
<222> (11)..(11)
<223> 5-methylcytosine; 2 '-O,4 '-C-methylene- linked bicyclic
ribonucleotide

<220>
<221> modified_base
<222> (12)..(12)
<223> 2 '-O,4 '-C-methylene- linked bicyclic ribonucleotide

<220>
<221> modified_base
<222> (13)..(13)
<223> 5-methylcytosine; 2 '-O,4 '-C-methylene- linked bicyclic
ribonucleotide

<400> 605
aaaggacata ctc 13

<210> 606
<211> 13
<212> DNA
<213> Artificial Sequence

<220>
<223> Synthetic oligonucleotide:Combined DNA/RNA Molecule: nucleotides
3 to 10 are deoxyribonucleotides-other nucleotides are
ribonucleotides

<220>
<221> modified_base
<222> (1)..(1)
<223> 2 '-O,4 '-C-methylene- linked bicyclic ribonucleotide

<220>
<221> modified_base
<222> (2)..(2)
<223> 2 '-O,4 '-C-methylene- linked bicyclic ribonucleotide

<220>
<221> modified_base
<222> (11)..(11)
<223> 2 '-O,4 '-C-methylene- linked bicyclic ribonucleotide

<220>
<221> modified_base

<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  606
gggatattca aaa                                                          13


<210>  607
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  607
tgggatattc aaa                                                          13


<210>  608
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  608
ttgggatatt caa                                                          13


<210>  609
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>

<221>  modified_base
<222>  (13)..(13)
<223>  2 '-O,4 '-C-methylene- linked bicyclic ribonucleotide

<400>  609
gtgcacttgg cag                                                          13


<210>  610
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  5-methylcytosine; 2 '-O,4 '-C-methylene- linked bicyclic
      ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  610
cctcagtgca ctt                                                          13


<210>  611
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>

<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  2 '-O,4 '-C-methylene- linked bicyclic ribonucleotide

<400>  611
tcctcagtgc act                                                          13


<210>  612
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  5-methylcytosine; 2 '-O,4 '-C-methylene- linked bicyclic
      ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine

<400>  612
ccttcctcag tgc                                                          13


<210>  613
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  613
agctgatggc tta                                                          13


<210>  614
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)

<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  614
cagctgatgg ctt                                                          13


<210>  615
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>

<221>  modified_base
<222>  (13)..(13)
<223>  2 '-O,4 '-C-methylene- linked bicyclic ribonucleotide

<400>  615
ccagctgatg gct                                                          13


<210>  616
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
       ribonucleotide

<400>  616
tccagctgat ggc                                                          13


<210>  617
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base

<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  617
tgtttccagc tga                                                          13


<210>  618
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  618
ttgtttccag ctg                                                          13


<210>  619
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  619
gcatgttgtg gca                                                          13


<210>  620
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  620
cgcatgttgt ggc                                                          13


<210>  621
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  5-methylcytosine; 2 '-O,4 '-C-methylene- linked bicyclic
      ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  2 '-O,4 '-C-methylene- linked bicyclic ribonucleotide

<400>  621
cattgtctgg tct                                                          13


<210>  622
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
       ribonucleotide

<400>  622
ccattgtctg gtc                                                          13


<210>  623
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  623
tccattgtct ggt                                                          13


<210>  624
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  624

atccattgtc tgg                                                          13

<210>  625
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
       ribonucleotide

<400>  625
atgtggcttt atc                                                          13

<210>  626
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)

<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  626
aatgtggctt tat                                                          13


<210>  627
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  5-methylcytosine; 2 '-O,4 '-C-methylene- linked bicyclic
      ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  627
ccaaatgtgg ctt                                                          13

<210>  628
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
       ribonucleotide

<400>  628
ccatcatggc agc                                                          13


<210>  629
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)

<223>  2 ′-O,4 ′-C-methylene- linked bicyclic ribonucleotide

<220>
<221>  modified_base
<222>  (11)..(11)
<223>  2 ′-O,4 ′-C-methylene- linked bicyclic ribonucleotide

<220>
<221>  modified_base
<222>  (12)..(12)
<223>  2 ′-O,4 ′-C-methylene- linked bicyclic ribonucleotide

<220>
<221>  modified_base
<222>  (13)..(13)
<223>  5-methylcytosine; 2 ′-O,4 ′-C-methylene- linked bicyclic
      ribonucleotide

<400>  629
tatccatcat ggc                                                          13


<210>  630
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  5-methylcytosine; 2 ′-O,4 ′-C-methylene- linked bicyclic
      ribonucleotide

<220>
<221>  modified_base
<222>  (2)..(2)
<223>  5-methylcytosine; 2 ′-O,4 ′-C-methylene- linked bicyclic
      ribonucleotide

<220>
<221>  modified_base
<222>  (11)..(11)
<223>  2 ′-O,4 ′-C-methylene- linked bicyclic ribonucleotide

<220>
<221>  modified_base
<222>  (12)..(12)
<223>  2 ′-O,4 ′-C-methylene- linked bicyclic ribonucleotide

<220>
<221>  modified_base
<222>  (13)..(13)
<223>  5-methylcytosine; 2 ′-O,4 ′-C-methylene- linked bicyclic
      ribonucleotide

<400>  630
cccagtttgg tac                                                          13

<210>  631
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  631
gaccagtttc cca                                                          13

<210>  632
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)

<223>  2 ′-O,4 ′-C-methylene- linked bicyclic ribonucleotide

<220>
<221>  modified_base
<222>  (11)..(11)
<223>  5-methylcytosine; 2 ′-O,4 ′-C-methylene- linked bicyclic
       ribonucleotide

<220>
<221>  modified_base
<222>  (12)..(12)
<223>  5-methylcytosine; 2 ′-O,4 ′-C-methylene- linked bicyclic
       ribonucleotide

<220>
<221>  modified_base
<222>  (13)..(13)
<223>  5-methylcytosine; 2 ′-O,4 ′-C-methylene- linked bicyclic
       ribonucleotide

<400>  632
agaccagttt ccc                                                          13


<210>  633
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 ′-O,4 ′-C-methylene- linked bicyclic ribonucleotide

<220>
<221>  modified_base
<222>  (2)..(2)
<223>  2 ′-O,4 ′-C-methylene- linked bicyclic ribonucleotide

<220>
<221>  modified_base
<222>  (11)..(11)
<223>  2 ′-O,4 ′-C-methylene- linked bicyclic ribonucleotide

<220>
<221>  modified_base
<222>  (12)..(12)
<223>  2 ′-O,4 ′-C-methylene- linked bicyclic ribonucleotide

<220>
<221>  modified_base
<222>  (13)..(13)
<223>  2 ′-O,4 ′-C-methylene- linked bicyclic ribonucleotide

<400>  633
ggcagaccag ttt                                                          13

<210>  634
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  634
tggcagacca gtt                                                          13

<210>  635
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  635
catggcagac cag                                                          13


<210>  636
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  2 '-O,4 '-C-methylene- linked bicyclic ribonucleotide

<400>  636
gcatggcaga cca                                                          13

<210>  637
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  5-methylcytosine; 2 '-O,4 '-C-methylene- linked bicyclic
       ribonucleotide

<400>  637
ggcatggcag acc                                                          13


<210>  638
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  638
tggcatggca gac                                                          13


<210>  639
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  5-methylcytosine; 2 '-O,4 '-C-methylene- linked bicyclic
      ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
      ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  639
cttggcatgg cag                                                          13


<210>  640

<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  640
acttggcatg gca                                                          13


<210>  641
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>

<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  641
aacttggcat ggc                                                          13


<210>  642
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  642
acaccacagt ggc                                                          13


<210>  643
<211>  13

<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  643
ggtacaccac agt                                                          13


<210>  644
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base

<222>  (11)..(11)
<223>  5-methylcytosine; 2 '-O,4 '-C-methylene- linked bicyclic
       ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  644
ccttggtaca cca                                                          13


<210>  645
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  5-methylcytosine; 2 '-O,4 '-C-methylene- linked bicyclic
       ribonucleotide

<400>  645
tccttggtac acc                                                          13


<210>  646

<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
       ribonucleotide

<400>  646
ctccttggta cac                                                          13

<210>  647
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  647
ctctccttgg tac                                                          13


<210>  648
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  5-methylcytosine; 2 '-O,4 '-C-methylene- linked bicyclic
      ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  648
ctctctcctt ggt                                                          13


<210>  649

<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  649
tctctctcct tgg                                                          13

<210>  650
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>

<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  650
ctctctctcc ttg                                                          13


<210>  651
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  651
agcattccat tct                                                          13


<210>  652
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  2 '-O,4 '-C-methylene- linked bicyclic ribonucleotide

<400>  652
gttccttgaa gca                                                          13

<210>  653
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  5-methylcytosine; 2 '-O,4 '-C-methylene- linked bicyclic
      ribonucleotide

<400>  653
tgttccttga agc                                                          13


<210>  654
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  654
tgtgttcctt gaa                                                          13


<210>  655
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are

ribonucleotides

<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  655
ctgtgttcct tga                                                          13


<210>  656
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)

<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  656
actgtgttcc ttg                                                          13


<210>  657
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  657
gaactgtgtt cct                                                          13


<210>  658
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  5-methylcytosine; 2 '-O,4 '-C-methylene- linked bicyclic
      ribonucleotide

<400>  658
agaactgtgt tcc                                                          13


<210>  659
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  5-methylcytosine; 2 '-O,4 '-C-methylene- linked bicyclic
      ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  659
gccagagaac tgt                                                          13


<210>  660
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  660
agccagagaa ctg                                                          13


<210>  661
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>

<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  661
gatgcatcag ttt                                                          13


<210>  662
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  662
ggatgcatca gtt                                                          13


<210>  663
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  5-methylcytosine; 2 '-O,4 '-C-methylene- linked bicyclic
       ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  663
cggatgcatc agt                                                          13


<210>  664
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>

<221>  modified_base
<222>  (2)..(2)
<223>  5-methylcytosine; 2 '-O,4 '-C-methylene- linked bicyclic
       ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  664
tcggatgcat cag                                                          13


<210>  665
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
       ribonucleotide

<220>
<221>  modified_base
<222>  (13)..(13)
<223>  2 '-O,4 '-C-methylene- linked bicyclic ribonucleotide

<400>  665

atcggatgca tca                                                          13


<210>  666
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  5-methylcytosine; 2 '-O,4 '-C-methylene- linked bicyclic
       ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  666
ggctttacat cgg                                                          13


<210>  667
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)

<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  667
agtgtgacat ttt                                                          13


<210>  668
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  668
aagtgtgaca ttt                                                          13


<210>  669
<211>  13

<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides



<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  669
ggttccttgg atg                                                          13


<210>  670
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides



<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 ′-O,4 ′-C-methylene- linked bicyclic ribonucleotide

<220>
<221>  modified_base
<222>  (13)..(13)
<223>  2 ′-O,4 ′-C-methylene- linked bicyclic ribonucleotide

<400>  670
aggttccttg gat                                                          13


<210>  671
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are
      ribonucleotides


<220>
<221>  modified_base
<222>  (1)..(1)
<223>  2 ′-O,4 ′-C-methylene- linked bicyclic ribonucleotide

<220>
<221>  modified_base
<222>  (2)..(2)
<223>  2 ′-O,4 ′-C-methylene- linked bicyclic ribonucleotide

<220>
<221>  modified_base
<222>  (11)..(11)
<223>  2 ′-O,4 ′-C-methylene- linked bicyclic ribonucleotide

<220>
<221>  modified_base
<222>  (12)..(12)
<223>  2 ′-O,4 ′-C-methylene- linked bicyclic ribonucleotide

<220>
<221>  modified_base
<222>  (13)..(13)
<223>  2 ′-O,4 ′-C-methylene- linked bicyclic ribonucleotide

<400>  671
taggttcctt gga                                                          13


<210>  672
<211>  13
<212>  DNA
<213>  Artificial Sequence

<220>
<223>  Synthetic oligonucleotide:Combined DNA/RNA Molecule: nucleotides
      3 to 10 are deoxyribonucleotides-other nucleotides are

ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  672
ttaggttcct tgg                                                          13

<210>  673
<211>  13
<212>  DNA
<213>  Artificial Sequence

<220>
<223>  Synthetic oligonucleotide:Combined DNA/RNA Molecule: nucleotides
       3 to 10 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  673
ggacatactc tgg                                                          13


<210>  674
<211>  13
<212>  DNA
<213>  Artificial Sequence

<220>
<223>  Synthetic oligonucleotide:Combined DNA/RNA Molecule: nucleotides
       4 to 10 are deoxyribonucleotides-other nucleotides are
       ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (3)..(3)
<223>  2 '-O,4 '-C-methylene- linked bicyclic ribonucleotide

<220>
<221>  modified_base
<222>  (11)..(11)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<400>  674
ggacatactc tgg                                                          13


<210>  675
<211>  14
<212>  DNA
<213>  Artificial Sequence

<220>
<223>  Synthetic oligonucleotide:Combined DNA/RNA Molecule: nucleotides
       4 to 11 are deoxyribonucleotides-other nucleotides are
       ribonucleotides

<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (3)..(3)
<223>  2 '-O,4 '-C-methylene- linked bicyclic ribonucleotide

<220>
<221>  modified_base
<222>  (12)..(12)
<223>  2 '-O,4 '-C-methylene- linked bicyclic ribonucleotide

<220>
<221>  modified_base
<222>  (13)..(13)
<223>  2 '-O,4 '-C-methylene- linked bicyclic ribonucleotide

<220>
<221>  modified_base
<222>  (14)..(14)
<223>  2 '-O,4 '-C-methylene- linked bicyclic ribonucleotide

<400>  675
aggacatact ctgg                                                         14


<210>  676
<211>  15
<212>  DNA
<213>  Artificial Sequence

<220>
<223>  Synthetic oligonucleotide:Combined DNA/RNA Molecule: nucleotides
      4 to 12 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (3)..(3)
<223>  2 '-O,4 '-C-methylene- linked bicyclic ribonucleotide

<220>

<221>  modified_base
<222>  (13)..(13)
<223>  2 '-O,4 '-C-methylene- linked bicyclic ribonucleotide

<220>
<221>  modified_base
<222>  (14)..(14)
<223>  2 '-O,4 '-C-methylene- linked bicyclic ribonucleotide

<220>
<221>  modified_base
<222>  (15)..(15)
<223>  2 '-O,4 '-C-methylene- linked bicyclic ribonucleotide

<400>  676
aaggacatac tctgg                                                        15


<210>  677
<211>  15
<212>  DNA
<213>  Artificial Sequence

<220>
<223>  Synthetic oligonucleotide:Combined DNA/RNA Molecule: nucleotides
      4 to 11 are deoxyribonucleotides-other nucleotides are
      ribonucleotides


<220>
<221>  modified_base
<222>  (1)..(1)
<223>  2 '-O,4 '-C-methylene- linked bicyclic ribonucleotide

<220>
<221>  modified_base
<222>  (2)..(2)
<223>  2 '-O,4 '-C-methylene- linked bicyclic ribonucleotide

<220>
<221>  modified_base
<222>  (3)..(3)
<223>  2 '-O,4 '-C-methylene- linked bicyclic ribonucleotide

<220>
<221>  modified_base
<222>  (12)..(12)
<223>  5-methylcytosine; 2 '-O,4 '-C-methylene- linked bicyclic
      ribonucleotide

<220>
<221>  modified_base
<222>  (13)..(13)
<223>  2 '-O,4 '-C-methylene- linked bicyclic ribonucleotide

<220>
<221>  modified_base
<222>  (14)..(14)
<223>  2 '-O,4 '-C-methylene- linked bicyclic ribonucleotide

<220>
<221>  modified_base
<222>  (15)..(15)

<223>  2 ′-O,4 ′-C-methylene- linked bicyclic ribonucleotide

<400>  677
aaggacatac tctgg                                                        15


<210>  678
<211>  15
<212>  DNA
<213>  Artificial Sequence

<220>
<223>  Synthetic oligonucleotide:Combined DNA/RNA Molecule: nucleotides
       5 to 12 are deoxyribonucleotides-other nucleotides are
       ribonucleotides


<220>
<221>  modified_base
<222>  (1)..(1)
<223>  2 ′-O,4 ′-C-methylene- linked bicyclic ribonucleotide

<220>
<221>  modified_base
<222>  (2)..(2)
<223>  2 ′-O,4 ′-C-methylene- linked bicyclic ribonucleotide

<220>
<221>  modified_base
<222>  (3)..(3)
<223>  2 ′-O,4 ′-C-methylene- linked bicyclic ribonucleotide

<220>
<221>  modified_base
<222>  (4)..(4)
<223>  2 ′-O,4 ′-C-methylene- linked bicyclic ribonucleotide

<220>
<221>  modified_base
<222>  (13)..(13)
<223>  2 ′-O,4 ′-C-methylene- linked bicyclic ribonucleotide

<220>
<221>  modified_base
<222>  (14)..(14)
<223>  2 ′-O,4 ′-C-methylene- linked bicyclic ribonucleotide

<220>
<221>  modified_base
<222>  (15)..(15)
<223>  2 ′-O,4 ′-C-methylene- linked bicyclic ribonucleotide

<400>  678
aaggacatac tctgg                                                        15


<210>  679
<211>  15
<212>  DNA
<213>  Artificial Sequence

<220>
<223>  Synthetic oligonucleotide:Combined DNA/RNA Molecule: nucleotides

5 to 11 are deoxyribonucleotides-other nucleotides are
ribonucleotides

<220>
<221> modified_base
<222> (1)..(1)
<223> 2 '-O,4 '-C-methylene- linked bicyclic ribonucleotide

<220>
<221> modified_base
<222> (2)..(2)
<223> 2 '-O,4 '-C-methylene- linked bicyclic ribonucleotide

<220>
<221> modified_base
<222> (3)..(3)
<223> 2 '-O,4 '-C-methylene- linked bicyclic ribonucleotide

<220>
<221> modified_base
<222> (4)..(4)
<223> 2 '-O,4 '-C-methylene- linked bicyclic ribonucleotide

<220>
<221> modified_base
<222> (12)..(12)
<223> 5-methylcytosine; 2 '-O,4 '-C-methylene- linked bicyclic
ribonucleotide

<220>
<221> modified_base
<222> (13)..(13)
<223> 2 '-O,4 '-C-methylene- linked bicyclic ribonucleotide

<220>
<221> modified_base
<222> (14)..(14)
<223> 2 '-O,4 '-C-methylene- linked bicyclic ribonucleotide

<220>
<221> modified_base
<222> (15)..(15)
<223> 2 '-O,4 '-C-methylene- linked bicyclic ribonucleotide

<400> 679
aaggacatac tctgg 15

<210> 680
<211> 16
<212> DNA
<213> Artificial Sequence

<220>
<223> Synthetic oligonucleotide:Combined DNA/RNA Molecule: nucleotides
5 to 12 are deoxyribonucleotides-other nucleotides are
ribonucleotides

<220>
<221> modified_base
<222> (1)..(1)

<223>  2 ′-O,4 ′-C-methylene- linked bicyclic ribonucleotide

<220>
<221>  modified_base
<222>  (2)..(2)
<223>  2 ′-O,4 ′-C-methylene- linked bicyclic ribonucleotide

<220>
<221>  modified_base
<222>  (3)..(3)
<223>  2 ′-O,4 ′-C-methylene- linked bicyclic ribonucleotide

<220>
<221>  modified_base
<222>  (4)..(4)
<223>  2 ′-O,4 ′-C-methylene- linked bicyclic ribonucleotide

<220>
<221>  modified_base
<222>  (13)..(13)
<223>  5-methylcytosine; 2 ′-O,4 ′-C-methylene- linked bicyclic
      ribonucleotide

<220>
<221>  modified_base
<222>  (14)..(14)
<223>  2 ′-O,4 ′-C-methylene- linked bicyclic ribonucleotide

<220>
<221>  modified_base
<222>  (15)..(15)
<223>  2 ′-O,4 ′-C-methylene- linked bicyclic ribonucleotide

<220>
<221>  modified_base
<222>  (16)..(16)
<223>  2 ′-O,4 ′-C-methylene- linked bicyclic ribonucleotide

<400>  680
aaaggacata ctctgg                                                    16